# EUROPEAN PATENT SPECIFICATION

(11) **EP 4 536 755 B1**
(45) Date of publication and mention of the grant of the patent: **25.02.2026**
(21) Application number: 23755475.3
(22) Date of filing: 13.07.2023
(51) Int. Cl.: C09D 1/00

(54) **COMPOSITIONS COMPRISING SURFACE MODIFIED GLOBULAR NANO-PARTICLES**
ZUSAMMENSETZUNGEN MIT OBERFLÄCHENMODIFIZIERTEN KUGELFÖRMIGEN NANOPARTIKELN
COMPOSITIONS COMPRENANT DES NANOPARTICULES GLOBULAIRES MODIFIÉES EN SURFACE

(30) Priority: 13.07.2022 GB 202210308; 22.11.2022 GB 202217477
(43) Date of publication of application: 16.04.2025
(73) Proprietor: Landa Labs (2012) Ltd., 7612301 Rehovot (IL)
(72) Inventor: ABRAMOVICH, Sagi, Ra'anana 4355318 (IL); AVIDOR, Gal, Rehovot 7637751 (IL); LANDA, Benzion, Nes Ziona 7405135 (IL)
(74) Representative: Harrison IP Limited
(86) International application number: PCT/IB2023/057187
(87) International publication number: WO 2024/013694

(56) References cited:
- WO-A1-2016/183209
- WO-A1-2022/243899
- WO-A2-2008/140594
- GOUVEIA PEDRO J ET AL: "Development of collagen-poly(caprolactone)-based core-shell scaffolds supplemented with proteoglycans and glycosaminoglycans for ligament repair", MATERIALS SCIENCE AND ENGINEERING C, ELSEVIER SCIENCE S.A, CH, vol. 120, 20 October 2020 (2020-10-20), XP086478717, ISSN: 0928-4931, [retrieved on 20201020], DOI: 10.1016/J.MSEC.2020.111657

## Description

### RELATED APPLICATIONS

This application claims Paris Convention priority from Great-Britain Application No. 2210308.9, filed on July 13, 2022, and from Great-Britain Application No. 2217477.5, filed on November 22, 2022. It is related to co-owned International Publication No. WO 2022/243899, published on November 24, 2022.

### FIELD

The present disclosure relates to compositions suitable in particular for dermatological purposes, including to achieve cosmetic and non-cosmetic effects. Methods of preparing these compositions as well as their uses are also disclosed.

### BACKGROUND

Skin plays an important role in protecting the body from hazards of the outer environment. It also displays the most visible signs of aging, such as drop in elasticity, tonicity and firmness leading to skin sagging, and such as superficial blemishes, or lesions, spanning from small lines to deep wrinkles.

The skin changes as a result of intrinsic and extrinsic factors. Intrinsic aging factors include genetics, cellular metabolism, hormones and metabolic processes. Such factors can cause diminished production of collagen and elastin, proteins widely present in the skin to ensure its structural integrity, and reduced production of glycosaminoglycans (GAGs), which are water-binding molecules contributing, together with elastin and collagen, to the skin matrix. Diminished functioning of the sweat and oil glands is another intrinsic process, which may also contribute to the skin becoming thinner and more fragile with age. Extrinsic factors include chronic light exposure, smoking, pollution, ionizing radiation, chemicals, toxins *etc.* They usually lead to thickening of the outermost skin layer (stratum corneum), pre-cancerous changes, likely leading to skin cancer, freckles and sun spots formation, as well as excessive loss of collagen, elastin, and GAGs. Together or alone, these processes give the skin the appearance of deep wrinkles, uneven tone, roughness and thin skin. Collagen, elastin, and GAGs, which can be referred to as structural skin polymers, do not only provide for the mechanical properties of the skin, but also fulfill biological functions, both in healthy and pathological conditions.

There are many approaches to reduce or delay skin aging, ranging from mild topical treatments to more extreme surgical ones. Early signs of aging can be treated with topical application of cosmetic products, including for example retinoids, vitamin C and α-hydroxy acids. Chemical peelings, dermabrasion, micro-needling, ultrasound energy devices, or laser resurfacing may be an option for moderate to severe skin damage. Deeper facial lines may be treated invasively, for instance by injecting botulinum toxin, dermal fillers, or skin polymers being depleted by the aging process, such as collagen itself, the size of these molecules preventing their delivery through the skin. Surgical interventions, such as a face lift, brow lift, or cosmetic surgery on the eyelids, are the more extreme measures taken against wrinkles and skin sagging.

Another approach used in anti-aging treatment of the skin involves increasing collagen synthesis *in situ.* There are many agents that are known to induce such synthesis, which can be administered orally, topically or parenterally. Orally-administered agents include food supplements such as vitamin C, ginseng, and nutrition-derived antioxidants (such as blueberries, cinnamon, certain herbs, *etc.*), among others. Aloe vera and retinol, are two of the agents known to boost collagen synthesis while applied topically on the skin. Hydroxyapatite, on the other hand, may achieve such an effect only if administered by injection.

Topical compositions are considered more convenient for application, as well as safer and typically more effective in covering large areas, compared to compositions administered parenterally (involving pain and infection risks) or orally (where a first-pass metabolism should be overcome to retain efficacy). Hence, while injectable compositions having improved efficacy are still being sought, topical compositions are more desired, especially for anti-aging treatments, such as described above.

However, to be able to permeate the skin, cosmetically-active agents (*i.e*., cosmeceuticals) or medically-active agents (*i.e.,* pharmaceuticals) in such topical compositions should be small enough, typically having a molecular weight (MW) of 500 g/mol or less, to be able to penetrate the skin barrier and achieve a satisfactory transdermal delivery. Such agents are preferably in the form of nano-materials, *e.g*., nano-fibers, nano-emulsions, nano-spheres, nano-capsules, nano-crystals, dendrimers, liposomes, nanotubes, *etc*., such as described in a review by Souto E.B. et al.; "Nanomaterials for Skin Delivery of Cosmeceuticals and Pharmaceuticals"; Applied Sciences, 2020, Vol. 10(5), 1594.

Besides the above exemplary compounds, known to boost synthesis of structural skin proteins, polymers (including proteins, and their fragmented / shorter versions known as peptides) have also been reported to promote the production of collagen, elastin, GAGs or other such molecules involved in maintaining the skin structural and functional integrity. Other polymers (or the same) may (alternatively or additionally) inhibit processes or enzymes (*e.g*., proteases) leading to the deterioration of natural skin proteins. For illustration, some peptides have been reported to boost collagen neo-synthesis, while others have been reported to inhibit collagenase, the enzyme responsible for collagen degradation.

Regardless of the type of biological activity, such materials (whether polymers or not) may have - positively stimulating neo-synthesis of structural skin proteins and/or negatively inhibiting down-regulators of such skin proteins - the end-result may range from reducing or delaying the diminution of skin proteins' amount, maintaining their level, or even increasing their presence. Such agents may be referred to herein as collagen-synthesis stimulating compounds (CSSC), or specifically as collagen-synthesis stimulating polymers (CSSP), the activity of such agents with respect to collagen (and/or other structural skin proteins) including not only the stimulation of its neo-synthesis but alternatively or additionally the prevention of its degradation.

Various conventional methods for preparing nano-materials (*e.g*., polymeric), deemed suitable for skin penetration, are described in the literature. However, solvents typically remain residually trapped within the particles prepared by such methods. When the solvents being used are volatile organic compounds (VOCs), numerous drawbacks are known, since despite their relative volatility, such solvents cannot be fully eliminated beyond a residual level which can be non-negligible. First, the presence of a VOC within particles, even at residual level, may cause toxicity to the skin, regulatory guidelines accordingly requiring very limited amounts of residual VOCs, which might not be feasibly obtained by traditional methods. Secondly, as a VOC may gradually evaporate over time until it reaches its residual level, the particles' structure or properties may be altered during this period, consequently affecting their efficacy. While cumbersome methods have been developed in an attempt to address the residual entrapment of undesired solvents, the approaches reported so far fail to provide nano-particles, or even micro-particles having a narrow size distribution, and/or may lack commercial feasibility in view of their complexity, their failure to form stable dispersions, or their low encapsulation efficiency, when active ingredients are additionally desired.

WO 2016/183209 discloses a topical formulation for application to the skin comprising core-shell particles having a hydrophobic core and a shell formed of hyperbranched polymers covalently bound to materials forming the core. WO 2008/140594 discloses a suspension of nanoparticles comprising at least one unmodified peptide of 2 to 30 amino acids having a biological activity in the skin, subcutaneous tissue or contiguous muscles.

As dermatological - pharmaceutical or cosmetic - products are constantly required in order to restore or maintain skin integrity (function and/or structure) for as long as possible, there remains a need to provide dermatological compositions that resolve at least some of the problems described above. Advantageously, the novel compositions would permit deposition within the skin layers or even transdermal transfer of molecules having a high molecular weight, whose conventional delivery would otherwise require injection.

### SUMMARY

In a first aspect of the disclosure, there is provided a composition according to claim 1 and claims appended thereto.

By way of introduction, the composition comprises core-shell nano-elements composed of a core including a water-insoluble collagen-synthesis stimulating compound (CSSC), such as a collagen-synthesis stimulating polymer (CSSP), and a shell including at least one fatty amine, the core-shell nano-elements being dispersed as nano-particles or nano-droplets in a polar carrier. Notably, the CSSC or CSSP molecules (which can be functionalized to enhance any of their desired properties) may have a molecular weight of 0.6 kDa or more. The nano-elements may further contain a second shell, formed of an active cosmetic or pharmaceutical agent, the second shell surrounding the fatty amine (first) shell. The compositions, which typically include at least one surfactant with the nano-elements (CSSC and/or shell(s)), the carrier, or both, may optionally further contain, in addition to the CSSCs or CSSPs, at least one active agent within the core, such as specified herein-below.

While these compositions, when dermatological compositions, have been developed in order to overcome, *inter alia,* at least some of the drawbacks associated with present delivery of CSSC(s) (*e.g*., CSSP(s)) and/or particular active agent(s) to the skin, transdermal delivery by topical application being preferred, their suitability for parenteral delivery by sub-cutaneous injection in the area to be dermatologically treated (*e.g.,* as dermal fillers) is not ruled out.

As used herein, the transdermal delivery of CSSC(s) and/or active agent(s) refers to the ability of such compounds to be retained upon the skin surface, and/or to penetrate into the skin layers to target sites within the skin or just below it. In some embodiments, this route of administration of dermatological compositions according to the present teachings may further allow the delivery of such compounds across the skin for systemic distribution. Also disclosed are methods for preparing such topical or injectable dermatological compositions as well as uses thereof.

The non-covalent association of the shell to the core is believed to advantageously maintain the original activity of each of the molecules of the core and the shell, none of their moieties being involved in covalent binding through electron sharing that could have reduced or modified their respective contribution to the potency of the composition. Moreover, the fact that the fatty amines are not covalently bound to the CSSCs allow them to migrate to the outer surface of the nano-elements to form a shell thereon.

In some embodiments, the CSSC has a native viscosity of 10⁷ millipascal-second (mPa·s) or less, 10⁶ mPa·s or less, 10⁵ mPa·s or less, 10⁴ mPa·s or less, or 10³ mPa·s or less, as measured at 50°C and a shear rate of 10 sec⁻¹.

In other embodiments, the native viscosity of the CSSC, typically a CSSP, is higher than 10⁷ mPa·s under the aforesaid measuring conditions, being for instance of up to 10¹¹ mPa·s, in which cases the CSSC can be combined with at least one plasticizing agent.

In some embodiments, the fatty amines, in addition to their role in forming the shell of the nano-elements, may also serve as a plasticizing agent capable of reducing the native viscosity of the CSSC of the core and facilitating its processing and incorporation as nano-elements into the present compositions. In other embodiments, reduction of viscosity of the CSSC, if desired, is obtained by adding a non-volatile liquid to the core to act as a plasticizing agent.

Hence, in some embodiments, the fatty amine(s) included in the composition and the non-volatile liquid(s) optionally added to act as a plasticizing agent to the CSSC are present each in a respective amount such that the viscosity of the nano-elements formed therewith is of no more than 10⁷ mPa·s, as measured at 50°C and a shear rate of 10 sec⁻¹. The viscosity of the bulk CSSC can be referred to as a first viscosity of the CSSC, whereas the viscosity of the nano-elements containing at least the CSSC(s) and the fatty amine(s) can be referred to as a second viscosity. More generally, a property characterizing the bulk CSSC(s) contained in the core can be called a "first" property, while an analogous property as measured on the nano-elements can be referred to as a "second" property, which can have same or different value as compared to the native CSSC.

In some embodiments, the nano-elements including in their core the CSSC having been optionally plasticized by the fatty amine(s) and/or by the non-volatile liquid(s) display a second viscosity of 5x10⁶ mPa·s or less, 10⁶ mPa·s or less, 5x10⁵ mPa·s or less, 10⁵ mPa·s or less, 5x10⁴ mPa·s or less, 10⁴ mPa·s or less, 5x10³ mPa·s or less, or 10³ mPa·s or less, as measured at a temperature of 50°C and a shear rate of 10 sec⁻¹.

When the viscosity measured on the nano-elements isolated from the composition is reduced as compared to the first viscosity of the bulk CSSC, the core of the nano-elements is considered plasticized and the CSSC of the core can be referred to as a "plasticized" or "swelled" CSSC.

In some embodiments, the nano-elements comprised in the present compositions (and/or obtained by the present methods) contain less than 2 wt.%, less than 1.5 wt.%, or less than 1 wt.% of a volatile organic compound (VOC), or a blend thereof, by weight of the nano-elements.

In some embodiments, the CSSC (and/or the core-shell nano-elements comprising it, optionally as a plasticized CSSC) is/are characterized by at least one, at least two, or at least three of the following structural properties:
i. the CSSC and/or the core-shell nano-elements is/are insoluble in the polar carrier;
ii. the CSSC and/or the core-shell nano-elements is/are biodegradable and/or biocompatible;
iii. the CSSC and/or the core-shell nano-elements has/have at least one of a melting temperature (***Tm***), a softening temperature (***Ts***), or a glass transition temperature (***Tg***) of at most 300°C, at most 250°C, at most 200°C, at most 180°C, at most 150°C, or at most 120°C, said temperatures being either a first (*i.e.,* native) ***Tm, Ts*** or ***Tg*** of the CSSC, or a second ***Tm, Ts*** or ***Tg*** of the nano-elements, or both;
iv. the CSSC and/or core-shell the nano-elements has/have each respectively a first and/or a second ***Tm*** of at least 0°C, at least 10°C, at least 20°C, at least 30°C, at least 40°C, at least 50°C, or at least 60°C;
v. the CSSC and/or the core-shell nano-elements has/have each respectively a first and/or a second ***Tm*** between 0°C and 300°C, between 10°C and 300°C, between 20°C and 300°C, between 20°C and 250°C, between 20°C and 200°C, between 30°C and 180°C, between 40°C and 180°C, or between 50°C and 150°C;
vi. the CSSC and/or the core-shell nano-elements has/have each respectively a first and/or a second ***Tg*** or ***Ts*** of -75°C or more, -50°C or more, -25°C or more, 0°C or more, 20°C or more, 30°C or more, 40°C or more, 50°C or more, or 60°C or more;
vii. the CSSC and/or the core-shell nano-elements has/have each respectively a first and/or a second ***Tg*** or ***Ts*** between -75°C and 300°C, between -50°C and 250°C, between -25°C and 200°C, between 0°C and 180°C, between 20°C and 180°C, or between 30°C and 150°C;
viii. the CSSC and/or the core-shell nano-elements has/have each respectively a first and/or a second viscosity of 10⁷ mPa·s or less, 5x10⁶ mPa·s or less, 10⁶ mPa·s or less, 5x10⁵ mPa·s or less, 10⁵ mPa·s or less, 5x10⁴ mPa·s or less, 10⁴ mPa·s or less, 5x10³ mPa·s or less, or 10³ mPa·s or less, as measured at 50°C and a shear rate of 10 sec⁻¹;
ix. the CSSC has a molecular weight of 0.7 kDa or more, 0.8 kDa or more, 0.9 kDa or more, 1 kDa or more, 2 kDa or more, or 5 kDa or more;
x. the CSSC has a molecular weight of 500 kDa or less, 300 kDa or less, 200 kDa or less, 100 kDa or less, 80 kDa or less, 50 kDa or less, 25 kDa or less, or 15 kDa or less; and
xi. the CSSC has a molecular weight between 0.6 kDa and 500 kDa, between 0.7 kDa and 300 kDa, between 0.8 kDa and 200 kDa, between 1 kDa and 100 kDa, or between 2 kDa and 80 kDa.

In some embodiments, the at least one structural property fulfilled by at least one of the CSSC and the core-shell nano-elements (optionally including a plasticized CSSC) is: property i) as above listed; property ii) as above listed; property iii) as above listed; property iv) as above listed; property v) as above listed; property vi) as above listed; property vii) as above listed; property viii) as above listed; property ix) as above listed; property x) as above listed; or property xi) as above listed.

In some embodiments, the at least two structural properties fulfilled by at least one of the CSSC and the core-shell nano-elements are: properties i) and ii); properties i) and v); properties i) and vii); properties i) and viii); properties i) and xi); properties ii) and v); properties ii) and vii); properties ii) and viii); properties ii) and xi); properties v) and xi); or properties vii) and xi); or properties viii) and xi) of the above-listed properties.

In some embodiments, the at least three structural properties fulfilled by at least one of the CSSC and the core-shell nano-elements are: properties i), ii) and iii); properties i), ii) and v); properties i), ii) and vii); properties i), ii) and viii); properties i), ii) and xi); properties i), v) and vii); properties i), v) and viii); properties i), vii) and viii); properties i), v) and xi); properties i), vii) and xi); or properties i); or viii) and xi) of the above-listed properties.

In particular embodiments, the CSSC is a CSSP, the chemical compound being a polymer formed of repeating structural units, such monomers being either same (forming homopolymers) or different (forming random or block copolymers). In another particular embodiment, the polymer of the CSSP is a thermoplastic polymer. While non-polymeric compounds typically have molecular weights of up to 2 kDa, generally not exceeding 1 kDa, CSSPs can be larger molecules of at least a few kDas.

As used herein, the term "nano-elements", as used with respect to the core-shell structures containing *inter alia* the CSSC (plasticized or not), refers to relatively solid nano-particles or relatively liquid nano-droplets having an average diameter of 200 nm or less, 150 nm or less, 100 nm or less, 75 nm or less, or 50 nm or less, such structures being dispersed (*e.g*., as a result of nano-sizing) in a homogeneous medium, forming therein a nano-suspension. Such core-shell nano-elements have typically an average diameter of 2 nm or more, 5 nm or more, 10 nm or more, 15 nm or more, or 20 nm or more. In some embodiments, the average diameter of the core-shell nano-elements of the compositions according to the present teachings is between 2 nm and 200 nm, between 5 nm and 150 nm, between 10 nm and 100 nm, between 15 nm and 75 nm, or between 20 nm and 50 nm. The average diameter of the core-shell nano-elements can be determined by any suitable method and may refer to the hydrodynamic diameter of the elements as measured by Dynamic Light Scattering (DLS) and is generally established for 50% of the nano-elements by number (D_{N}50).

In view of their intended use and/or method of preparation, CSSCs suitable for the present invention are advantageously, but not necessarily, relatively solid at room temperature (*circa* 25°C) and up to body temperatures (*e.g*., *circa* 37°C for human subjects). Such preferences are extended to the nano-elements and their cores comprising the optionally plasticized CSSC, which further takes into account the fatty amine(s) and/or non-volatile liquid(s) and their relative amounts, or the presence of any other material affecting the thermal behavior of the product. As can be appreciated by persons skilled in the art, as the CSSCs can be thermoplastic polymers, a "relative solidity" of such materials, or such materials being "relatively solid", at any particular temperature is referring to the fact that they are not necessarily solid but display a viscoelastic behavior. Without wishing to be bound by any particular theory, such feature of the CSSCs should ensure, to the extent necessary, that the nano-elements made therefrom are relatively non-sticky, facilitating their even distribution in a composition according to the present teachings. From the standpoint of skin penetration as required for transdermal delivery, it is believed that the relatively malleable / deformable nature of the core (whether intentionally plasticized or not to have a suitable viscosity as herein disclosed) facilitates the transfer of the nano-elements through the skin, the pathways across the stratum corneum, or down hair follicles or sweat glands being often narrow and tortuous.

The compositions of the present invention are in the form of a nano-suspension. Depending on the ***Tm*** or ***Ts*** of the CSSCs (either plasticized within the core of the nano-elements or having the desired viscosity in their native form), the composition can be at room temperature in the form of a nano-dispersion (*i.e.,* if the ***Tm*** or ***Ts*** is above 20°C, *e.g.,* between 25°C and 80°C), the core-shell nano-elements being relatively solid nano-particles, or in the form of a nano-emulsion (*i.e.,* if the ***Tm*** or ***Ts*** is below 20°C), the core-shell nano-elements being relatively liquid nano-droplets.

The CSSC (*e.g.,* CSSP) can be a natural compound, even if artificially synthesized, or a synthetic compound, having no natural occurrence. Some compounds suitable for the present teachings may, under particular circumstances, assemble to form larger molecules considered then as polymers. Such compounds shall be referred to as polymerizable before such connections are made.

In some embodiments, the polymerizable CSSC is a natural compound selected from: resins, gums, gum-resins and combinations thereof. In a particular embodiment, the polymerizable CSSC is shellac or gum rosin.

In other embodiments, the CSSC is non-polymerizable, such as a quinone, in particular ubidecarenone also called 1,4-benzoquinone or coenzyme Q10 (CoQ10).

In other embodiments, the CSSC is a CSSP, which may also be of synthetic or natural origin. In some embodiments, the thermoplastic polymer is a biodegradable polymer, selected from a group of polymer families comprising: aliphatic polyesters, such as polycaprolactone (PCL), polylactic acid (PLA), poly(L-lactide) (PLLA), poly(D-lactide) (PDLA), poly(D,L-lactide) (PDLLA), polyglycolic acid (PGA), poly(p-dioxanone) (PPDO) and poly(lactic-co-glycolic acid) (PLGA); polyhydroxy-alkanoates, such as polyhydroxybutyrate (PHB), poly-3-hydroxy-butyrate (P3HB), poly-4-hydroxy-butyrate (P4HB), polyhydroxy-valerate (PHV), poly(3-hydroxybutyrate-co-3-hydroxyvalerate) (PHBV), polyhydroxy-hexanoate (PHH) and polyhydroxyoctanoate (PHO); poly(alkene dicarboxylates), such as poly(butylene succinate) (PBS), poly(butylene succinate-co-adipate) (PBSA) and poly(ethylene succinate) (PES); polycarbonates, such as poly-(trimethylene carbonate) (PTMC), poly(propylene carbonate) (PPC) and poly[oligo-(tetramethylene succinate)-co(tetramethylene carbonate)]; aliphatic-aromatic co-polyesters, such as poly(ethylene terephtalate) (PET) and poly(butylene adipate-co-terephtalate) (PBAT); isomers thereof, copolymers thereof and combinations thereof. While some of the afore-said polymers have natural counterparts, these exceptions are typically commercially available almost exclusively in artificially prepared form, so that the entire group is often considered as representative of synthetic polymers.

Alternatively, the thermoplastic polymer can be a non-biodegradable synthetic polymer such as a polyamide (PA), a polyethylene (PE), a poly(ethylene-co-acrylic acid) (PEAA), a poly(ethylene-co-methacrylic acid) (PEMAA), a poly(ethylene-co-n-butyl acrylate) (PEBA), a poly(ethylene-co-vinyl acetate) (PEVA), a polymethylmethacrylate (PMMA), a polypropylene (PP), a polysiloxane, a polystyrene (PS), a polytetrafluoroethylene (PTFE), polyurethane (PU), or a polyvinyl chloride (PVC). In particular such embodiments, the CSSC being a non-biodegradable CSSP is selected from a group comprising PA, PE, PEAA, PEMAA, PEBA, PEVA, substituted or modified versions thereof, ionomers thereof and combinations thereof.

In other embodiments, the CSSP is a natural biodegradable polymer selected from polysaccharides, lignin and combinations thereof.

In particular embodiments, the CSSC is a biodegradable material such as CoQ10, shellac, gum rosin or a CSSP being an aliphatic polyester. In a further particular embodiment, the aliphatic polyester of the CSSP is selected from polycaprolactone, polylactic acid, poly(lactic-co-glycolic acid), poly(butylene succinate-co-adipate), isomers thereof, copolymers thereof and combinations thereof.

In some embodiments, the non-volatile liquid that may be added to the CSSC to lower at least one of its first (native) viscosity, ***Tm, Tg*** and ***Ts*** is selected from a group comprising: monofunctional and polyfunctional aliphatic esters, fatty esters, cyclic organic esters, aromatic esters, fatty acids, terpenes, aromatic alcohols, aromatic ethers, aldehydes and combinations thereof. In some embodiments, the non-volatile liquid is an aliphatic or aromatic fatty ester. In particular embodiments, the non-volatile liquid is selected from: dibutyl adipate, dibutyl sebacate, triethyl O-acetylcitrate, C₁₂-C₁₅ alkyl benzoate and dicaprylyl carbonate.

In other embodiments, when the polymer is a non-biodegradable synthetic polymer, the non-volatile liquid may additionally be selected from a group comprising mineral oils, natural oils, vegetal oils, essential oils, synthetic oils and combinations thereof, provided that they satisfy the present requirements. Non-limiting examples of synthetic oils include synthetic isoparaffins (*e.g*., Isopar^{™} M and Isopar^{™} V), C₁₂-C₁₅ alkyl ethylhexanoate, C₁₂-C₁₅ alkyl benzoate, or isononlyl isononanoate, to name but a few. Non-limiting examples of suitable vegetal oils include castor oil, corn oil, pomegranate seeds oil, or avocado oil, to name but a few. Non-limiting examples of essential oils include clove leaf oil, lavender oil, or oregano oil, to name but a few. In some embodiments, the water-insoluble fatty amine is a primary, a secondary, a tertiary or a quaternary C₈-C₂₂ straight, branched, or cyclic, saturated or unsaturated, aliphatic or aromatic, alkyl or aryl amine, including combinations thereof. Comprehensive lists of fatty amines can be found in chemical databases, the following compounds being only provided for illustration of each class. Stearylamine (C₁₈H₃₉N) is an example of a straight, saturated, aliphatic primary alkyl amine (of general formula R-NH₂), iso-stearylamine being a branched counterpart and oleylamine (C₁₈H₃₇N) differing by including one unsaturated bond in an alkyl chain of same length. Dinonylamine is a secondary amine (of general formula R₁-NH-R₂) and trioctylamine is a tertiary amine (N-R₁,R₂,R₃). Quaternary fatty amines can be quaternary ammonium salts or cationic fatty imidazolines, the latter illustrating fatty amines including a heterocyclic ring. In some embodiments, the water-insoluble fatty amine is a primary, a secondary, a tertiary or a quaternary C₈-C₂₀ straight, branched, or cyclic, saturated or unsaturated, aliphatic amine. In particular embodiments, the water-insoluble fatty amine is selected from a group consisting of oleyl amine, octyl amine, N,N-Bis-(2-hydroxyethyl)C₁₂-C₁₈-alkylamine, N,N-dimethyl-dodecylamine (DMDA), cetrimonium chloride (cetyl-trimethyl-ammonium chloride (CTAC)) and combinations thereof.

In some embodiments, the polar carrier in which the core-shell nano-elements comprising the CSSC can be dispersed includes water, glycols (*e.g*., propylene glycol, 1,3-butanediol, 1,4-butanediol, 2-ethyl-1,3-hexanediol and 2-methyl-2-propyl-1,3-propanediol), glycerol, precursors and derivatives thereof, collectively termed herein "glycerols", (*e.g*., acrolein, dihydroxyacetone, glyceric acid, tartronic acid, epichlorohydrin, glycerol tertiary butyl ether, polyglycerol, glycerol ester and glycerol carbonate) and combinations thereof. In a particular embodiment, the polar carrier comprises water, consists of water or is water.

In some embodiments, the chemical identity and respective amounts of the materials constituting the core and the materials constituting the shell, as well as the medium in which they are dispersed, are such that the core-shell nano-elements formed thereby have a positive charge of +5 mV or more, +10 mV or more, +20 mV or more, +30 mV or more, or +40 mV or more. In other embodiments, the core-shell nano-elements have a negative charge between -60 mV and -5 mV, between -50 mV and -5 mV, between -40 mV and -5 mV, between -30 mV and -5 mV, or between -20 mV and -5 mV. In further additional embodiments, a near neutral charge is formed on the core-shell nano-elements, being between -5 mV and +5 mV. The above charges are measured in the presence of water at room temperature.

As known to persons skilled in the measurement of charge and zeta potential of compositions, it is stressed that in this particular context an aqueous polar carrier need only to contain an amount of water (*e.g*., ≥ 5 wt.%) sufficient to promote the protonation of the fatty amines of the shell. Such values, which can be measured with any suitable equipment, can be determined in the composition "as is" or in a diluted sample thereof. Depending on the nature of the amine groups of the fatty amines, the pH providing optimal charging may vary, and the aforesaid (*e.g*., positive) charges of the nano-elements can be observed at at least one pH in the range of pH 1 to pH 10, as can be preliminary estimated with a suitable pH test strip and confirmed with a pH meter.

In some embodiments, the fatty amine, in addition to its role in forming the shell and optional role as a plasticizing agent, may also serve as a surfactant for the purpose of stabilizing the core-shell nano-elements within the nano-suspension. In this case, the composition may not require the addition of a surfactant in the polar carrier to stabilize the core-shell nano-elements in the liquid medium. This can be advantageous when the composition is to be applied to surfaces which can be sensitive to the presence of surfactants, such as skins.

In other embodiments, the dermatological (*e.g*., topical) composition further comprises at least one surfactant, selected from an emulsifier and a hydrotrope. The surfactant(s) may be present in the CSSC in the core of the core-shell nano-elements (*e.g*., if being CSSC-miscible and polar-carrier-insoluble surfactant(s)), in the liquid phase containing the polar carrier (*e.g.,* if being polar-carrier-soluble surfactant(s)), or in both (*e.g.,* if being intermediate emulsifiers).

In some embodiments, the dermatological (*e.g*., topical) composition further comprises at least one skin-penetration enhancer. Such agents are typically found in the liquid phase in which the nano-elements containing the CSSC are dispersed.

In some embodiments, the dermatological (*e.g*., topical) composition further comprises an active agent within the core including the CSSC, the active agent being miscible with the CSSC and substantially insoluble in the polar carrier (hence, referred to as a "carrier-insoluble active agent"). The carrier-insoluble active agent can be a blend of different agents, each having a same or different activity.

In some embodiments, the dermatological (*e.g*., topical) composition further comprises an active agent forming a second shell on the surface of the core-shell nano-elements, the active agent being soluble in the polar carrier (hence, referred to as a "carrier-soluble active agent"). In some embodiments, the second shell is composed of at least one layer of the carrier-soluble active agent. Nano-elements comprising a core of CSSC (optionally including CSSC-miscible / polar-carrier-insoluble surfactant(s) and/or active agent(s)), a first shell of fatty amine(s) and a second shell being at least one layer of a carrier-soluble active agent, can be referred to as core-shells or as core-multi-shells nano-elements.

Advantageously, the first shell of a core-multi-shells nano-element which is positively charged in an aqueous polar carrier of the composition facilitates the non-covalent formation of the second shell, for instance by electrostatic interactions with carrier-soluble active agents having a negative charge in the polar carrier. In such a case, the nano-elements which were formerly positively charged with a single shell of fatty amines in the medium being considered, may become less positively charged, electrostatically neutral, or negatively charged. Additionally, or alternatively, the first shell of fatty amines can form covalent bounds with the second shell of carrier-soluble active agents facilitating their indirect attachment to the core.

Preferably, the covalent or non-covalent wrapping of the second shell around the first shell is such that the nano-elements do not increase in size beyond the size ranges herein disclosed as suitable when transdermal delivery is envisioned.

In some embodiments, the core-shell nano-elements have a first surface zeta potential (ζ1), and the carrier-soluble active agent has a second zeta potential (ζ2) such that an absolute value of a zeta potential differential (Δζ) defined as Δζ = |ζ2 - ζ1| is at least 5 mV, as measurable in the presence of water. Such values can be determined as described for the charge of the core-shell nano-elements, the conditions (e.g., equipment, temperature and pH) selected for the measurements being the same for the nano-elements and the carrier-soluble active agent.

As used herein, a material is deemed to be insoluble in a liquid carrier, being for instance a "polar-carrier-insoluble active agent" (or a "carrier-insoluble active agent"), if having a solubility within the carrier it is immersed in of less than 5 wt.% (and more typically, less than 4 wt.%, less than 3 wt.%, less than 2 wt.%, less than 1 wt.%, or less than 0.5%) by weight of the polar carrier, at a temperature of 20°C.

Conversely, a material is deemed to be soluble in a liquid carrier, being for instance a "polar-carrier-soluble active agent" (or a "carrier-soluble active agent"), if having a solubility within the carrier it is immersed in of 5 wt.% or more (and more typically, 6 wt.% or more, 7 wt.% or more, 8 wt.% or more, 9 wt.% or more, or 10 wt.% or more) by weight of the polar carrier, at a temperature of 20°C. As appreciated by a skilled person some materials having a sought activity can be polar-carrier-insoluble in one chemical form, and polar-carrier-soluble in another, salts of a material typically increasing its solubility.

In some embodiments, the dermatological (*e.g*., topical) compositions comprise more than one active agent in addition to the CSSC, the active agents being either in same or different phase. For illustration, a first active agent, being carrier-insoluble, can be contained within the cores of the nano-elements and a second active agent, being carrier-soluble, can form the second shell surrounding a first shell of fatty amines.

The active agents that can be incorporated in the core-shell(s) nano-elements of the present disclosure, can have a cosmetic or a pharmaceutic purpose, having for illustration one or more activities selected from a group comprising: analgesic, anesthetic, anti-acne, anti-aging, anti-arthritic, anti-bacterial, anti-dandruff, anti-fungal, anti-inflammatory, anti-oxidant, antiparasitic, anti-rheumatic, anti-viral, immunoregulatory, hair growth promoting or inhibiting, skin bleaching or tanning, and any like cosmetic or therapeutic activity beneficial to the skin or the treatment of its disorders, on the surface of the skin, within its layers or underneath it.

As used herein, the term "treatment" and its grammatical variants includes prevention, amelioration, attenuation, delay or arrest of progression and cure of the condition being treated; or may increase the efficacy of co-administered treatments directed to a same condition. For illustration, if a subject is to receive, in order to treat a skin wound, an autologous or heterologous skin graft, an heterograft or a synthetic skin substitute, the present compositions can improve the value of such treatment by promoting the neo-synthesis of skin proteins in the area being grafted and/or by locally providing immunosuppressors so as to facilitate the acceptance of the graft. The extent of treatment or co-treatment, as afore-exemplified, may be assessed, in some embodiments, by a diminution of the symptoms relevant to the condition being treated.

Advantageously, the dermatological (*e.g*., topical) compositions of the present invention can have a relatively high concentration (*e.g.,* 1 wt.% or more) of a CSSC (*e.g.,* a CSSP) and/or of an optional active agent (*e.g*., retinol within the core or HA in a second shell, surrounding the core first shell), and/or the CSSC(s) and/or optional active agent(s) can have a relatively high molecular weight, as compared to conventional topical compositions comprising such ingredients. Without wishing to be bound by theory, the relatively higher loading of the CSSC(s) and/or optional active agent(s), and/or the relatively higher potency thereof (when MW-dependent), is expected to provide a higher gradient of concentration, favoring transdermal delivery, and ultimately cosmetic or pharmaceutic efficacy.

It is noted that nano-particles or nano-droplets of a CSSC having a particle size within the range of dimensions disclosed herein was found unexpectedly successful by the Inventors, as such forms of the CSSCs, in particular if being plasticized CSSPs, were expected to aggregate in view of their anticipated stickiness.

In a further aspect of the disclosure, there is provided a method for preparing a composition comprising core-shell(s) nano-elements according to claim 10 and claims appended thereto.

By way of introduction, the method comprises the steps of:
a) providing at least one CSSC, wherein the CSSC, or each CSSC:
   i. is water-insoluble;
   ii. has a molecular weight of at least 0.6 kDa;
   iii. has at least one of a first melting temperature (***Tm***), a first softening temperature (***Ts***), and a first glass transition temperature (***Tg***) of 300°C or less; and
   iv. has a first viscosity optionally higher than 10⁷ mPa·s, as measured at 50°C and a shear rate of 10 sec⁻¹;
b) mixing the at least one CSSC with at least one water-insoluble fatty amine miscible with the CSSC(s), the mixing being at a mixing temperature equal to or higher than at least one of the first ***Tm, Ts,*** and ***Tg*** of the CSSC, whereby a homogeneous mixture including the fatty amine(s) and an optionally plasticized CSSC(s) is formed, the mixture having:
   A- a second ***Tm, Ts,*** or ***Tg*** optionally lower than the respective first ***Tm, Ts,*** or ***Tg*** of the CSSC by at least 5°C, at least 10°C, at least 15°C, at least 20°C, at least 25°C, at least 30°C, at least 35°C, at least 40°C, at least 45°C, or at least 50°C; and
   B- a second viscosity optionally lower than the first viscosity, at least one of the first and the second viscosity being of 10⁷ mPa·s or less, as measured at 50°C and a shear rate of 10 sec⁻¹;
c) combining a polar carrier with the homogenous mixture obtained in step b); and
d) nano-sizing the combination of step c) by applying shear at a shearing temperature equal to or higher than at least one of the first and second ***Tm, Ts,*** and ***Tg,*** so as to obtain a nano-suspension of core-shell nano-elements dispersed in the polar carrier;
wherein at least 50% of the total number of the core-shell nano-elements have an average diameter (*e.g*., D_{N}50) of 200 nanometer (nm) or less.

The materials being mixed with the CSSC(s) in step b) should be miscible therewith (and any other ingredients composing the mix). They are typically insoluble in the polar carrier if intended to be incorporated in the core of the present nano-elements, so as to avoid their leaching out when undesired. This former property of the ingredients is, however, not essential, if their release from the core is sought or if their prospective leaching can be prevented by including the ingredient in the liquid phase in an amount yielding a blocking gradient. A material is said to be CSSC-miscible, if capable of forming a homogeneous mixture with CSSC without causing phase separation or any other like alteration of the blend due to at least form the continuous core.

In some cases, the mixing temperature in step b) is higher than the at least one first ***Tm, Ts,*** or ***Tg*** of the CSSC by 5°C or more, 10°C or more, 20°C or more, 30°C or more, or 40°C or more, as long as the mixing is performed at a temperature at which an insignificant part of the fatty amine, as well as of the non-volatile liquid is boiled away. Assuming that the fatty amine has a boiling temperature ***Tbₐ*** and the non-volatile liquid has a boiling temperature ***Tbₗ*** at the pressure of the mixing step, then in some embodiments the mixing temperature can additionally be lower than the lowest of the boiling temperatures ***Tbₐ*** of the fatty amine and the ***Tbₗ*** of the non-volatile liquid. When the duration of mixing is sufficiently brief and/or the fatty amine and/or non-volatile liquid are in sufficient excess, the mixing temperature can alternatively be at boiling temperatures ***Tbₐ*, *Tbₗ*** or above.

Advantageously, the afore-said methods do not require the solubilization of a water-insoluble CSSC within a solvent being a VOC, nor the addition of a VOC for any other reason, so that nano-elements obtained by the present methods may in some embodiments have a relatively low wt.% content of a VOC by weight of the nano-elements as detailed for the compositions.

In some cases, the CSSC provided in step a) of the method is biodegradable and/or biocompatible.

In some cases, the shearing temperature is higher than the second ***Tm, Ts,*** or ***Tg*** of the mixture including the plasticized CSSC (or higher than the first ***Tm, Ts,*** or ***Tg,*** in case of an unplasticized CSSC) by 5°C or more, 10°C or more, 20°C or more, 30°C or more, or 40°C or more, as long as the nano-sizing is performed at a temperature or under any other conditions at which an insignificant part of the polar carrier is boiled away. Assuming that the polar carrier has a boiling temperature ***Tb_{c}*** at the pressure of the nano-sizing step, then in some embodiments, the shearing temperature can additionally be lower than the boiling point ***Tbₐ*** of the fatty amine, and/or the boiling point ***Tbₗ*** of the non-volatile liquid (if added) and/or lower than the boiling point ***Tb_{c}*** of the polar carrier, under the pressure at which the nano-sizing step is performed, by 5°C or more, 10°C or more, 20°C or more, 30°C or more, or 40°C or more. When the duration of nano-sizing is sufficiently brief and/or the polar carrier is in sufficient excess, the nano-sizing temperature can alternatively be at boiling temperature ***Tb_{c}*** or above.

In some cases, the obtained nano-suspension is a nano-emulsion, and the method comprises an additional step, wherein the obtained nano-emulsion is cooled to a temperature lower than at least one of the first or second ***Tm, Ts,*** or ***Tg*** of the CSSC or of the blend containing it. While such cooling may passively occur upon termination of nano-sizing, the temperature of the nano-suspension naturally decreasing over time to room temperature (or a lower temperature of storage, if desired), in some embodiments, the cooling is performed by actively lowering the temperature of the nano-emulsion by any suitable cooling method. Additionally, or alternatively, the cooling is performed under continued shearing or any other method maintaining the agitation of the composition. While the composition may remain a nano-emulsion following its active or passive cooling, in some embodiments, the composition can then form a nano-dispersion.

In some cases, a surfactant is added during step b) and/or during step c), the surfactant being either an emulsifier or a hydrotrope, as herein described.

In some cases, the polar carrier is not water, and the method further comprises a step of replacing at least part of the polar carrier by water.

In some cases, the method further comprises combining at least one CSSC-miscible / polar-carrier-insoluble active agent with the CSSC(s) and water-insoluble fatty amine(s) (including the optional non-volatile liquid(s) and/or surfactant(s) if present), said combination being performed a) whilst mixing the CSSC with a water-insoluble fatty amine and, if applicable, a non-volatile liquid and/or at least one surfactant; b) by mixing the CSSC-miscible / polar-carrier-insoluble active agent with the plasticized CSSC (when applicable) prior to combining it with the polar carrier; or c) whilst mixing the CSSC (optionally plasticized), previously combined with the fatty amine, with the polar carrier or nano-sizing the compositions components, the polar-carrier-insoluble active agent(s) being either in the nano-elements (if added as in a) or b)) or separately dispersed in the polar liquid phase (if added as in c)).

In some cases, the method further comprises adding at least one polar-carrier-soluble active agent to the nano-suspension of core-shell nano-elements obtained in step d) and mixing the two, whereby a second shell, comprising the carrier-soluble active agent, is formed on the surface of the core-shell nano-elements. Such nano-elements can be referred to as active agent-coated core-shell nano-elements, core-shells nano-elements, or core-multi-shells nano-elements. The addition of such active agents can be performed at various steps during the preparation of the composition, depending on the resistance of the active agent to temperatures, mixing or shearing conditions applied at the envisioned step. Relatively resistant active agents can be added a) whilst combining with the polar carrier, the CSSC and fatty amine mixture (optionally including additional CSSC-miscible ingredients, such as a non-volatile liquid); or b) whilst nano-sizing the compositions components so as to obtain the core-shell nano-elements including the CSSC and water-insoluble fatty amine. Alternatively, the active agent(s) which are soluble in the polar carrier, in particular if shear-sensitive, can be added to the obtained nano-suspension, relatively heat-sensitive agents being preferably combined with the nano-emulsion or nano-dispersion after cooling.

In some cases, the formation of the second shell requires the core-shell nano-elements to be positively charged, so that a polar-carrier-soluble active agent being negatively charged can be electrostatically attracted to them, arranging around the core-shell nano-elements and engulfing them.

When the core-shell nano-elements have a negative charge or an insufficiently positive charge in the polar carrier to attract compounds of an opposite charge due to form a second shell, the method may further comprise adding a pH-modifying agent, in an amount suitable to increase the charge of the core-shell nano-elements (e.g., raising a positive charge), while retaining the negative charge of the carrier-soluble active agent. Depending on the functional groups present on the fatty amine molecules of the first shell, the pH-modifying agent may be either an acid or a base, an acidic pH-modifying agent being generally preferred.

Regardless of the number of shells present on or to be formed on a core, it may be desirable to modulate the charge of the nano-elements in the dermatological compositions according to the present teachings. Without wishing to be bound by any particular theory, the charge of the nano-elements is expected to affect their distribution, hence their relative efficacy, across the skin. It is believed that nano-elements being relatively more positive tend to remain on or near the skin surface, whereas nano-elements being relatively less positive or more negative may have a relatively higher tendency to penetrate skin layers towards its lowermost boundary.

The charge of the nano-elements can depend on the materials from which they are constituted and *inter alia* from the pH conditions to which they are subjected. In some embodiments, a pH-modifying agent is added in an amount resulting in the composition having a pH between 1 and 10, between 1 and 9, between 1 and 8, between 2 and 7, or between 2 and 5. In particular embodiments, the pH modifying agent is added in an amount leading the composition to have a pH of 7 and less, 6 or less, 5 or less, or 4 or less.

While pH-modifying agents are typically added to the liquid carrier in which the nano-elements are dispersed, they may alternatively, or additionally, be added to the nano-elements, for instance incorporated into their core, regardless of the number of shells that may surround them. pH-modifying agents suitable for incorporation into the core, from which they would desirably leach out in due time, should be miscible and compatible therewith.

In some cases, the method further comprises combining a first active agent, being miscible with the CSSC and insoluble in the polar carrier, with the CSSC and fatty amine, the combination being performed as aforesaid, and adding to the polar carrier a second active agent, being carrier-soluble, as aforesaid, the composition prepared thereby including a first active agent in the core of the nano-elements containing the CSSC and a second active agent forming the second shell of the nano-elements surrounding the first shell of fatty amines.

In some cases, the method further comprises adding a skin-penetration enhancer to the polar carrier phase of the nano-suspension. The addition of such a skin-penetration enhancer can be performed at various steps during the preparation of a dermatological composition, and typically as above described for the sake of incorporating an active agent soluble in the polar carrier.

In some cases, the CSSC, the water-insoluble fatty amines, the polar carrier, and if desired for the preparation of the composition, the non-volatile liquid, the surfactant, the skin-penetration enhancer, the carrier-insoluble active agent and the carrier-soluble active agent that can be used in the present method, are substantially as described above and herein detailed.

It is noted in this context that while compounds have been for simplicity categorized according to their main role in the present invention, in particular with respect to the preparation methods, such functions are not exclusive one of the other. For illustration, a fatty amine, typically serving to create the first shell, may also be capable of plasticizing the CSSC, and the fatty amine and/or the non-volatile liquid may also serve as a surfactant for the nano-elements. A polar carrier (*e.g*., glycols), serving as liquid medium for the dispersed nano-elements, or a surfactant (*e.g*., urea) intended to increase the dispersibility of the nano-elements, may additionally serve as a skin-penetration enhancer once the composition is applied on the skin. The predominance of one role over the other may depend upon a material inherent potency in the respective fields, but also on the relative presence of the material in the composition. For instance, a material deemed a carrier if constituting a significant enough part (*e.g.,* more than 20 wt.%) of the liquid phase, may be considered to fulfil a distinct function if in a relatively lower amount, such amount being more adapted to its secondary roles.

In some embodiments, the dermatological compositions of the present invention can be prepared according to the methods herein disclosed and may further contain any additive conventionally present in such compositions.

While for the sake of illustration, the present compositions and methods are being described with respect to dermatological compositions intended for activities of relevance to the skin, such as being a product improving skin appearance and/or health, this should not be construed as limiting. The dermatological compositions according to the present teachings may additionally serve to transdermally deliver active agents targeting organs other than skin or disorders other than skin-specific, or they may serve to protect the skin from external factors. Such additional pharmaceutical agents can also be incorporated in the present compositions, either in the core of the nano-elements if relatively water-insoluble, or in the shell(s) if relatively water-soluble. Thus, the present dermatological compositions can be more broadly considered for the preparation of a veterinary product for the treatment of a non-human animal or a medicament for the treatment of a human subject.

Furthermore, the surfaces upon which the present compositions can be applied are not limited to the skin of a living subject. Considering that in some embodiments the present composition can serve to deliver an active agent by releasing it from the cores of its core-shell nano-elements, the surfaces to be treated therewith can additionally be of inert objects or can be the environment or the surfaces (other than skin) of living subjects. For illustration of the former case, the composition can be applied to surfaces of buildings (or their inert contents, such as furniture or textiles), the compositions for example serving to release a pesticide over time. In such a case, the compositions can be considered industrial compositions.

Alternatively, the active agent to be released from the present compositions can be an agrochemical agent, in which instance the treatable surface can be the outer surface of a plant or a pest, or the soil or the water in which plants are growing or pests are present, and the composition can be considered an agrochemical composition. Such agents may be further used to protect inanimate surfaces, for industrial use.

All compositions, regardless of end-use, and all surfaces treatable therewith in accordance with the active agent present in the cores and/or on the shells of the core-shell nano-elements, are encompassed.

In a further aspect of the disclosure, there are provided uses according to claim 15 for the present compositions, which by way of introduction can be for the preparation of a cosmetic product, an agrochemical product, an industrial product, a veterinary product, or a medicament for the treatment of a human subject, the composition being applied externally to a surface to be treated therewith, the composition comprising core-shell nano-elements dispersed in a polar carrier, according to the present teachings including the aspects and embodiments herein described.

In some embodiments, the product is a cosmetic product, the surface to be treated therewith is a skin and the composition is a cosmetical composition for achieving a cosmetic effect for improving skin appearance (as *inter alia* resulting from stimulating the neo-synthesis of skin structural proteins, such as collagen, elastin or glycosaminoglycans, and/or for preventing their degradation) and/or for treating signs of ageing of the skin, combating wrinkles and fine lines, combating wizened skin, combating flaccid skin, combating thinned skin, combating dull, lifeless skin, combatting uneven skin pigmentation, or combating lack of elasticity and/or tonicity of the skin, and any like skin condition in which the CSSCs and/or the active agents are known to have a beneficial cosmetic activity. The cosmetic effects may be derived from the presence of a cosmetically active agent in the core-shell nano-elements, the visibly detectable activity being for illustration of skin bleaching or tanning. Such cosmetical products and uses thereof are generally intended for application to the skin of a human subject.

In other embodiments, the product is a veterinary product or a medicament, the surface to be treated therewith is respectively a skin of a non-human animal or of a human subject and the composition is a pharmaceutical composition for achieving a pharmaceutic effect. The pharmaceutical effect consists of improving skin health (and in turn often skin appearance as well), the effect including, by way of non-limiting examples, at least one of treating chronic skin disorders, skin lesions, restoring skin integrity (optionally accelerating or providing for cutaneous reinnervation and/or revascularization), promoting wounds or surgical incisions healing, facilitating skin grafting, alleviating or treating local skin inflammation, skin irritation, skin infection, skin rashes, skin pain, skin burns, skin swelling and/or skin allergy; promoting or inhibiting hair growth or dandruff; and any like condition directly or indirectly affecting the skin in which the CSSCs and/or the active agents are known to have a beneficial therapeutic activity.

In alternative embodiments, the skin is not being treated *per se* but serves as substrate, the core-shell nano-elements including agents protecting the skin from external factors, whether living, physical or chemical. For illustration, the nano-elements may include insect repellents, UV-filters or nerve agents' protectors. Such agents may be considered pharmaceutic agents.

The present pharmaceutical compositions may also be used to treat organs other than the skin, while being delivered through the skin. In such a case, the core-shell nano-elements typically further contain an active agent adapted to delay, alleviate, prevent or treat a transdermally treatable disorder. In some embodiments, the effect that can be achieved according to the active agent present in the pharmaceutical composition can be, for instance, an ADHD-relieving effect, a neuroprotective effect for the treatment of neurodegenerative diseases or their symptoms (*e.g*., the active agent being effective against Amyotrophic Lateral Sclerosis (ALS), Alzheimer's disease, Huntington's disease, Parkinson's disease, and like disorders of the nervous system), an analgetic effect, an anthelmintic effect, an anti-addiction withdrawal effect, an anti-anginal effect, an anti-arthritic effect, an anti-depressant effect, an anti-emetic effect, an anti-psychotic effect, anti-rheumatic effect, an anti-seizure effect, an antispasmodic effect, a blood pressure regulating effect, a contraceptive effect, a glaucoma relieving effect, a hormone replacement effect, a thyroid regulating effect and a protective effect from nerve agents poisoning.

The skin upon which the cosmetic or pharmaceutical composition can be applied may be found in any relevant part of the body, such as on the face, scalp, trunk, or limbs.

In other embodiments, the product is an agrochemical and/or an industrial product, the surface to be treated therewith is the surface of an inert object, a plant or a pest, or the soil or the water in which plants are growing or pests are present, and the composition is an agrochemical composition or an industrial composition. The effect that can be achieved by these compositions can be as an acaricide, an algicide, an anthelmintic, an anti-moth, an avicide, a bactericide, a chemo-sterilant, a fertilizer, a fungicide, a herbicide, an insecticide including an ovicide, an insect repellent, an insect pheromone, a molluscicide, a nematicide, a nitrification inhibitor, a pesticide, a pest repellent, a plant growth promotor, a rodenticide, a soil fertilizer, a termicide, a virucide, and a plant wound protectant.

Additional objects, features and advantages of the disclosure will be set forth in the detailed description which follows, and in part will be readily apparent to those skilled in the art from the description or recognized by practicing the disclosure as described in the written description and claims hereof, as well as the appended drawings. Various features and sub-combinations of embodiments of the disclosure may be employed without reference to other features and sub-combinations.

### BRIEF DESCRIPTION OF THE DRAWINGS

Some embodiments of the disclosure will now be described further, by way of example, with reference to the accompanying figures, where like reference numerals or characters indicate corresponding or like components. The description, together with the figures, makes apparent to a person having ordinary skill in the art how some embodiments of the disclosure may be practiced. The figures are for the purpose of illustrative discussion and no attempt is made to show structural details of an embodiment in more detail than is necessary for a fundamental understanding of the disclosure. For the sake of clarity and convenience of presentation, some objects depicted in the figures are not necessarily shown to scale.
In the Figures:
Figure 1 depicts a simplified schematic diagram of a method for preparing core-shell nano-elements according to embodiments of the present teachings;
Figure 2 shows the particle size distribution of core-shell nano-elements prepared according to the present teachings, as measured by dynamic light scattering (DLS) and presented per number;
Figure 3 schematically illustrates an uncharged core-shell nano-element in a non-aqueous polar carrier;
Figure 4 schematically illustrates a positively charged core-shell nano-element, surrounded by a shell formed of molecules of an active agent;
Figure 5A shows a CryoTEM image of a nano-dispersion prepared according to the present teachings, comprising core-shell nano-particles surrounded by a second shell of a carrier-soluble active agent;
Figure 5B shows a CryoTEM image of a nano-dispersion comprising core-shell nano-particles surrounded by a second shell which have undergone partial coalescence;
Figure 6A is a histological picture taken with a fluorescence microscope at a magnification of x10 of a cross-section of pig ear skin treated with a dermatological composition according to the present teachings, the tested sample comprising core-shell nano-elements including a fluorescent marker within their core;
Figure 6B is a histological picture of a cross-section of pig ear skin treated with a dermatological composition according to the present teachings, the tested sample comprising core-multi-shell nano-elements including a fluorescent marker within their core, the picture being taken similarly as for Figure 6A;
Figure 6C is a histological picture of a cross-section of pig ear skin treated with a dermatological composition according to the present teachings, the tested sample comprising core-multi-shell nano-elements including a fluorescent marker within their core, the picture being taken at a magnification of x20; and
Figure 6D is a histopathological picture of a cross-section of pig ear skin treated with a control composition lacking a fluorescent marker, the picture being taken similarly as for Figures 6A and 6B.

### DETAILED DESCRIPTION

The present invention relates to (*e.g*., dermatological) compositions comprising core-shell nano-elements, *e.g*., nano-particles or nano-droplets, comprising a core composed of a water-insoluble compound capable of stimulating neo-synthesis of structural skin proteins, such as collagen, elastin or glycosaminoglycans, and/or of inhibiting processes leading to their degradation (in short, a CSSC or a CSSP), and a shell of at least one fatty amine surrounding the core, the fatty-amine(s) non-covalently interacting with the CSSC, the nano-elements being dispersed as nano-suspension in a polar carrier. Advantageously, the CSSC may have a molecular weight of 0.6 kDa or more and can be, if desired, plasticized by or swelled with a non-volatile liquid, which can also be referred to as a plasticizing or swelling agent. The core-shell nano-elements may by themselves provide for a desired dermatological effect, either as a result of the activity of the CSSC itself, or as a result of its combination with a carrier-insoluble active agent in the core. The nano-elements may alternatively or additionally be used as an anchor or nano-carriers for other cosmetic or pharmaceutical active agents, which are carrier-soluble and can form a second shell on the surface of the nano-elements, the second shell surrounding the first shell of fatty amine(s).

When applied to the skin, the nano-elements comprised in the composition can remain on the skin surface or penetrate the skin barrier to a varying extent and provide, *inter alia,* an anti-wrinkle effect or any such effect restoring skin look, contributed at least in part thanks to the capacity of the CSSC in the cores of the nano-elements to maintain sufficient collagen presence in the skin. If an active agent added to be part of the core and/or to form a second shell is selected to be a cosmetic agent having a cosmetic effect similar to the core-shell, then the initial cosmetic effect that the sole CSSC might have provided can be enhanced (*e.g*., boosting an anti-wrinkle effect). However, a cosmetic agent of a core (if carrier-insoluble) or of a second shell (if carrier-soluble) may provide for a different cosmetic effect, the core-shell(s) nano-elements combining the effect of the core CSSC and of the cosmetic agent (*e.g.,* of the second shell). As mentioned, the active agent may alternatively serve a more curative goal and can similarly be added as part of the core or as a second shell.

The compositions may include any other desired compound, such as any material miscible with the cores or any of the shells of the core-shell(s) nano-elements to enable or increase the dispersibility of the nano-elements in the composition, to modulate their charge or any other property adapted to increase its stability over time and/or to further enhance or modify the biological activity of the composition. Surfactant(s), activity modifying agent(s) and/or skin permeation enhancer(s) can be present in the polar carrier, if soluble therein. Methods for preparing such compositions and uses thereof (*e.g.,* for cosmetical or pharmaceutical effects) are also disclosed.

Before explaining at least one embodiment in detail, it is to be understood that the disclosure is not necessarily limited in its application to the details of construction and the arrangement of the components and/or methods set forth herein. The disclosure is capable of other embodiments or of being practiced or carried out in various ways. The phraseology and terminology employed herein are for descriptive purpose and should not be regarded as limiting.

It is to be understood that both the foregoing general description and the following detailed description, including the materials, methods and examples, are merely exemplary of the disclosure, and are intended to provide an overview or framework to understanding the nature and character of the invention, and are not intended to be necessarily limiting.

### Biological activity of CSSCs

The CSSCs that can be used in the present invention are selected for their ability to promote *inter alia* collagen formation within the skin and/or to prevent its degradation (or of any other skin structural protein). Without wishing to be bound by any particular theory, it is believed that such compounds, upon their application and penetration into the skin, or their injection therein, can trigger biological signals culminating in neo-synthesis of skin structural proteins. Such compounds may be biodegradable, being for instance biodegradable polymers, in which case the CSSCs can be broken down by certain biological mechanisms and cause local inflammation. This process may induce the formation of skin proteins such as collagen, for the purpose of healing the inflamed area, this newly synthesized collagen also contributing to the firmness of the skin. Alternatively, the CSSCs may be non-biodegradable, triggering a similar effect of skin protein (*e.g*., collagen) formation or protection, and consequent healing of the treated area, without having to undergo compound degradation.

In view of their intended use, the CSSCs are preferably biocompatible and typically biodegradable in a physiological environment, such as found following their application to the skin and their transdermal delivery. Suitable CSSCs can also be termed bioresorbable or bioabsorbable in the general literature, depending on their *in vivo* fate and prospective elimination from the body, but for simplicity all such compounds will be generally referred to herein as "biodegradable". A CSSC is said to be biodegradable if breaking down relatively rapidly after fulfilling its purpose (*e.g.,* by a bacterial decomposition process in the environment or by an *in vivo* enzymatic or metabolic process) to result in natural by-products. Biodegradable CSSCs are known, and new ones are being developed. Their relative biodegradability in various environments can be assessed by a number of methods, which depending on the conditions of interest can be procedures based on or modified from standards such as ASTM F1635.

Despite its propensity to naturally decompose under suitable physiological conditions, a biodegradable CSSC adapted to the present invention should be stable and durable enough for its intended use during storage and application, which can be particularly challenging if this use involves conditions that would enhance biodegradability. For example, spreading topical compositions containing CSSCs as a thin layer on the skin is expected to form a high surface area, which might increase the exposure of the resulting film to factors (*e.g.,* light, chemicals, or micro-organisms) promoting the degradation of the CSSC before it is able to penetrate the skin and reach its target, hence, the selection of suitable CSSCs for the dermatological (*e.g*., topical) compositions of the present invention should take these factors into consideration.

As the compounds encompassed by the term CSSCs may additionally serve to deliver or release on surfaces other than the skin active agents of relevance to non-dermatological (*e.g*., agrochemical or industrial) products, it is appreciated that no actual promotion of synthesis of skin structural proteins are expected in such context. Then the term CSSC should be more generally understood to refer to a thermoplastic compound fulfilling the characteristics herein described in the context of CSSCs more specifically adapted to the skin.

### Insolubility

Besides their possible biodegradability, the CSSCs are preferably substantially non-soluble in the liquid phase of the composition including the polar carrier (*e.g*., water), in which they are dispersed as nano-elements.

As used herein, the solubility of a material (*e.g*., a CSSC, a fatty amine, a non-volatile liquid, or an active agent) refers to the amount of such component that can be introduced into the liquid (*e.g.,* polar) carrier, while maintaining the clarity of the liquid medium. The solubilities of specific components of the composition within any particular liquid are typically assessed in the sole polar carrier in absence of any other possible components of the compositions but may be alternatively determined with respect to the final composition of the liquid phase including the carrier.

CSSCs (or any other material of interest for the present invention) are deemed insoluble if their solubility in the polar carrier, or in the liquid phase containing it, is 5 wt.% or less, 4 wt.% or less, 3 wt.% or less, 2 wt.% or less, 1 wt.% or less, 0.5 wt.% or less, or 0.1 wt.% or less by weight of the carrier, or of the liquid phase. For illustration, no more than 5 g of a material that is non-soluble in a polar carrier would dissolve in 100 g of the carrier. This substantial insolubility, while typically measured at room temperature, should preferably apply at any temperature at which these ingredients are combined and processed, *i.e*., even at relatively elevated temperatures, the solubility of these compounds in the polar carrier should remain within the required ranges. A material satisfying these conditions can be referred to as a "polar-carrier-insoluble" material.

Such insolubility of the material is expected to prevent or reduce leaching out into their surrounding media of one or more of the CSSC (or of any other one of the constituents of the cores of the nano-elements). Such leaching out, were the material soluble in the polar carrier, may affect the relative proportions of the constituents of the nano-elements, their size, or any other such parameter that may ultimately adversely affect the efficacy of the composition.

Regardless of the composition of the polar liquid phase including the polar carrier in which the core-shell nano-elements are to be dispersed, the CSSC can first be characterized as being water-insoluble (*i.e.,* having a solubility of less than 5 wt.% in water as typically established at room temperature).

### Molecular weight

Advantageously, the present invention allows for the delivery of core-shell nano-elements comprising CSSCs having relatively high molecular weights as compared to compounds that may conventionally sufficiently penetrate the skin barrier to display any efficacy. CSSCs suitable for the present compositions, methods, and uses can have a molecular weight (MW) of 0.6 kDa or more, 0.7 kDa or more, 0.8 kDa or more, 0.9 kDa or more, 1 kDa or more, CSSPs also displaying MW of 2 kDa or more, 5 kDa or more, or 10 kDa or more. Typically, their molecular weight does not exceed 2 kDa if the compound is not a polymer, though polymerizable compounds may be larger, CSSPs reaching MWs of up to 500 kDa, and being generally of 300 kDa or less, 200 kDa or less, 100 kDa or less, 80 kDa or less, 50 kDa or less, 25 kDa or less, or of 15 kDa or less. In another embodiment, the molecular weight of the CSSCs is between 0.6 kDa and 500 kDa, between 0.7 kDa and 300 kDa, between 0.8 kDa and 200 kDa, between 1 kDa and 100 kDa, or between 2 kDa and 80 kDa.

As used herein, the term "molecular weight" (or "MW") refers either to the actual molecular weight as can be calculated for a non-polymeric CSSC, which can also be expressed in grams/mole, or to the weight average MW of polymerizable CSSCs or CSSPs, which may be a blend of polymers each containing a slightly different number of repeating units, weight average MW of polymers being typically expressed in Daltons.

The molecular weight of the CSSCs can be provided by their suppliers and can be independently determined by standard methods including for instance gel permeation chromatography, high pressure liquid chromatography (HPLC), size-exclusion chromatography, light scattering or matrix-assisted laser desorption/ionization time-of-flight mass spectroscopy MALDI-TOF MS, some of these methods are described in ASTM D4001 or ISO 16014-3.

Such general rules concerning the molecular weight exemplified with respect to the CSSCs can be applied to any other constituent of the composition, whether polymeric or not, and in some embodiments, active agents added to the cores and/or the shells of the nano-elements may also be relatively large molecules having a MW of 0.6 kDa or more, and any other values as specifically recited for the CSSCs.

### Characterizing temperatures

While the vast majority of non-polymeric compounds can be characterized by a melting temperature (***Tm***) at which they change from a solid phase to a liquid one, polymeric compounds can additionally or alternatively be defined by a glass transition temperature (***Tg***) if amorphous, pure amorphous polymers lacking a ***Tm.*** Pure crystalline polymers can be characterized by their ***Tm,*** semi-crystalline polymers often displaying two characterizing temperatures (*e.g*., ***Tg*** and ***Tm***) reflecting the respective proportion of amorphous and crystalline parts in the molecule. Such polymers may also be defined by their softening temperature (***Ts***) midway the log step to melting. As the glass transition temperature describes the transition of a glass state into a rubbery state, and the softening temperature an intermediate inflection in the thermal analysis of a material, they typically relate to a range of temperatures or one at which the process will first be observed.

Therefore, depending on the chemical nature of the CSSC, the temperature that may characterize its thermal behavior can be at least one of a ***Tm,*** a ***Ts*** and a ***Tg.*** Hence, when a CSSC (or nano-elements) is defined as suitably having at least one of a first (or second) ***Tm*, *Ts*** and ***Tg*** within a particular range, the temperature considered is as relevant to the material. Some compounds may be identified by two such characterizing temperatures, in which case performing a method step at a temperature above any of the two temperatures could be above the lowest of the two (which would prolong the step) or the highest of the two (which would accelerate the step). Conversely, performing a method step at a temperature below any of the two temperatures could be below the highest of the two or the lowest of the two. Taking for illustration a semi-crystalline polymer that can be characterized by all three temperatures, ***Tm, Ts,*** and ***Tg*** in order of decreasing values, heating above ***Tg*** (*i.e*., above at least one), might be insufficient to reach ***Ts*** or ***Tm,*** while heating above ***Ts*** (*i.e*., above at least two), might be insufficient to reach ***Tm.*** Only heating above ***Tm*** would ensure that the temperature of heating is higher than all three temperatures that may characterize such exemplary polymer.

In some embodiments, the CSSCs suitable for the present compositions are characterized by at least one of a first (native) melting temperature (***Tm***), softening temperature (***Ts***) or glass transition temperature (***Tg***) being of at most 300°C, at most 250°C, at most 200°C, at most 180°C, at most 150°C, or at most 120°C.

In some embodiments, the first ***Tm*** of the CSSCs is at least 0°C, at least 10°C, at least 20°C, at least 30°C, at least 40°C, at least 50°C, or at least 60°C. In some embodiments, the first ***Tm*** of the CSSCs is between 0°C and 300°C, between 10°C and 300°C, between 20°C and 300°C, between 20°C and 250°C, between 20°C and 200°C, between 30°C and 180°C, between 40°C and 150°C, or between 50°C and 120°C.

In some embodiments, the CSSCs are characterized by at least one of a first ***Ts*** and a first ***Tg*** being of -75°C or more, -50°C or more, -25°C or more, 0°C or more, 25°C or more, or 50°C or more. In some embodiments, at least one of the first ***Ts*** and ***Tg*** of the CSSCs is between -75°C and 300°C, between -50°C and 250°C, between -25°C and 200°C, between 0°C and 180°C, between 20°C and 180°C, or between 30°C and 150°C.

The characterizing temperatures (***Tm*, *Ts*** or ***Tg***) of a CSSC may be referred to as a "first" ***Tm, Ts*** or ***Tg,*** when relating to the native / unmodified compound, and may be referred to as a "second" ***Tm, Ts*** or ***Tg,*** when relating to the CSSC as modified to constitute the cores of the present core-shell nano-elements. In some embodiments, the second ***Tm, Ts*** or ***Tg*** that can be measured on the blend obtained upon mixing of the CSSC with a fatty amine, and optionally with a non-volatile liquid or any other desirable ingredient and/or on the core-shell nano-elements containing the same, is similar to the first respective ***Tm, Ts*** or ***Tg*** of the CSSC. Alternatively, the second ***Tm, Ts*** or ***Tg*** can be lower than the respective first characterizing temperature(s) of the CSSC, in which case the CSSC is considered plasticized or swelled.

Such general rules concerning a suitable thermal behavior exemplified with respect to the CSSCs can be applied to any other constituent of the cores of the nano-elements which may display or affect a characterizing temperature as herein described. Hence, in some embodiments a constituent of the core (other than the CSSCs) either alone or in combination with the CSSCs and all other materials due to form the core (*e.g.,* active agents, surfactants, *etc.*) may satisfy the aforesaid ranges. In other words, the aforesaid said thermal properties of the bulk, native, CSSCs (*e.g.,* a first ***Tm,*** a first ***Ts,*** or a first ***Tg*** and the values they should preferably satisfy) can be read to similarly apply to the core-shell nano-elements and their respective second ***Tm,*** second ***Ts*** or second ***Tg*.**

Such thermal characteristics of a CSSC or any other ingredient can be provided by its manufacturer or can be independently determined by standard methods, in particular for the core-shell nano-elements. Thermal analysis methods, *e.g*., Differential Scanning Calorimetry (DSC), are described, for instance, in ASTM 3418, ISO 3146, ASTM D1525, ISO 11357-3, or ASTM E1356. Such measurements may be performed on the raw materials (*e.g*., bulk CSSC) or on intermediate (*e.g.,* a mix) or end-products (*e.g.,* the nano-elements) containing the CSSC. For this purpose, the core-shell nano-elements containing the CSSC may be isolated from the polar carrier and other agents present therein. The separation of the nano-elements from their liquid medium can be performed according to standards methods, such as by evaporation of the carrier by drying (*e.g.,* in a vacuum oven) or freeze-drying, or by destabilization of the composition (*e.g*., by changing the pH) allowing the precipitation of the nano-elements. Nano-elements isolated by any suitable method from their samples can be additionally rinsed (*e.g.,* with water) to remove residues that may affect the intended measurements, the nano-elements being then separated from the rinsing liquid. For instance, nano-elements precipitated out of their initial medium can be separated by centrifugation, the precipitate can be rinsed and centrifuged again in cycles until isolated and desirably rinsed nano-elements are obtained.

### Polymeric and non-polymeric CSSCs

In some embodiments, the collagen-synthesis stimulating compounds (CSSCs) used in the present compositions, methods and uses are collagen-synthesis stimulating polymers (CSSPs). As the CSSPs are desirably adapted for biodegradation once applied on their target surface, such as being delivered into the physiological environment of the skin (*e.g.,* on, within or beneath it), such polymers generally contain hydrolysable or enzymatically cleavable sites. The presence of hydrolysable or otherwise cleavable sites is however non-essential, and polymers considered non-biodegradable may lack them.

In some embodiments, the CSSCs (or CSSPs) may be non-reactive to build up more complex interactions, being only able to biodegrade, such as PCL. In other embodiments, the CSSCs (or CSSPs) may be biodegradable and have reactive moieties enabling interactions with additional different molecules, such as PLA, or additional same molecules, such as polymerizable natural resins having for instance aldehyde moieties.

Similarly, when the CSSCs are non-biodegradable thermoplastic compounds, such materials can also be reactive or not. Non-reactive non-biodegradable synthetic thermoplastic polymers can be for illustration polyethylene or polypropylene polymers, while polymers containing reactive groups can be *e.g.,* ethylene-acrylic acid or ethylene-methacrylic acid copolymers.

Suitable CSSPs, which can be of natural or synthetic origin, are thermoplastic in nature, their shapes being capable of reversible modifications upon suitable heating and cooling. Appropriate CSSPs can also be plasticized with a suitable non-volatile liquid, such optional treatment of the CSSPs facilitating their nano-sizing to an extent expediting transdermal delivery of the dispersed core-shell nano-elements, when this is their intended use.

Synthetic CSSPs can be biodegradable and selected from aliphatic polyesters, polyhydroxy-alkanoates, poly(alkene dicarboxylates), polycarbonates, aliphatic-aromatic co-polyesters, enantiomers thereof, copolymers thereof and combinations thereof.

To the extent that the monomers forming the CSSPs have chiral centers, all enantiomers and stereoisomers are encompassed. For illustration, lactic acid (2-hydroxypropionic acid, LA), exists as two enantiomers, L- and D-lactic acid, so that PLA has stereoisomers, such as poly(L-lactide) (PLLA), poly(D-lactide) (PDLA), and poly(DL-lactide) (PDLLA). A CSSP may therefore be a mixture of isomers of a same molecule or a specific stereoisomer (or a stereo copolymer).

In some embodiments, the CSSP is biodegradable and selected from a group comprising: aliphatic polyesters, such as poly-caprolactone (PCL), polylactic acid (PLA), poly(L-lactide) (PLLA), poly(D-lactide) (PDLA), poly(D,L-lactide) (PDLLA), polyglycolic acid (PGA), poly(lactic-co-glycolic acid) (PLGA), and poly(p-dioxanone) (PPDO); polyhydroxy-alkanoates (PHA), including polyhydroxybutyrate (PHB) (such as poly-3-hydroxy-butyrate (P3HB), poly-4-hydroxy-butyrate (P4HB), poly(3-hydroxybutyrate-co-3-hydroxyvalerate) (PHBV), poly-hydroxyvalerate (PHV), polyhydroxyhexanoate (PHH), and polyhydroxyoctanoate (PHO); poly(alkene dicarboxylates), such as poly(butylene succinate) (PBS), poly(butylene succinate-co-adipate) (PBSA) and poly(ethylene succinate) (PES); polycarbonates, such as poly-(trimethylene carbonate) (PTMC), poly(propylene carbonate) (PPC) and poly-[oligo(tetramethylene succinate)-co(tetramethylene carbonate)]; aliphatic-aromatic co-polyesters, such as poly(ethylene terephtalate) (PET) and poly(butylene adipate-co-terephtalate) (PBAT); their isomers, copolymers and combinations thereof.

In a particular embodiment, the biodegradable CSSP is or includes an aliphatic polyester, isomers, copolymers and combinations thereof. In a further particular embodiment, the CSSP is PCL. In another further particular embodiment, the CSSP is PLA. In a further particular embodiment, the CSSP is PLGA. In another further particular embodiment, the CSSP is PBSA.

Natural biodegradable CSSPs can be selected from polysaccharides (such as: cellulose, starch, chitin and chitosan), lignin and combinations thereof.

In some embodiments, the CSSP is non-biodegradable and selected from polyamide (PA), polyethylene (PE), poly(ethylene-co-acrylic acid) (PEAA), poly(ethylene-co-methacrylic acid) (PEMAA), poly(ethylene-co-n-butyl acrylate) (PEBA), poly(ethylene-co-vinyl acetate) (PEVA), polymethylmethacrylate (PMMA), polypropylene (PP), polysiloxane, polystyrene (PS), polytetrafluoroethylene (PTFE), polyurethane (PU), or polyvinyl chloride (PVC). In a particular embodiment, the non-biodegradable CSSP is selected from PA, PE, PEAA, PEMAA, PEBA, PEVA, substituted or modified versions thereof, ionomers thereof and combinations thereof.

The above-mentioned polymers may be identified according to their respective characteristic functional groups as detectable by standard methods known to the skilled persons, for instance, by Fourier-transform infrared (FTIR) spectroscopy.

Non-polymeric CSSCs suitable for the compositions, methods and uses of the present invention can also be natural or synthetic. Under suitable conditions, some natural CSSCs, may react in a polymerization reaction, resulting in CSSPs.

Natural CSSCs that may be polymerizable can be selected from: natural resins (such as: shellac, rosin, damar, copal, sandarac, amber, mastic and manila); natural gums (either originating from gum-yielding trees, such as Acacia nilotica (babul), Acacia catechu (khair), Steruculia urens (kullu), Anogeissus latifolia (dhawra), Butea monosperma (palas), Bauhinia retusa (semal), Lannea coromandelica (lendia) and Azadirachta indica (neem), or originating from seeds of plants such as guar, tamarind and Cassia tora); natural gum-resins (such as asafoetida, myrrh, salai and guggul); and combinations thereof. In a particular embodiment, the natural polymerizable CSSC is shellac or gum rosin.

Non-polymerizable CSSCs include quinones. In a particular embodiment, the non-polymeric CSSC is coenzyme Q10 (CoQ10).

Additionally, a CSSC can be a blend of different compounds, whether polymeric, polymerizable or not, the properties of the mixture (*e.g*., a characterizing temperature, a viscosity, *etc.*) satisfying the ranges set for a suitable individual compound. For instance, a CSSC or CSSP having a ***Tm, Ts*** or ***Tg*** out of a range previously deemed suitable (*e.g.,* being lower than 20°C or higher than 300°C) may be combined with a CSSC or CSSP having a ***Tm, Ts*** or ***Tg*** adapted to "correct" the characterizing temperature of the obtained mixture to be suitable for the purpose of the present invention. For illustration, a CSSC can be a blend of polymers or a copolymer including at least one of the aforementioned CSSPs, such copolymers may contribute to the biocompatibility, biodegradability and mechanical and optical properties of the nano-elements.

The compositions of the present invention may contain core-shell nano-elements each prepared using various types of CSSCs. For instance, a composition may include nano-elements prepared using one type of CSSC combined with nano-elements prepared using a different type of CSSC. Alternatively, or additionally, the present compositions can contain core-shell nano-elements which differ in their function, for instance each type of nano-elements including a different optional active agent.

### Viscosity

CSSCs can alternatively (or additionally) be selected for their viscosity to be initially adapted to their shearing in the present methods for preparing the compositions. CSSCs suitable for the present methods can typically have a viscosity which does not exceed of 10¹¹ millipascal-second (mPa·s, being equivalent to a centipoise), and which is often of 5x10¹⁰ mPa·s or less, 10¹⁰ mPa·s or less, 5x10⁹ mPa·s or less, 10⁹ mPa·s or less, 5x10⁸ mPa·s or less, 10⁸ mPa·s or less, 5x10⁷ mPa·s or less, 10⁷ mPa·s or less, or of 5x10⁶ mPa·s or less, as determined at a temperature of 50°C and a shear rate of 10 sec⁻¹.

For efficient shearing to take place, the viscosity of the CSSCs should preferably be of 10⁷ mPa·s or less at a temperature of 50°C and a shear rate of 10 sec⁻¹. Such viscosity can relate to the native property of the isolated unmodified CSSC, in which case it can be referred to as a "first viscosity", or it may refer to the viscosity of the CSSC as modified by its mixing with materials miscible therewith, in which case it can be referred to as the "second viscosity" of the mixture containing the CSSC. For illustration, the second viscosity can be of a CSSP plasticized with a suitable plasticizing agent (*e.g.,* a non-volatile liquid and/or a fatty amine). The viscosity of a material (whether modified or not by the presence of others) at any temperature of interest (or in a range thereof) can be determined by routine thermo-rheological analysis, such as described in ASTM D3835 or ASTM D440.

The native viscosity of the CSSCs may be lowered by the fatty amine(s) (added to create the shell of the core-shell nano-elements), but a non-volatile liquid can alternatively be added to the CSSC or CSSP regardless of their native viscosity. Such dedicated plasticizing materials are typically used in the present compositions or methods when a reduction in viscosity more significant than optionally achieved by the sole presence of fatty amines is desired (*e.g*., when the CSSC has a relatively high first viscosity, such as higher than 10⁷ mPa·s, at 50°C and a shear rate of 10 sec⁻¹, the first viscosity being however optionally lower). The non-volatile liquid (which may also be referred to as a plasticizing liquid) is contained in the core of the nano-elements, the liquid being typically adsorbed or otherwise retained by the CSSC.

Such "plasticizing" or "swelling" typically results in weight gain and/or a volume gain relative to the CSSC own mass or volume in its native form. Such plasticizing of the CSSC renders the plasticized CSSC softer and more malleable, as demonstrated by its reduced viscosity (*i.e.,* the second viscosity being smaller than the first), facilitating their later nano-sizing (together with any other material mixed therewith).

Advantageously, the reduced viscosity should be adapted to the shearing process (e.g., shearing equipment, shearing temperature, *etc.*) being elected to nano-size the plasticized CSSC (*e.g.,* a plasticized CSSP) mixed with any other materials miscible in the core and the fatty amine(s) intended to form a first surrounding shell. For instance, the non-volatile liquid and its proportion relative to the CSSC can be selected to lower the viscosity of the CSSC by at least half-a-log, or at least one log, and so on, as might be required. For illustration, if the CSSC has a first viscosity of 10⁸ mPa·s, a plasticizing agent and its amount would enable a reduction of half-a-log if the CSSC so plasticized has a second viscosity of 5x10⁷ mPa·s, or (if in a higher amount or if alternatively selected to be a more potent agent) would enable a reduction of one log if the CSSC so plasticized has a second viscosity of 10⁷ mPa·s, as measured at a temperature of 50°C and a shear rate of 10 sec⁻¹.

In some embodiments, the first viscosity of the CSSC or the second viscosity of a plasticized CSSC (and/or of core-shell nano-elements comprising it) is between 10² mPa·s and 10⁷ mPa·s, between 5x10² mPa·s and 10⁶ mPa·s, between 5x10² mPa·s and 10⁵ mPa·s, between 10³ mPa·s and 5x10⁴ mPa·s, or between 10³ mPa·s and 10⁴ mPa·s, as measured at 50°C and at a shear rate of 10 sec⁻¹. Viscosity can be measured with any suitable rheometer equipped with a spindle adapted to the intended range of viscosities at the appropriate shear rate.

Viscosity, however, is not only relevant to the method of preparation of the core-shell nano-elements. It is believed that nano-elements displaying the aforesaid viscosities *(i.e.,* not exceeding 10⁷ mPa·s) are relatively malleable, such deformability being expected to expedite transdermal delivery of the core-shell nano-elements, when this is their intended use.

### Plasticization of CSSCs

While mentioned above for their effects on the viscosity of the CSSCs, to the extent reducing it would be desired, the non-volatile liquids that may be incorporated with the CSSC in the cores of the nano-elements may fulfil additional functions. Plasticizing in particular CSSPs can be visually observed when the swelled polymer is at a temperature below melting. At higher temperatures, the effect of the non-volatile liquid or of the fatty amines can be detected via their plasticizing activity, which includes the ability to lower at least one of the temperatures characterizing the native CSSCs.

Decreasing a characterizing temperature of the CSSC, allows accordingly lowering processing temperatures at which the compositions can be prepared. For illustration, while the CSSC can have a first (native) ***Tm, Ts*** or ***Tg*** of 200°C or less in absence of a suitable non-volatile liquid, the addition of such plasticizing agents may yield a plasticized CSSC having a second (modified) ***Tm, Ts*** or ***Tg,*** lower than the first, the second temperature being for instance of 95°C or less. The drop in temperature afforded by the presence of the plasticizing agent need not be as dramatic as illustrated, obviously depending on the value of the first ***Tm, Ts*** or ***Tg*** of the native CSSC, on the second ***Tm, Ts*** or ***Tg*** as may be desired to facilitate preparation of the composition and/or later penetration of the core-shell nano-elements, preferably on the boiling temperatures (***Tb***) of liquids which are to remain present in the composition (but not necessarily if the steps are brief enough and/or the liquids in excess in case some are boiled away), and/or on the concentration of the plasticizing agent with respect to the compound being plasticized.

Typically, the second ***Tm, Ts,*** or ***Tg*** of the plasticized CSSC (together with any other ingredients miscible with the CSSC) is lower than the respective first ***Tm, Ts,*** or ***Tg*** of the bulk CSSC, by at least 5°C, at least 10°C, at least 15°C, at least 20°C, at least 25°C, at least 30°C, at least 35°C, at least 40°C, at least 45°C, or at least 50°C.

A characterizing temperature of a CSSC may be satisfactorily reduced solely by the fatty amine(s) intended for the formation of the shell. Alternatively, a non-volatile liquid may be additionally combined with the CSSCs in order to further reduce at least one of their characterizing temperatures.

If the mixture of CSSC(s) and plasticizing agent(s) further comprises ingredients (*e.g*., rheological modifiers, surfactants, preservatives, or any like material miscible with the CSSC which may have a plasticizing effect) that may impact the softening property of the resulting combination due to form the core, or be within, the nano-elements, then additionally and alternatively, the thermal characteristics deemed suitable for the present invention would apply to the entire mixture.

Hence, in some embodiments, the at least one of a second ***Tm, Ts*** or ***Tg*** of the core-shell nano-elements is at most 290°C, at most 250°C, at most 200°C, at most 190°C, at most 180°C, at most 170°C, at most 150°C, or at most 120°C.

In some embodiments, the core-shell nano-elements comprising the CSSC in their cores have a second ***Tm*** of 0°C or more, 10°C or more, 20°C or more, 30°C or more, 40°C or more, 50°C or more, or 60°C or more. In some embodiments, the second ***Tm*** of the core-shell nano-elements is within a range of 0°C to 290°C, 10°C to 290°C, 20°C to 290°C, 10°C to 250°C, 20°C to 250°C, 20°C to 200°C, 30°C to 190°C, between 50°C and 170°C or between 50°C and 150°C. 30°C to 180°C, 40°C to 180°C, 40°C to 150°C, 50°C to 170°C, 50°C to 150°C, or 50°C to 120°C.

In some embodiments, the core-shell nano-elements have at least one of a second ***Ts*** and second ***Tg*** being -75°C or more, -50°C or more, -25°C of more, -20°C or more, -10°C or more, 0°C or more, 10°C or more, 20°C or more, 25°C of more, 30°C or more, 40°C or more, 50°C or more, or 60°C or more. In other embodiments, the at least one of the second ***Ts*** and ***Tg*** measured on the core-shell nano-elements is within a range of -75°C to 290°C, -50°C to 290°C, -25°C to 290°C, -20°C to 290°C, -10°C to 290°C, 0°C to 290°C, 10°C to 250°C, 20°C to 200°C, 30°C to 190°C, 30°C to 180°C, 40°C to 180°C, 50°C to 170°C, 50°C to 150°C, or 50°C to 120°C.

Such thermal behavior and characterizing temperatures can be assessed while preparing the plasticized CSSC or mixtures including the same, or upon completion of the preparation method of the composition, namely on the core-shell nano-elements that may be isolated therefrom.

### Non-volatile plasticizing liquids

While the role that the presence of non-volatile liquids may have in the efficacy of delivering the nano-elements including the CSSC core is not ignored, the selection of such materials is mainly considered with a view of improving the processability of the CSSC, so as to facilitate the preparation and dispersion of the core-shell nano-elements within the polar carrier phase. Particularly suitable non-volatile liquids can both lower the viscosity of the CSSC and lower at least one of its ***Tm, Ts*** and ***Tg,*** as previously separately discussed. Advantageously, suitable non-volatile liquids improve the processability of the CSSC under conditions suitable for its shearing into nano-particles, the shearing temperature causing initially the formation of nano-droplets.

First, as implied by their names, agents adapted to plasticize a CSSC according to the present teachings are liquid at the temperature at which the CSSC is to be processed, namely at least at one of the temperatures of mixing with the CSSC and of shearing. Such liquid agents can also be liquid at room temperature.

To ensure that their effect would perdure, the plasticizing liquids are preferably non-volatile. As used herein, the term "non-volatile", as can be used with regards to a liquid that may plasticize the CSSC, refers to liquids exhibiting a low vapor pressure, such as less than 40 Pascal (Pa, also Newton per square meter) at a temperature of about 20°C. In some embodiments, a non-volatile liquid (or any other ingredient for which a low volatility is preferred) can have a vapor pressure of 20 Pa or less, 5 Pa or less, 1 Pa or less, 0.1 Pa or less, or 0.01 Pa or less as measured at about 20°C. Such vapor pressure values are typically provided by the manufacturer of the liquid, but can be independently determined by standard methods, such as described in ASTM D2879, E1194, or E1782 according to the range of the vapor pressure. The low or substantially null volatility of the non-volatile liquids that may be used to plasticize a CSSC, if so desired, should preferably be maintained at the highest temperature at which the plasticized CSSC is processed. The use of such non-volatile liquids allows the CSSCs to remain in their plasticized or swelled state, without the risk of evaporation or elimination of the liquids, even at high temperatures of preparing the compositions according to the present methods.

Suitable non-volatile liquids are also characterized by having a boiling point (***Tbₗ***) that is higher than room temperature, higher than body temperature, and higher than an elevated temperature as may be desired for the preparation of the composition, as it is preferred that the liquids selected for plasticizing the CSSCs of the present invention do not substantially evaporate during or after the preparation of the compositions. That having been said, some boiling away may be tolerated if the mixing step at which the non-volatile liquids plasticize the CSSC is brief enough to ensure a residual presence as desired, and/or if the non-volatile liquids are added in sufficient excess to compensate for any partial boiling away that may take place.

For similar reasons of being desirably maintained with the CSSC to be plasticized therewith, and retained in the nano-elements comprising it, the non-polar liquid should preferably be unable to migrate to the polar carrier phase. Hence, suitable non-volatile liquids are essentially not miscible in such polar carriers (*e.g*., water), their solubility in the pure polar carrier or in the liquid phase containing it being as previously detailed for the CSSC, namely being of 5 wt.% or less, 4 wt.% or less, 3 wt.% or less, 2 wt.% or less, 1 wt.% or less, of 0.5 wt.% or less, or of 0.1 wt.% or less by weight of the carrier or the phase containing it.

Such non-volatile liquids need to be compatible with the CSSC of the composition (*i.e*., able to plasticize it: *e.g.,* decreasing its ***Tm, Ts*** or ***Tg,*** and/or decreasing its viscosity). A non-volatile liquid adapted for a particular CSSC can be selected accordingly by routine experimentation. For instance, given a particular CSSC, various non-volatile liquids can be mixed with it, at one or more relative concentrations, and optionally at an elevated temperature, facilitating the plasticizing, and their effects on the CSSC being plasticized monitored by thermo-rheology (for their ability to decrease viscosity as a function of temperature) and by thermal analysis (*e.g.,* by DSC, for their ability to decrease the ***Tm, Ts*** or ***Tg*** of the native CSSC). The non-volatile liquids most potent with respect to the particular CSSC can be selected accordingly.

Fundamentally, a material or a chemical composition is compatible with another if it does not prevent its activity or does not reduce it to an extent that would significantly affect the intended purpose. Such compatibility may be from a chemical standpoint, for instance, sharing similar functional chemical groups or each material having respective moieties that may desirably interact with one another. This kind of compatibility can be demonstrated by the combined materials forming a homogeneous mixture, rather than separate into different phases. A material would be incompatible with another if degrading it, and for illustration, a polar liquid phase would be incompatible with the nano-elements if dissolving them, destabilizing them, improperly charging them to have a charge incompatible with their intended use, and so on.

Materials should also be compatible with the methods used for the preparation of the composition, not being adversely affected by any of the steps the material would be subjected to in the process, nor being volatile (or otherwise eliminated) at the temperature(s) they are incorporated in the compositions. Understandingly, the materials need also be compatible with their intended use, which in the present case may include for illustration being biocompatible, non-irritating, non-immunogenic, and having any such characteristic providing for their regulatory approval at a concentration adapted for efficacious cosmetic or pharmaceutic compositions as herein-disclosed.

While compatibility is required between the CSSC and the non-volatile liquid, dissolution of the CSSC within the non-volatile liquid is unwanted, and as such, the non-volatile liquid does not function as a solvent with respect to the CSSC, but as a plasticizing agent intended to remain therewith.

It is believed that a solvent, generally being made of relatively small molecules, is able to enter between molecules of CSSC and distance them one from the other, to such an extent that the CSSC readily dissolves within the solvent, forming a homogeneous solution even at room temperature. As used herein, the term "solvent" refers to a liquid wherein more than about 5 wt.% of the CSSC can dissolve at room temperature.

A non-volatile (*e.g*., plasticizing) liquid, in contrast, being typically larger in size compared to molecules of solvents, would not readily separate CSSC molecules to form a solution at room temperature, at which it could at best provide for a preliminary swelling. Elevated temperatures are required to move the CSSC molecules sufficiently away from one another to allow enough non-volatile liquid to enter within the formed gaps. Even under such favorable conditions, mixing may be required to homogeneously form the plasticized CSSC. Such elevated temperatures can be equal to or higher than at least one of the first ***Tm*, *Ts*,** and ***Tg*** of the CSSC.

Non-volatile liquids suitable for the present invention can be selected from: monofunctional or polyfunctional aliphatic esters (such as dimethyl glutarate, dimethyl maleate, dimethyl methyl glutarate, dipropylene glycol dibenzoate and lactic acid isoamyl ester); fatty esters (such as 2-ethylhexyl lactate, benzyl benzoate, butyl butyryl lactate, C₁₂-C₁₅ alkyl benzoate, a mixture of caprylyl caprate and caprylyl caprylate, decyl oleate, dibutyl adipate, dicaprylyl carbonate, dibutyl maleate, dibutyl sebacate, diethyl succinate, ethyl oleate, glyceryl monooleate, glyceryl monocaprate, glyceryl tricaprylate, glyceryl trioctanoate, isopropyl myristate, isopropyl palmitate, L-menthyl lactate, lauryl lactate, n-pentyl benzoate, PEG-6 caprylic/capric glycerides, propylene glycol monolaurate, propylene glycol mono-caprylate, triacetin, triethyl citrate, triethyl o-acetylcitrate, tris(2-ethylhexyl) o-acetyl-citrate, tributyl o-acetylcitrate and tributyl citrate); cyclic organic esters (such as decanoic lactone, gamma decalactone, menthalactone and undecanoic lactone); aromatic esters (such as diethyl phthalate); fatty acids (such as caprylic acid, cyclohexane carboxylic acid, isostearic acid, lauric acid, linoleic acid, linolenic acid, myristic acid, oleic acid, palmitic acid and stearic acid); terpenes (such as citronellol, eugenol, farnesol, hinokitiol, linalool, menthol, menthone, neridol, terpineol and thymol); aromatic alcohols (such as benzyl alcohol); aromatic ethers (such as phenoxy ethanol); aldehydes (such as cinnamaldehyde); and combinations thereof.

In some embodiments, the non-volatile liquid that may be used to plasticize a CSSC as herein-disclosed is a polyfunctional aliphatic ester (PFAE), being a diester derivative of common dicarboxylic acids: namely adipic (C₆), azelaic (C₉) and sebacic (C₁₀) acids, the alcohol portion of the diesters generally falling in the C₃-C₂₀ carbon number range, including linear and branched, even and odd numbered alcohols. In particular embodiments, the non-volatile liquid, being a PFAE, is selected from dibutyl adipate (*e.g*., commercially available as Cetiol^{®} B), dibutyl sebacate, triethyl O-acetylcitrate (*e.g*., commercially available as Citrofol^{®} AII), C₁₂-C₁₅ alkyl benzoate (*e.g*., commercially available as Pelemol^{®} 256) and dicaprylyl carbonate (*e.g*., commercially available as Cetiol^{®} CC).

When the CSSC is non-biodegradable and/or when the surface to which the composition is to be applied is not skin, additional non-volatile liquids can be used. Such additional liquids can be mineral oils, natural oils, vegetal oils, essential oils, synthetic oils and combinations thereof, provided they preferably fulfill present requirements.

### Fatty amines

Fatty amines suitable for forming the shell (or the first shell) surrounding the CSSC core of the nano-elements need first be miscible with the CSSC, capable of forming a homogeneous mixture when combined therewith.

Suitable fatty amines are primary, secondary, tertiary or quaternary C₈-C₂₂ amines, having a hydrophobic straight, branched, or cyclic, saturated or unsaturated, aliphatic or aromatic, alkyl or aryl chain. Combinations of such fatty amines may also be used. In some embodiments, the hydrophobic chain of the fatty amine is a C₈-C₂₀ or a C₈-C₁₈ aliphatic alkyl chain. Preferably, the fatty amine is liquid at room temperature, however this is not essential. Typically, primary, secondary and tertiary fatty amines can be liquid at room temperature, *i.e.,* having a melting temperature of at most 25°C, at most 20°C, at most 15°C, or at most 10°C, whereas quaternary amines tend to have melting temperatures higher than 25°C.

Alkyl or aryl chains of such lengths (*e.g*., C₈-C₂₂, C₈-C₂₀, or C₈-C₁₈) create a fatty "tail", attached to the amine "head". Hence, the amine molecule contains a hydrophobic part (*i.e.,* the fatty tail) and a hydrophilic part (*i.e.,* the amine head). The arrangement of the amine molecules within the core-shell nano-elements is to be discussed further below.

The hydrophobic tail renders the fatty amine water-insoluble, *i.e.,* having a solubility of 5 wt.% or less, 4 wt.% or less, 3 wt.% or less, 2 wt.% or less, 1 wt.% or less, 0.5 wt.% or less, or 0.1 wt.% or less by weight of the polar carrier (similarly to the CSSCs).

The fatty amines while thoroughly mixed at an elevated temperature to form a homogenous mixture with the CSSC in the presence or absence of a non-volatile liquid (or any other ingredients miscible with the CSSC and the blend formed therewith), they are not intended to entirely remain within the core. On the contrary, they are believed to migrate towards the outer surface of the nano-elements, allowing their hydrophilic heads to be exposed to the polar carrier surrounding the nano-elements, while their hydrophobic tails tend to remain within the water-insoluble environment of the core. This phenomenon results in the formation of the shell, which can be confirmed by the change in charge of the nano-elements. While a core of CSSC deprived of a shell of fatty amines would display a negative charge in an aqueous polar liquid (*e.g.,* in water), the formation of a shell of fatty amines as obtained by the above method masks the core, reducing its negative charge, and may even provide a positive charge to the core-shell nano-elements depending on the respective charge density and amounts of the core and shell materials and on the liquid environment (*e.g*., pH) which may modulate said charges.

Noticeably, the fatty amines forming the shell of the present nano-elements are not covalently bound to the CSSC of the core, enabling the fatty amines to migrate to the core surface to form the shell and the materials of the core and the shell to retain their original respective potency.

Primary amines are advantageous with respect to the charge they may provide to the shell, as compared to secondary and tertiary amines. Without wishing to be bound by any particular theory, it is believed that secondary and tertiary amines are advantageous when transdermal delivery of nano-elements having only a single shell of fatty amines is sought, as they have a lower tendency to form reactive nitrogen hydrides (*e.g*., NH₃⁺) which might cause skin irritation. Such secondary and tertiary amines can be selected from: N,N-Bis-(2-hydroxyethyl)C₁₂-C₁₈-alkylamine octylamine (such as commercially available Genamin^{®} O 020 Special), and N,N-dimethyl-dodecylamine. Quaternary amines can be quaternary ammonium salts such as cetrimonium chloride. In a particular embodiment, the fatty amine is selected from the group comprising: N,N-Bis-(2-hydroxyethyl)C₁₂-C₁₈-alkylamine, N,N-dimethyl-dodecylamine, oleyl amine, octyl amine and cetrimonium chloride.

At high temperatures, a fatty amine may be prone to degradation, resulting in amine loss (due to amidation by oxidation of the amine). Analysis of such degradation can be performed by placing the fatty amine at a tested temperature for a sufficient amount of time, *e.g.,* 24 hours, and then measuring the amine value by routine analysis using standard methods, such as described for instance in ASTM D 2074-07. By way of non-limiting example, the amine value can be assessed by titration of the fatty amine (having been subjected to the tested temperature) with hydrochloric acid, the amine value corresponding to the volume of 0.1N HCl in milliliters needed to neutralize 10g of product. The obtained result is then compared to the amine value of the same fatty amine in natural, non-heated form, which is provided by the manufacturer, or can be independently measured as described above. Any reduction in the value of the amine number of the heated fatty amines may suggest the degradation of the amines at the tested temperature (such temperature being referred to as the "degradation point" of the fatty amine). The temperatures used in preparing the core-shell nano-elements of the present invention should accordingly be preferably below the degradation point, so as to substantially maintain the amine groups in a reduced/non-oxidized state.

As previously described, besides forming the shell, some of the fatty amines (*e.g*., oleyl amine) may act as plasticizers to the core, their addition decreasing the viscosity of the CSSC used therewith, such an effect being described *e.g*., in Example 1.

Alternatively, or additionally, the fatty amines (*e.g*., N,N-Bis-(2-hydroxyethyl)C₁₂-C₁₈-alkylamine octylamine) may also serve as surfactants, suitable to stabilize the core-shell nano-elements within the polar carrier. In some embodiments, the fatty amines' portion exposed in the shell may enable charging of the core-shell nano-elements to such an extent that the elements can remain stably dispersed in the composition in absence of a dedicated surfactant externally provided in the liquid phase (such as demonstrated in Examples 1 and 2). In such a case, the core-shell nano-elements can be viewed as "self-emulsifying" or "self-emulsified".

Alternatively, or additionally, the fatty amines may further serve as polar-insoluble active agents, not only forming the shell of the core-shell nano-elements but also exerting their own activity (*e.g*., as pesticides).

### Polar medium

The liquid medium forming the continuous phase in which nano-elements including the core made of CSSC are dispersed is polar. In some embodiments, the liquid phase consists essentially of a polar carrier, whereas in other cases additional components can be present within the polar carrier. Such additional components can be, for illustration, surfactants, carrier-soluble active agents, or skin permeation enhancers, as herein-detailed, or any other additives conventionally present in the (*e.g*., dermatological) compositions. A polar carrier suitable for the present invention can be selected from a group comprising water, glycols (*e.g*., ethylene glycol, propylene glycol, dipropylene glycol, and 1,2-butanediol 1,3-butanediol, 1,4-butanediol, 2-ethyl-1,3-hexanediol and 2-methyl-2-propyl-1,3-propanediol), glycerols including glycerol, precursors and derivatives thereof (*e.g*., acrolein, dihydroxyacetone, glyceric acid, tartronic acid, epichlorohydrin, glycerol tertiary butyl ether, polyglycerol, glycerol ester and glycerol carbonate) and combinations thereof.

A polar medium may be formed of one or more suitable polar carriers, the resulting liquid being often referred to as an aqueous solution (or an aqueous phase) when water is the preponderant polar carrier. In some cases, a liquid deemed not sufficiently polar by itself (such as a fatty alcohol) can be present in the liquid phase in addition to the polar carrier(s), provided that the liquid insufficiently polar to form the entire liquid polar phase is a) soluble in the main polar carrier (*e.g*., having a water-solubility of 5 wt.% or more) so as to form a unique liquid phase therewith; and b) the overall polarity of the liquid phase is maintained. The polarity index of the resulting liquid phase may be of 3 or more, 4 or more, or 5 or more, water having for reference a polarity index of 9-10.

As the polarity index of a solvent refers to its relative ability to dissolve in test solutes, a liquid may additionally or alternatively be classified as polar or non-polar in view of its dielectric constant (*εᵣ*). Liquids having a dielectric constant of less than 15 are generally considered non-polar, while liquids having a higher dielectric constant are considered polar, the relative polarity of a liquid increasing with the value of the dielectric constant. Preferably, the polar carrier suitable for the present compositions has a dielectric constant of 20 or more, 30 or more, 40 or more, 50 or more, or 60 or more, as established at room temperature. For illustration, the dielectric constant of propylene glycol is 32, the dielectric constant of glycerol is 46, and the dielectric constant of water is 80. While for simplicity, this guidance is provided for a neat polar carrier, this in fact should preferably apply to the entire polar liquid phase prepared therefrom (*e.g*., including additional polar-soluble materials and/or consisting of a mixture of liquid carriers). Noticeably, a liquid polar phase can be constituted of a mix of formally polar solvents (*e.g*., having *εᵣ* ≥ 15) with formally non-polar ones (*e.g.,* having *εᵣ*< 15), as long as their respective volume allows for the entire liquid phase to be polar (*e.g*., having *εᵣ* ≥ 15). The dielectric constant of a liquid is typically provided by the manufacturer but can be independently determined by any suitable method, such as described in ASTM-D924.

As discussed, the composition of the polar liquid phase should be such that the core-shell nano-elements including the CSSCs in their cores and the fatty amines in their first shells can remain essentially non-soluble and stably dispersed therein, with no significant leaching of the contents of the nano-elements into their surrounding medium, when undesired. The polar liquid phase should furthermore enable a suitable charging of the nano-elements, as adapted, for instance, for a desired distribution of their delivery and efficacy on or across skin layers.

As the polar medium may comprise additional liquids and/or materials dissolved therein, the polar carrier can constitute at least 50 wt.%, at least 60 wt.%, at least 70 wt.%, at least 80 wt.%, or at least 90 wt.%, by weight of the liquid phase.

In some embodiments, the polar carrier comprises water (*e.g.,* 45 wt.% water, 45 wt.% propylene glycol, and 10% of a fatty alcohol), consists of water (*e.g*., including between 51 wt.% and 80 wt.% of water), consists essentially of water (*e.g*., including between 81 wt.% and 99 wt.% of water), or is water.

### Surfactants

Some CSSCs may remain nano-dispersed in the composition in view of their inherent chemical properties, the nano-elements for instance having a charge sufficient to ensure repulsion of the particles, consequently ensuring their stable dispersion. Other CSSCs may alternatively or additionally remain nano-dispersed in view of being plasticized with a non-volatile-liquid additionally serving as a surfactant to a sufficient extent. The fatty amines may also serve as surfactants, aiding in dispersing the nano-elements within the polar carrier.

In such cases, and regardless of the underlying rationale, the nano-elements can be considered self-emulsifying (*e.g*., remaining as discrete individual nano-elements in the liquid medium in absence of a surfactant devoted to that purpose). The core-shell nano-elements are deemed dispersed, when the particles have reached a desirable size (and/or particle size distribution) and "stably dispersed" when the particles can retain a desirable size (or PSD) over time. The original size and PSD can be as determined upon completion of preparation of the core-shell nano-particles, or at any other desired timepoint. As size measurements may vary between repeats at a same time point, a size or PSD at a subsequent time point is considered substantially similar to values obtained at a previous time point, if being within 10% or within 5% of previously determined values. Therefore, the particles are considered stably dispersed if measurements of their sizes and/or size distribution do not vary by more than 10% or 5% for at least 1 day, for at least 2 days, or for at least 3 days. Such stability can be assessed under any desired storage conditions, either at room temperature, at cooler temperatures (*e.g*., 4-8°C), or on the contrary at higher temperatures (*e.g*., 30-40°C), when accelerated stability tests are desired.

While the present compositions can be stable as a result of the constituents of the core-shell nano-particles and/or their environmental conditions (*e.g*., pH of liquid medium), in some embodiments, the composition may further comprise at least one (dedicated) surfactant, for the core-shell nano-elements to remain stably dispersed (hence also in their intended size range).

Surfactants suitable for the purpose of the present invention lower the surface tension between the core-shell nano-elements containing the CSSCs in their cores and the fatty amines in their first shells and the environment in which they are immersed. Depending on their chemical formula (and on the CSSC and polar carrier being considered), the surfactants can be miscible with the CSSCs (but able to diffuse to its outer surface) or with the polar carrier wherein the nano-suspension is formed.

Surfactants suitable for the present compositions and methods are generally amphiphilic, containing a polar or hydrophilic part and a non-polar or hydrophobic part. Such surfactants may be characterized by Hydrophilic-Lipophilic Balance (HLB) values within the range of 1 to 35, wherein the HLB values, which generally imply compatibility with water systems, are typically provided on Griffin scale.

Suitable surfactants for the purpose of the present invention can be anionic, cationic, amphoteric or non-ionic surfactants.

When it is desired that the core-shell nano-elements of the present invention be positively charged in aqueous environments, for instance to promote the attachment of additional shells made of materials (*e.g*., active agents) having an opposite charge in the same medium or to prolong the retention of the nano-elements on the skin surface, cationic surfactants can be suitable for the purpose of maintaining, and even augmenting the positive charge of the nano-elements. Non-ionic or amphoteric surfactants, and even more so anionic surfactants, are more likely to reduce a positive charge of core-shell nano-elements, therefore their concentration should be adapted so that the overall charge remains sufficiently positive, when desired. In some embodiments, when a non-ionic, amphoteric, or anionic surfactants are used, they are added at a weight ratio of at most 1: 1 with respect to the weight of the fatty amine(s).

When the surfactants are relatively water-soluble, or for simplicity of preparation regardless of solubility, surfactants can be added to the liquid phase in which the core-shell nano-elements are present. When the surfactants are relatively water-insoluble, they can alternatively be incorporated with the CSSC in the cores of the nano-elements.

Cationic surfactants can be selected from the group including quaternary ammonium compounds, most being highly soluble in water (*e.g*., benzalkonium chloride, trimethyl ammonium methyl sulfate). Quaternary ammonium compounds being relatively water-insoluble (*e.g*., cetrimonium chloride, stearalkonium chloride) and satisfying other requirements set herein for the fatty amines can be considered as quaternary fatty amines, such amines being therefore capable of acting as cationic surfactants in addition to the formation of a shell of positively chargeable amine heads.

Non-ionic surfactants can be selected from the group including: fatty alcohols (*e.g.,* cetearyl alcohol); ethoxylated fatty alcohols (*e.g*., C₈-C₁₈ alcohol polyglycol, polyoxyl 6 stearate and polyoxyl 32 stearate); poly (ethylene glycol) block copolymers (*e.g*.; poloxamer); ethylene oxide (EO)/propylene oxide (PO) copolymers; alkylphenol ethoxylates (*e.g*., octylphenol polyglycol ether and nonylphenol polyglycol ether); alkyl glucosides and polyglucosides (*e.g.,* lauryl glucoside); fatty alkanolamides (*e.g.,* lauramide diethanolamine and cocamide diethanolamine); ethoxylated alkanolamides; ethoxylated fatty acids; sorbitan derivatives (*e.g*., polysorbates, sorbitan laurate, sorbitol, 1,4-sorbitan, iso-sorbide and 1,4-sorbitan triester, PEG-80); alkyl carbohydrate esters (*e.g*., saccharose fatty acid monoester); amine oxides; ceteareths; oleths; alkyl amines (other than fatty amines); fatty acid esters (*e.g.,* ascorbyl palmitate, ethylene glycol stearate, polyglyceryl-6 esters, polyglyceryl-6 pentaoleate, polyglyceryl-10 pentaoleate and polyglyceryl-10 pentaisostearate); polyoxylglycerides (*e.g*., oleoyl polyoxyl-6 glycerides); natural oil derivatives; ester carboxylate (*e.g*., D-α-tocopherol polyethylene glycol succinate (vitamin E TPGS)); and urea.

Amphoteric surfactants can be selected from the group including betaines (*e.g*., cocamidopropyl betaine); alkylamphopropionates (*e.g*., cocoamphopropionate); alkyliminopropionates (*e.g.,* sodium lauraminopropionate); and alkylamphoacetates (*e.g*., cocoamphocarboxyglycinate.

Anionic surfactants can be selected from the group including: alkyl sulfates (*e.g*., sodium lauryl sulfate, ammonium lauryl sulfate and ammonium laureth sulfate); sulfosuccinates (*e.g*., disodium lauryl sulfosuccinate, disodium laureth sulfosuccinate, sodium dioctyl sulfosuccinate and their mixtures with sulfonic acids and lauramidopropyl betaine); alkyl benzene sulfonates (*e.g*., sodium tosylate, cumene sulfonate, toluene sulfonic acid, xylene sulfonic acid, cumene sulfonic acid and salts (*e.g*., sodium, potassium, calcium, ammonium) thereof); acyl methyl taurates (*e.g*., sodium methyl lauroyl taurate and sodium methyl cocoyl taurate); acyl sarcocinates (*e.g*., sodium lauroyl sarcosinate, sodium cocoyl sarcosinate and sodium myristoyl sarcosinate); isethionates (*e.g*., sodium butyl isethionate, sodium capryloyl isethionate and sodium lauroyl isethionate); propyl peptide condensates; monoglyceride sulfates; ether sulfonates and fatty acid salts (*e.g*., sodium stearoyl lactylate).

These surfactants may be categorized into emulsifiers and hydrotropes, according to their mechanism of action. Emulsifiers readily form micelles (thus being characterized by a critical micelle concentration (CMC) value) and are believed to increase the dispersibility of the CSSC (or plasticized CSSC) when later combined with a polar carrier to yield a nano-suspension. As a rule, emulsifiers typically relate to surfactants ensuring the dispersion of one liquid into another, the liquids having opposite polarity, whereas dispersants relate to surfactants ensuring the dispersion of a solid into a liquid. As the present method may provide for nano-emulsions and nano-dispersions, surfactants referred to as emulsifiers at a step in which the nano-suspension is an emulsion, may in fact become dispersants, to the extent that an initial nano-emulsion later yields a nano-dispersion at a lower temperature. Hence, as used herein, the term "emulsifier(s)" also includes surfactants otherwise known as dispersants.

Emulsifiers that are lipophilic in nature, *i.e.,* include a relatively large hydrophobic part, are more suitable to be combined with the CSSC (and any other material not miscible in the polar carrier, *e.g.,* a fatty amine or a non-volatile liquid), and may therefore be referred to as polar-carrier-insoluble emulsifiers (or surfactants, in general). Hence, such relatively hydrophobic emulsifiers are expected to be within the core of the nano-elements of the composition. These relatively hydrophobic emulsifiers generally have HLB values of 9 or less, 8 or less, 7 or less, or 6 or less, on Griffin scale. The behavior of such surfactants may be considered similar to that of the fatty amines since the hydrophobic part of the surfactants turns toward the cores and is embedded therein, and their hydrophilic part turns towards the shell, and may even be part of the shell including the amine heads.

Emulsifiers that are more hydrophilic in nature have a relatively large hydrophilic part and would be more compatible with the polar phase of the composition, and may therefore be referred to as polar-carrier-soluble emulsifiers (or surfactants, in general). Such relatively hydrophilic emulsifiers generally have HLB values of 11 or more, 13 or more, 15 or more, 17 or more, or 20 or more.

Emulsifiers having HLB values within the range of 9 and 11 are considered "intermediate", the hydrophobic and hydrophilic parts of such emulsifiers being fairly well-balanced. Such intermediate emulsifiers can be added in the present methods either to the CSSC or the polar carrier and may accordingly be found in the nano-elements or in their medium, the ability of a portion of such surfactants to migrate between the two phases being also envisioned.

Surfactant serving as emulsifiers are selected from a group comprising alkyl sulfates, sulfosuccinates, alkyl benzene sulfonates, acyl methyl taurates, acyl sarcocinates, isethionates, propyl peptide condensates, monoglyceride sulfates, ether sulfonates, ester carboxylates, fatty acid salts, quaternary ammonium compounds, betaines, alkylampho-propionates, alkyliminopropionates, alkylamphoacetates, fatty alcohols, ethoxylated fatty alcohols, poly (ethylene glycol) block copolymers; ethylene oxide (EO)/propylene oxide (PO) copolymers, alkylphenol ethoxylates, alkyl glucosides and polyglucosides, fatty alkanolamides, ethoxylated alkanolamides, ethoxylated fatty acids, sorbitan derivatives, alkyl carbohydrate esters, amine oxides, ceteareths, oleths, alkyl amines (other than fatty amines), fatty esters, polyoxylglycerides, natural oil derivatives and ester carboxylate.

In a particular embodiment, the emulsifier is selected from: vitamin E TPGS, poly (ethylene glycol) block copolymer, a mixture of polyoxyl 6 stearate type I, ethylene glycol stearates and polyoxyl 32 stearate type I (such as commercially available as Tefose^{®} 63 from Gattefossé, France), mixtures comprising olive oil-derived extracts (such as commercially available under the brand Olivatis^{®} from Medolla Iberia, Spain), ascorbyl palmitate, polyglyceryl-10 pentaoleate, polyglyceryl-10 pentaisostearate, oleoyl polyoxyl-6 glycerides (such as commercially available as Labrafil^{®} M 1944 CS from Gattefossé, France), disodium laureth sulfosuccinate, disodium lauryl sulfosuccinate, a mixture of disodium lauryl sulfosuccinate, sodium C₁₄-C₁₆ olefin sulfonate and lauramidopropyl betaine (such as commercially available as Cola^{®}Det EQ-154 from Colonial Chemical, USA) and a mixture of olive oil and glutamic acid (such as commercially available as Olivoil^{®} glutamate from Kalichem, Italy).

While surfactants acting as emulsifiers are generally sufficient to stabilize nano-elements of the present compositions, the Inventors have found that when CSSCs are present at a relatively high concentration, as enabled by the invention, the addition of another type surfactants, namely hydrotropes, assisted in achieving a satisfactory stability.

Hydrotropes are also amphiphilic molecules, but contrary to emulsifiers, they contain a relatively shorter lipophilic chain. As the lipophilic portion of the hydrotropes is generally too short to allow micelle formation, the hydrotropes alternatively solubilize hydrophobic compounds in the polar carrier and permit co-emulsification, together with the emulsifier. Generally, hydrotropes are miscible mainly in the polar carrier phase (*e.g*., aqueous phase) of the nano-suspension, and are characterized by having HLB values of 10 or more, 12 or more, 15 or more or 18 or more.

Suitable hydrotropes can be selected from the group including sodium dioctyl sulfosuccinate, urea, sodium tosylate, adenosine triphosphate, cumene sulfonate, toluene sulfonic acid, xylene sulfonic acid, cumene sulfonic acid) and salts thereof. In a particular embodiment, the hydrotrope is selected from: sodium dioctyl sulfosuccinate, urea and a salt of xylene sulfonic acid, such as ammonium xylenesulfonate.

### Active agents

While the present compositions can be biologically active as a result of the presence of the CSSC by itself, in particular when the compositions are dermatological compositions to be applied to the skin to promote synthesis of skin structural proteins, their uses can be enhanced and/or modified by inclusion of active agents having similar functions, so as to enhance efficacy, and/or different functions, so as to broaden the scope of efficacy. In such a case, the core-shell nano-elements can be considered as nano-carriers for other active agents to be administered topically.

In some embodiments, the (*e.g*., dermatological) composition further comprises one or more polar-carrier-insoluble active agent(s). Such carrier-insoluble active agent(s) are generally comprised within the cores of the core-shell nano-elements, as they are miscible with the components included therein, *i.e.,* CSSCs, and optional non-volatile liquid, as well as with the hydrophobic tails of the fatty amines, embedded within the cores. The constituents of the cores are miscible one with the other when forming a unique phase.

Such active agents which alone, and more so in combination with the CSSC, are expected to improve skin appearance and health are known and are only exemplified below. Information on additional compounds known to have activities favorable to cosmetic treatment of the skin can be found *e.g.,* in the list of cosmetic ingredients that are generally recognized as safe (GRAS), maintained at the FDA's website, in the list provided in the European Cosmetic Ingredient Database (COSING), or in any similar authoritative list issued by relevant professionals in the cosmetic industry. Active agents suitable for pharmaceutical treatment can be found *e.g.,* in the FDA's Drugs@FDA database, in the European Medicines Agency (EMA)'s European Public Assessment Reports (EPARs) database, or in any similar authoritative list issued by relevant professionals in the pharmaceutic industry. Active agents suitable for agrochemical or industrial compositions can be found *e.g.,* in the Pesticides Product Information System (PPIS) maintained by the US Environmental Protection Agency (EPA), in the EU Pesticides Database maintained by the European Commission's Directorate-General for Health and Food Safety (DG SANTE), or in any similar authoritative list issued by relevant professionals in the agrochemical industry.

While selected according to the activity they are to provide to the compositions, the CSSC-miscible / polar-carrier-insoluble active agents may have secondary functions, and for illustration optionally act as plasticizers, when capable of decreasing the viscosity of the CSSC within the core.

In some embodiments, and similar to the CSSC and non-volatile liquid described above, the optional CSSC-miscible / carrier-insoluble active agent should have a solubility of 5 wt.% or less, 4 wt.% or less, 3 wt.% or less, 2 wt.% or less, 1 wt.% or less, 0.5 wt.% or less, or 0.1 wt.% or less by weight of the polar carrier or the liquid phase including it.

The CSSC-miscible / carrier-insoluble active agent, incorporated into the nano-elements of dermatological compositions, may include, by way of non-limiting examples, albendazole, benzoyl peroxide, bakuchiol, castor oil, ceramide, erythromycin, finasteride, ibuprofen, ketoconazole, lidocaine, macrolides, mebendazole, neem oil, retinol, salicylic acid, tadalafil, tetracyclines, tretinoin, vitamin A, vitamin D, and vitamin K. In particular embodiments, the carrier-insoluble active agents are bakuchiol, castor oil or retinol (*e.g.,* for a cosmetic use), benzoyl peroxide or tadalafil (*e.g.,* for a therapeutic use) and neem oil (*e.g.,* as an insect repellent for humans).

In some embodiments, the (*e.g*., dermatological) composition further comprises one or more polar-carrier-soluble active agent(s), which would surround the first shell of the fatty amines, forming a second shell. The second shell may be composed of more than one layer of a carrier-soluble active agent(s). As already mentioned, the formation of such second shells can be favored by non-covalent (*e.g*., electrostatic) interactions between the fatty amines of the first shell and the carrier soluble agents, or by covalent binding between the two. Excess of polar-carrier-soluble active agent(s) may additionally be found in dissolved form within the polar carrier.

Exemplary active agents that are carrier-soluble and may form second shells of nano-elements in dermatological compositions can be selected from: azelaic acid, biotin, clindamycin, collagen, elastin, famciclovir, folacin, hyaluronic acid (HA), niacin, pantothenic acid, riboflavin, thiamin, vitamin B12, vitamin B6 and vitamin C. In particular embodiments, the carrier-soluble agent is cosmetically active HA, which is one type of glycosaminoglycan.

It is known that high molecular weight (HMW) carrier-soluble active agents are unable to transfer through the skin when formulated into topical compositions. Such HMW compounds, when dissolved in an aqueous environment, tend to unfold and form expanded structures, which are less likely to pass through any of the pathways existing across the skin. HMW molecules are therefore expected to remain on the outer surface of the skin, when not totally washed away therefrom. In contrast, the objective of the nano-elements of the present invention is to penetrate into the skin, where they can act *inter alia* as collagen-synthesis stimulants, but more generically as nano-carriers. The core-shell structure of the nano-elements allows HMW active agents to align as an outer (second) shell and be thus transported into the skin in a compacted manner. The core-multi-shells architecture is therefore expected to enable transdermal delivery of active agents even having a HMW, so that they may display their activity within their respective target sites.

In some embodiments, the carrier-soluble active agent that may be combined with the core-shell nano-elements of the invention is hyaluronic acid and both HA of low molecular weight (LMW), *i.e.,* having a MW of less than 600 kDa, and HA of high molecular weight (HMW), *i.e.,* having a MW of more than 600 kDa can be used. In some embodiments, the HA is a LMW HA having a MW of 500 kDa or less, 400 kDa or less, 300 kDa or less, 200 kDa or less, or 100 kDa or less. In particular embodiments, the LMW HA has a molecular weight not exceeding 50 kDa, not exceeding 25 kDa, or not exceeding 10 kDa.

In some embodiments, the (*e.g*., dermatological) composition may comprise both a carrier-insoluble active agent and a carrier-soluble active agent.

Plant-based products and extracts, serving as active agents, may also be added to the compositions, and can either be carrier-insoluble or carrier-soluble. As used herein, the term "plant extracts" refers both to natural fractions isolated from any relevant part of any suitable plant (*e.g.,* flowers, fruits, herbs, leaves, peels, roots, seeds, stems, *etc.*) and to the synthetic version of the active agents of the natural extracts. Plants, the natural products or extracts of which are traditionally used for cosmetic or therapeutic effects when applied to the skin, are known to the skilled persons and too numerous to be comprehensively listed. By way of example, a plant product or extract containing an active agent suitable for the present invention can be isolated or obtained from apple, bergamot, broccoli, cannabis, coffee, corn, curcumin, fennel, garden angelica, ginseng, grapefruit, honeybush, Japanese red pine, kale, orange, paprika, passion fruit, raspberry, rooibos, soybean, spinach, tea and tomatoes. Such plant extracts are known to have *inter alia* anti-acne, anti-oxidant, anti-inflammatory, and/or anti-aging activity. In a particular embodiment, the plant-based active agent used in the compositions is curcumin.

The carrier-insoluble and/or carrier-soluble active agents optionally added to the present dermatological compositions may have a cosmetic function, for instance, have a dermal filling effect, or may, by themselves, be capable of enhancing collagen synthesis (and/or reducing its degradation). Considering the collagen synthesis stimulating ability of the CSSCs or CSSPs within the core of the nano-elements on their own, adding such active agents, which may serve a similar purpose, can result in a combined activity providing for an even higher collagen formation or presence within the skin. For illustration, the CSSC of the core may promote the neo-synthesis of collagen, elastin and/or glycosaminoglycans (and/or reduce their degradation) and the core-shell nano-elements may further include as water-soluble active agents on an outer shell of the nano-elements collagen, elastin, hyaluronic acid, or fragments thereof. Regardless of the exact cosmetic contribution of the active agents, the resulting dermatological compositions may be regarded as "cosmetically active".

Alternatively, the active agents (carrier-soluble or -insoluble) may serve pharmaceutical purposes, rendering the compositions "pharmaceutically active". All active agents, in particular if already used in existing topical formulations serving the therapeutic purposes exemplified herein, are encompassed. For illustration, the pharmaceutic agent capable of treating a skin condition (*e.g.*, having an analgetic, anti-inflammatory and/or anti-arthritic activity) can be any known cortico-steroidal drug (*e.g*., amciconide, betamethasone, clobetasol, desonide, dexamethasone, fluticasone, hydrocortisone, methylprednisolone, mometasone, *etc.*) or any known non-steroidal drug (*e.g*., diclofenac, etodolac, flurbiprofen, ibuprofen, indomethacin, ketoprofen, ketorolac, meloxicam, nabumetone, naproxene, piroxicam, sulindac, *etc.*)*.*

Hence, the core-shell nano-elements, in addition to their collagen-synthesis abilities, may be used as nano-carriers for cosmetic or pharmaceutical agents to be applied to the skin of a subject.

While active agents have been described above mainly for activities of relevance to the skin, this should not be construed as limiting the use of dermatological compositions according to the present teachings. Drugs that can be transdermally delivered by traditional means and targeting other organs or disorders can also be incorporated in the present compositions, either in the core of the nano-elements if relatively water-insoluble, or in the shell(s) if relatively water-soluble. Such active agents, typically used in medicaments intended for human subjects, can serve various purposes, such as anti-depression, blood pressure regulation, contraception, and pain relief, to name a few.

While it is expected based on Example 12 that the present core-shell nano-elements may carry active agents that cannot currently be delivered transdermally by conventional methods, such as active agents having a molecular weight of 600 g/mol or more, they understandingly may carry active agents already known to be transdermally deliverable. Some transdermally deliverable active agents are exemplified below but additional such compounds may be reported in future clinical studies or marketing authorizations.

Non-limiting examples of such active agents include amitriptyline, apomorhine, asenapine, buprenorphine, capsaicin, clonidine, deutetrabenazine, ensam, estrogen, fentanyl, gabapentin, granisetron, ketamine, nicotine, methimazole, methyl phenidate, nitroglycerine, oxybutynin, physostigmine, pramipexole, progestin, riluzole, rivastigmine, rotigotine, scopolamine, selegiline, sumatriptan, and testosterone.

Most of the active agents listed above to exemplify uses of relevance to the treatment of human subjects can also be used in veterinary products for the treatment of non-human animals. Dermatological compositions for veterinary uses can additionally contain different active agents adapted to the physiology of the animal to be treated, to the effect to be obtained (*e.g*., increasing meat, milk, or wool production), and/or to the animal specific disorder. For illustration, active agents more suited to veterinary products and uses include agents improving the productivity of the livestock or protecting the animals (*e.g*., various pesticides, such as methoxychlor and carbaryl; anti-infection agents, such as isoflupredone; and anti-bacterial agents, such as thiostrepton; to name a few).

Moreover, the present core-shell nano-elements can be used to deliver active agents to surfaces other than the skin of a living animal. In such cases, the active agents can be considered as agrochemicals and/or industrial agents that can be incorporated into the CSSC nano-elements, to be used in these fields. Nano-elements containing such agents can be used at least as acaricides, algicides, animal repellents, anthelmintics, anti-moth agents, avicides, bactericides, chemo-sterilants, fertilizers, fungicides, herbicides, insect pheromones, insect repellents, insecticides, molluscicides, nematicides, nitrification inhibitors, ovicides, plant growth activators, rodenticides, termicides and virucides.

While attempts have been made to classify the active agents according to the composition they may supplement or the particular activity they may contribute for use in cosmetic products, pharmaceutical products for veterinary or human use, agrochemical products or industrial products, it is stressed, as previously mentioned, that overlaps exist. For illustration, a fungicide may be used as a preservative in a cosmetic composition, as an active agent to treat a fungal infection on the skin of a living subject, as a transdermally deliverable active agent to treat a systemic fungal infection in animals, as an active agent to treat plant fungal infections or as an active agent to prevent the growth of molds on inert surfaces.

Regardless of their activity and intended use, active agents can be CSSC-miscible / carrier-insoluble (in which case they are incorporated within the core of the core-shell nano-elements) or carrier-soluble (in which case, they can be incorporated as a second shell surrounding the core-shell nano-elements).

### Skin-penetration enhancers

While the polar carriers may suffice to enable enough retention of the CSSC nano-elements on the skin and their delivery through the skin, and some surfactants (if present) can promote it, in some embodiments, the topical composition further comprises a skin-penetration enhancer.

Suitable skin-penetration enhancers can be selected from the group comprising: C₁-C₂₂ alcohols (such as short chains alcohols: ethanol, isopropyl alcohol, and hexanol, and fatty alcohols: octanol, decanol, lauryl alcohol, myristyl alcohol, oleyl alcohol and octyl dodecanol); amides such as 1-dodecylazacycloheptan-2-one (also known as laurocapram and commercialized as Azone^{®}) and its analogues, N-alkyl-azacycloheptan-2-ones, where the alkyl has the general formula CₓH₂ₓ₊₁, X being an integer selected from 1, 3-10, and 14, azacycloheptan-2-ones N-substituted by branched and/or unsaturated chains, N-acylazepan-2-ones, substituted 2-(2-oxoazepan-1-yl) alkanoic acid and its esters, N-alkyl-azacycloheptan-2-thiones, N-alkyl-azacycloheptenones, 4-alkyl-1,4-oxazepan-5,7-dione; cyclic amines with alkyl having the general formula CₓH₂ₓ₊₁, X being an integer selected from 10-12, 14, 16, and 18; long-chain N-acylazepanes, dehydrogenated azacycloheptane derivatives, six-membered ring analogues such as: azacycloheptadienes, N-substituted piperidin-2-ones, derivatives of six-membered ring analogues of Azone^{®}, esters of 2(2-oxopiperidin-1-yl)acetic acid, N-substituted derivatives of 6-oxopiperidine-2-carboxylic acid, N-1-(2-alkylsulfanylethyl)-piperidine-3-carboxylic acids, (thio)morpholines, morpholine-dione derivatives, long-chain N-acylmorpholines, long-chain N-morpholinylalkenones, five-membered ring analogues: long-chain N-acylmorpholines and morpholinoethanol derivatives, 1-piperazin-1-yl-alkan-1-ones and 1-(4-methylpiperazin-1-yl)-alkan-1-ones; aromatic esters (such as octyl salicylate and 2-ethylhexyl 4-(dimethylamino)benzoate); ether alcohols (such as 2-(2-ethoxy-ethoxy)ethanol); glycols; pyrrolidones (such as 2-pyrrolidone and N-methyl-2-pyrrolidone); and sulphoxides (such as dimethyl sulphoxide (DMSO) and decylmethyl sulphoxide).

Typically, skin-penetration enhancers, if present, are added to the polar liquid phase. To the extent that they would be alternatively provided within the core-shell(s) nano-elements, attention should be given to skin penetration enhancers (or any other additives), which may be considered as volatile agents, in which case their presence by weight of the nano-elements should preferably not exceed 2 wt.%.

It can be noted that some materials above defined as skin penetration enhancers may additionally serve as part of the liquid phase, provided that their combination with the main polar carriers does not affect the overall polarity of the liquid and the lack of solubility of the nano-elements therein.

### Volatile organic compounds

Methods for preparing nano-materials are abundantly described in the literature, the main ones suitable for hydrophobic compounds, such as the materials used in the present invention, involving emulsification. The traditional emulsification methods suited for this purpose include oil-in-water (O/W) emulsification or W/O/W emulsification, generally achieved by solvent evaporation or solvent displacement (*e.g*., performed by solvent diffusion or salting-out). These methods are cumbersome and time-consuming, some to an extent being commercially unfeasible. More importantly, they typically result in solvents remaining residually (but not negligibly) trapped within the nano-particles prepared by such methods, which can be particularly critical when the solvent being used is a volatile organic compound (VOC) having a high vapor pressure and low boiling point at room temperature.

As used herein, the term "volatile organic compound" ("VOC") refers to an organic compound having a vapor pressure of 0.1 kPa or more, as measured at a temperature of about 20°C.

Typically, when nano-particles were reported to have been obtained following dissolution of a polymer in a volatile solvent and its subsequent evaporation, the authors remained understandingly silent about their true VOC contents, in view of the difficulties to fully eliminate. When relatively low amounts of VOCs were actually measured and reported, the particles obtained were at least micro-particles (*e.g*., having a particle size of 2-100 micrometer (µm)) and not nano-particles.

As opposed to methods commonly used for preparing micro- or nano-particles, the present invention does not utilize an emulsification technique which requires addition of a solvent (such as a VOC) and dissolution of the CSSC (specifically CSSP) therein, requiring the subsequent removal of the solvent. When volatile solvents are later removed from particles conventionally prepared (presumably by lengthy and burdensome methods), few phenomena may be observed. The elimination of the solvent may yield porous and friable particles, moreover, as the elimination of the solvent may concurrently require the elimination of the medium in which the particles may be dispersed, this process inherently causes the collapse of the dispersion and the drying of the particles, which may then turn into aggregates or agglomerates. Once dried, in an attempt to eliminate the solvent having served for their preparation, the particles are believed to have typically a poor dispersibility as individual particles and furthermore to have a poor malleability, if any. Such particles, prepared according to methods of the art, are believed to be ineffective for the purposes of the present invention.

Thus, VOCs such as conventionally used acetone, acetonitrile, aniline, benzene, carbon tetrachloride, chloroform, cyclohexanone, dichloromethane, dioxane, dimethylesulfoxide, ethyl acetate, hexafluoroisopropyl alcohol, methylene chloride, N,N-dimethylformamide, 2-nitropropane, 1,1,2,2-tetrachloroethane, tetrahydofuran, 1,1,2-trichloroethane and toluene are essentially absent from nano-elements according to the present teachings, either as individual solvents or as blends of two or more VOCs.

In some embodiments, the nano-elements including the CSSC in their cores contain less than 2 wt.%, less than 1.5 wt.%, less than 1 wt.% of a VOC (or a blend thereof) by weight of the nano-elements. In particular embodiments, the nano-elements contain less than 0.5 wt.%, less than 0.4 wt.%, less than 0.3 wt.%, less than 0.2 wt.%, or less than 0.1 wt.% of a VOC (or a blend thereof) by weight of the nano-elements. In further particular embodiments, the nano-elements contain less than 0.09 wt.%, less than 0.08 wt.%, less than 0.07 wt.%, less than 0.06 wt.%, less than 0.05 wt.%, less than 0.04 wt.%, less than 0.03 wt.%, or less than 0.02 wt.% of a VOC (or a blend thereof) by weight of the nano-elements. In some embodiments, the nano-elements may contain up to 0.001 wt.% (which corresponds to 10 parts per million - ppm), up to 0.002 wt.% (20 ppm), up to 0.003 wt.% (30 ppm), up to 0.004 wt.% (40 ppm), up to 0.005 wt.% (50 ppm), up to 0.006 wt.% (60 ppm), up to 0.007 wt.% (70 ppm), up to 0.008 wt.% (80 ppm), up to 0.009 wt.% (90 ppm), or up to 0.01 wt.% (100 ppm) of a VOC (or a blend thereof) by weight of the nano-elements.

In particular embodiments, the nano-elements contain between 0 wt.% and 0.5 wt.%, between 0.0001 wt.% and 0.5 wt.%, between 0.0002 wt.% and 0.4 wt.%, between 0.0003 wt.% and 0.3 wt.%, between 0.0004 wt.% and 0.2 wt.%, between 0.0005 wt.% and 0.1 wt.%, between 0.0001 wt.% and 0.09 wt.%, between 0.0002 wt.% and 0.08 wt.%, between 0.0003 wt.% and 0.07 wt.%, between 0.0004 wt.% and 0.06 wt.%, or between 0.0005 wt.% and 0.05 wt.% of a VOC (or a blend thereof) by weight of the nano-elements.

It is stressed that the afore-said low levels of VOCs are provided with respect to the nano-elements, as the liquid phase in which they are dispersed may tolerate higher amounts of such materials, as may be allowed by the resistance of the nano-elements to solubilization and/or as may be authorized by relevant regulatory authorities governing the use of compositions for any of the purposes for which the present compositions are suitable.

The nature and amount of hypothetical VOCs in nano-elements can be determined by routine analysis, *e.g*., by eliminating the polar carrier, and analyzing the nano-elements isolated therefrom by, *e.g.,* Gas Chromatography. Exemplary standard methods for determining VOCs are described in ASTM D4526 or VDA 277.

### Compositions

Having reviewed the various components that may be used in the present (*e.g.,* dermatological) compositions, suitable concentrations or respective proportions shall be provided below. It is to be noted that some of the components according to the present teachings can serve in more than one role. For instance, fatty amines may have plasticizing and/or dispersing abilities, some providing by themselves an activity that may be viewed as cosmetic or pharmaceutic; and some non-volatile liquids, such as aliphatic esters (*e.g*., dimethyl glutarate); fatty acids (*e.g*., lauric acid, linoleic acid, linolenic acid, myristic acid, oleic acid, palmitic acid, stearic acid and isostearic acid); fatty acid esters (*e.g*., ethyl oleate, glyceryl monooleate, glyceryl monocaprate, glyceryl tricaprylate, isopropyl myristate, isopropyl palmitate, propylene glycol monolaurate and propylene glycol monocaprylate); and terpenes (*e.g.,* eugenol, D-limonene, menthol, menthone, farnesol and neridol); may also have skin penetration enhancing properties. In yet another example, some polar carriers, such as water and certain glycols and glycerols, may also assist skin-penetration, or even serve as surfactants. Thus, when referring, for instance, to the concentration of skin-penetration enhancers in the composition, the information refers only to dedicated compounds intentionally added to serve this role, excluding compounds having a different primary role in the composition.

In some embodiments, the concentration of the CSSC (or combination thereof) in the nano-elements is within the range of 1 wt.% to 99 wt.%, 1 wt.% to 90 wt.%, 5 wt.% to 80 wt.%, 10 wt.% to 50 wt.%, or 15 wt.% to 40 wt.% by total weight of the nano-elements.

In some embodiments, the concentration of the CSSC(s) in the composition is within the range of 0.1 wt.% to 30 wt.% by total weight of the composition, preferably in the range of 0.5 wt.% to 25 wt.%, 1 wt.% to 20 wt.%, or of 1.5 wt.% to 15 wt.%. In other embodiments, the CSSC(s) concentration is at least 0.1 wt.%, at least 0.5 wt.%, at least 1 wt.%, or at least 1.5 wt.%, by total weight of the composition. In other embodiments, the concentration of the CSSC(s) is at most 30 wt.%, at most 25 wt.%, at most 20 wt.%, or at most 15 wt.% by total weight of the composition.

In some embodiments, the concentration of the fatty amine (or combination thereof) in the nano-elements is within the range of 1 wt.% to 99 wt.%, 1 wt.% to 90 wt.%, 5 wt.% to 80 wt.%, 10 wt.% to 50 wt.%, or 15 wt.% to 40 wt.% by total weight of the nano-elements.

In some embodiments, the concentration of the fatty amine(s) in the composition is within the range of 0.1 wt.% to 30 wt.% by total weight of the composition, preferably in the range of 0.5 wt.% to 25 wt.%, 1 wt.% to 20 wt.%, or of 1.5 wt.% to 15 wt.%. In other embodiments, the fatty amine(s) concentration is at least 0.1 wt.%, at least 0.5 wt.%, at least 1 wt.%, or at least 1.5 wt.%, by total weight of the composition. In other embodiments, the concentration of the fatty amine(s) is at most 30 wt.%, at most 25 wt.%, at most 20 wt.%, or at most 15 wt.% by total weight of the composition.

In some embodiments, the concentration of the non-volatile liquid(s), if present in the nano-elements, is within the range of 1 wt.% to 98 wt.% by total weight of the nano-elements, preferably in the range of 5 wt.% to 90 wt.%, 10 wt.% to 80 wt.%, or 20 wt.% to 70 wt.%. In some embodiments, the concentration of the non-volatile liquid(s) is at least 1 wt.%, at least 5 wt.%, at least 10 wt.%, or at least 20 wt.% by weight of the nano-elements. In other embodiments, the concentration of the non-volatile liquid(s) is at most at most 98 wt.%, at most 90 wt.%, at most 80 wt.%, or at most 70 wt.% by weight of the nano-elements.

In some embodiments, the concentration of the non-volatile liquid(s), if present in the composition, is within the range of 1 wt.% to 30 wt.% by total weight of the composition, preferably in the range of 2 wt.% to 30 wt.%, 3 wt.% to 25 wt.%, or of 5 wt.% to 20 wt.%. In some embodiments, the concentration of the non-volatile liquid(s) is at least 1 wt.%, at least 2 wt.%, at least 3 wt.%, or at least 5 wt.% by weight of the composition.

In other embodiments, the concentration of the non-volatile liquid(s) is at most at most 30 wt.%, or at most 25 wt.%, at most 20 wt.% by weight of the composition. When a non-volatile liquid(s) (or a combination thereof) is added, it is at a concentration of up to 400 wt.% by weight of CSSC, preferably within a range of 5 wt.% to 350 wt.%, 10 wt.% to 200 wt.%, 25 wt.% to 150 wt.%, 25 wt.% to 100 wt.%, or 50 wt.% to 125 wt.%. In some embodiments, the concentration of the surfactant(s), if present in the nano-elements, is within the range of 1 wt.% to 50 wt.%, within the range of 5 wt.% to 50 wt.%, within the range of 10 wt.% to 50 wt.%, within the range of 15 wt.% to 45 wt.%, or within the range of 20 wt.% to 40 wt.% by total weight of the nano-elements. These concentrations refer to dedicated surfactants intentionally added for this purpose and do not include the presence of any other material capable of also serving as surfactants, such as some fatty amine(s).

In some embodiments, the combined concentration of the dedicated surfactants (including, for instance, the emulsifiers and/or hydrotropes), if present in the composition, is within the range of 0.1 wt.% to 60 wt.%, within the range of 0.5 wt.% to 50 wt.%, within the range of 1 wt.% to 40 wt.%, or within the range of 1 wt.% to 30 wt.% by total weight of the composition. In some embodiments, the combined concentration of the surfactants is at least 0.1 wt.%, at least 0.5 wt.%, or at least 1 wt.% by total weight of the composition. In other embodiments, the combined concentration of the surfactants is at most 60 wt.%, at most 50 wt.%, at most 40 wt.%, or at most 30 wt.% by total weight of the composition. Alternatively, the composition is substantially devoid of a dedicated surfactant, the concentration of one or more surfactants being less than 0.1 wt.% by total weight of the composition.

In some embodiments, the polar carrier is present in the composition within the range of 10 wt.% to 99.8 wt.%, 20 wt.% to 99 wt.%, 30 wt.% to 90 wt.%, or 40 wt.% to 85 wt.% by total weight of the composition.

In some embodiments, the carrier-insoluble active agent, if present in the nano-elements, is added to the CSSC at a concentration of up to 50 wt.% by weight of CSSC, preferably within the range of 0.5 wt.% to 35 wt.%, within the range of 1 wt.% to 20 wt.%, within the range of 2.5 wt.% to 15 wt.%, or within the range of 0.5 wt.% to 10 wt.%, such ranges also applying to the nano-elements formed thereby.

In some embodiments, the carrier-soluble active agent, if present, is added to the nano-suspension at a concentration of up to 200 wt.% by weight of CSSC, preferably within the range of 0.1 wt.% to 170 wt.%, within the range of 1 wt.% to 150 wt.%, within the range of 5 wt.% to 100 wt.%, within the range of 5 wt.% to 50 wt.%, within the range of 5 wt.% to 40 wt.%, or within the range of 10 wt.%, up to 20 wt.%.

In some embodiments, the concentration of any one of the active agents, either carrier-soluble or carrier-insoluble, or of all of them if more than one, in the composition, is within the range of 0.01 wt.% to 15 wt.% by total weight of the composition, preferably in the range of 0.05 wt.% to 15 wt.%, 0.1 wt.% to 13 wt.%, 0.5 wt.% to 11 wt.%, or 0.5 wt.% to 10 wt.%. In some embodiments, the concentration of any one of the active agents is at least 0.01 wt.%, at least 0.05 wt.%, at least 0.1 wt.%, or at least 0.5 wt.% by total weight of the composition. In other embodiments, the concentration of all the active agents or the sole one is at most 15 wt.%, at most 13 wt.%, at most 11 wt.%, or at most 10 wt.% by total weight of the composition.

In some embodiments, the concentration of the skin-penetration enhancer(s), if present in the dermatological composition, is within the range of 0.01 wt.% to 30 wt.%, 0.1 wt.% to 25 wt.%, 1 wt.% to 20 wt.%, 3 wt.% to 15 wt.%, or 5 wt.% to 15 wt.% by total weight of the composition.

Preferably, when the compositions are intended to be dermatological compositions, the aforesaid ingredients are approved for cosmetic or pharmaceutic use at the envisioned concentrations. For instance, they do not irritate the skin, nor lead to allergic reactions, or any other acute or chronic adverse effect. Moreover, all ingredients need be compatible one with another, such compatibility being as described above. As readily understood, this principle of compatibility, which can be affected not only by the chemical identity of the materials, but by their relative proportions according to the intended use, should preferably guide the selection of all materials necessary for the compositions disclosed herein.

Similar principles may be followed for agrochemical or industrial compositions, the ingredients being in such cases preferably of a chemical nature and at concentrations approved by the regulatory authorities governing such products.

### Method of preparation

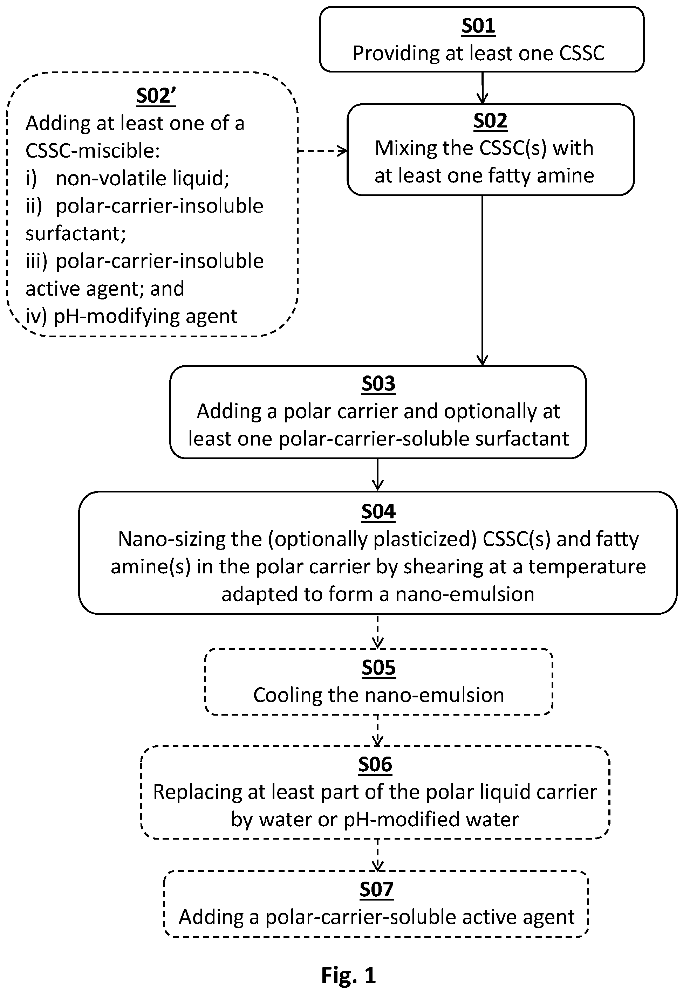

In another aspect of the present invention, there is provided a method for preparing a (*e.g.,* dermatological) composition comprising nano-elements having a core made of a water-insoluble collagen-synthesis stimulating compound (CSSC), the core including in particular embodiments a water-insoluble collagen-synthesis stimulating polymer (CSSP), and a shell made of fatty amines, the core-shell nano-elements being dispersed as a nano-suspension in a polar liquid. The properties and characteristics of the materials used in the present method are as described above for each of the materials. The steps of the present method are briefly displayed in **Figure 1** and further detailed hereinbelow, a step having a dashed contour being optional.

In a first step (**S01**) of the method, at least one CSSC (*e.g.,* at least one CSSP) is provided.

In a second step (**S02**) of the method, the CSSC(s) is/are mixed with at least one fatty amine. As depicted in optional step **S02',** the CSSC(s) can be additionally mixed with one or more non-volatile liquid(s), which may facilitate the plasticizing or swelling of the CSSC(s) by the liquid. The addition of the non-volatile liquid(s) is optional as the viscosity of the CSSC(s) provided in **S01** can be sufficiently low for further processing (*e.g*., 10⁷ mPa·s or less, as measured at a temperature of 50°C and a shear rate of 10 sec⁻¹), or the fatty amine(s) mixed therewith may sufficiently plasticize the CSSC(s), if so desired.

The mixing of the CSSC(s) with the fatty amine(s) and any additional agent, such as the optional plasticizing liquid(s), can be performed at any suitable mixing temperature and/or mixing pressure suitable for such compounding.

The temperature at which the mixing (which may include a plasticizing of the CSSCs) is performed is typically selected according to temperatures characterizing the substances involved in the process, for instance, by taking into account a ***Ts, Tm*** and/or ***Tg*** characterizing the CSSC(s) and, optionally, a ***Tb*** of the fatty amine(s) (referred to as ***Tbₐ***) and/or a ***Tb*** of the non-volatile liquid(s) (referred to as ***Tbₗ**)* and/or the degradation point(s) of the fatty amine(s). As previously detailed, a mixing temperature would suitably be higher (*e.g.,* by at least 5°C, at least 10°C, at least 15°C, or at least 20°C) than at least one of the characterizing temperatures of the CSSC(s) and lower (*e.g.,* by at least 5°C, at least 10°C, at least 15°C, or at least 20°C) than the lowest of the boiling temperature and/or degradation point of the fatty amine and/or the boiling temperature of the non-volatile liquid at a pressure the mixing step is performed, though this upper limit is not essential as long as the selected mixing temperature does not significantly boil away or degrade any of the materials to be mixed with the CSSCs so as to form the cores of the nano-elements. Hence, in some cases, the mixing temperature can even be at ***Tbₐ**, **Tbₗ**,* or the fatty amine degradation point if the step is brief enough and/or the non-volatile liquid in sufficient excess and/or the mixing performed in a chamber sufficiently sealed to limit its evaporation / favor its condensation back to the mixture.

One can readily appreciate that a change in the properties of the substance reflected by these temperatures dropping from first to second values can alternatively take place at a lower mixing temperature or a higher mixing temperature, if the pressure in a sealed chamber hosting the mixing process ensuring plasticization of the CSSC(s) were to be accordingly reduced or increased. Therefore, while in the description of a method suitable for the preparation of a composition according to the present teachings, reference can be made to specific temperatures and duration of times assuming the process is carried out under standard atmospheric pressure, such guidance should not be viewed as limiting, and all temperatures and durations achieving a similar outcome with respect to the behavior of the plasticized CSSC(s) and/or the chargeability of the nano-elements in view of the activity of the fatty amines are encompassed.

It is noted in this context, when the CSSC is a CSSP, that while the ***Tm*** and/or ***Tg*** of a polymer may set relatively clear temperatures below and above which a polymer may display a distinct behavior, this typically does not apply to the ***Ts**.* In view of their viscoelastic properties, a polymer or a plasticized polymer may remain "sufficiently solid" even at a temperature moderately higher than its formal softening point.

The mixing or plasticizing can be performed under a variety of conditions, such as elevated temperatures (*i.e.,* 30°C or more, *e.g.,* at 40°C or more, at 50°C or more, at 60°C or more, at 75°C or more, or at 90°C or more) and/or elevated pressure (*i.e.,* 100 kPa or more, *e.g.,* at 125 kPa or more, 150 kPa or more, 175 kPa or more, 200 kPa or more, 250 kPa or more, or 300 kPa or more). As the mixing step often achieves at least some plasticizing of the CSSC, it may also be referred to as a plasticizing step the aforesaid temperatures and/or pressures typically accelerating the plasticizing process (*i.e.,* shortening the duration of the plasticizing period) or enabling a desired modification of a boiling temperatures ***Tbₐ*** or ***Thₗ*** at which the fatty amine or the non-volatile liquid might evaporate. As mixing at elevated pressure increases ***Tbₐ*** and/or ***Tbₗ**,* the range of temperatures at which plasticizing could be performed can be accordingly widened. Conversely, plasticizing the CSSC under conditions less favorable than arbitrarily set to assess the ability of a CSSC to be plasticized by a specific agent, such as at a temperature of less than 50°C and/or a reduced pressure of less than 100 kPa, may prolong the plasticizing process, if desired. The ability of a CSSC to be plasticized or swelled by a particular plasticizing agent may be assessed under any one of the above temperature or pressure conditions.

Mixing of the CSSC(s) with the fatty amine(s) and optionally the non-volatile liquid(s) by agitating the mixture can also shorten the plasticizing period, such agitating additionally ensuring that all parts of the CSSC(s) are plasticized in a relatively uniform manner, the plasticized CSSC behaving reasonably homogeneously with respect to subsequent steps of the method and results expected therefrom. If excess of the non-volatile liquid is used during the plasticizing process, it can be optionally removed before proceeding to following step(s). When the materials to be plasticized have a relatively high viscosity, the mixing step can also be referred to as compounding, and the mixing equipment can be accordingly selected.

The duration of plasticizing will *inter alia* depend on the CSSC(s) being plasticized, the non-volatile liquid(s) being used, the plasticizing conditions (*e.g*., temperature, pressure, and/or agitation), and the desired extent of plasticizing. The plasticizing period can be of at least 1 minute and at most 4 days.

In some embodiments, additional materials may optionally be incorporated within the CSSC(s) and added during the mixing step **S02,** in accordance with the requirements of the process and/or of the final composition. These materials, which are miscible with the CSSC(s) and typically insoluble in the polar carrier, can (as shown in step **S02'**) be at least one polar-carrier-insoluble surfactant, the surfactant(s) serving as an emulsifier, and/or at least one polar-carrier-insoluble active agent, the active agent(s) enhancing or modifying the biological activity of the composition, and/or at least one CSSC-miscible pH modifying agent, or any desirable additive. The mixing conditions may be adapted to the presence of such additional constituents.

The mixing may be performed by any method known to the skilled artisan, such as: sonication, using a double jacket planetary mixer or a high shear mixer, *etc.* When the materials being mixed have a relatively high viscosity, the mixing step can be performed with a two-roll mill, a three-roll mill, an extruder and such type of equipment. In a particular embodiment, the mixing is performed by sonication.

In a third step (**S03**) of the method, the mixture of the CSSC(s) and fatty amine(s) (optionally further containing at least one CS SC-miscible material, such as a non-volatile liquid, a surfactant, a carrier-insoluble active agent and a pH modifying agent) is combined with at least one polar carrier. At least one surfactant can be added to the polar carrier, if desired, at this step, the surfactant being a relatively polar emulsifier or a hydrotrope. Additional materials which are soluble in the polar carrier could also be added at this step but may equally be introduced after the following nano-sizing step.

The mixture is nano-sized in a fourth step (**S04**) to form a nano-suspension, whereby nano-elements having a core including the CSSC(s) and optionally containing other or CSSC-miscible and/or polar-carrier-insoluble materials and a shell including the fatty amine(s) (and optionally some surfactants or other materials having a hydrophobic tail and a hydrophilic head capable of integrating the shell with the amine moieties of the fatty amines) are dispersed in a polar liquid including the polar carrier optionally combined with other polar materials.

The nano-emulsion so produced may be "self-emulsified", so that no dispersing agent needs to be added to obtain the dispersion of the sought core-shell nano-droplets. Without wishing to be bound by theory, it is believed that the present method embeds at least a portion of the hydrophobic tails of the fatty amines (or an hydrophobic moiety of any other carrier-insoluble material mixed with the CSSC), naturally oriented towards the hydrophobic core, while the amine heads of the fatty amines (or the hydrophilic moieties of additional suitable materials) turn towards the polar carrier, forming the shell surrounding the core. The method therefore allows the preparation of nano-droplets having hydrophobic cores of the CSSC (optionally with a non-volatile liquid and/or a carrier-insoluble active agent *etc*.), surrounded by the amine heads pointing outwardly, forming the first shell, the nano-droplets being dispersed within the polar carrier. As the amine groups of the shell can be charged by the polar liquid, the similarly charged nano-droplets can repulse one another, remaining accordingly dispersed.

As the nano-sizing is typically performed by applying shear at a relatively elevated temperature, the core-shell nano-elements including the CSSC(s) are generally nano-droplets during that step and the resulting nano-suspension is a nano-emulsion.

The nano-emulsion can be obtained by nano-sizing the mixture of desired materials by any method capable of shearing the CSSC (whether plasticized or not, or including additional compounds), the shearing method being selected from the group comprising: sonication, milling, attrition, high pressure homogenization, high shear mixing and high shear microfluidization. In a particular embodiment, the nano-sizing is performed by sonication.

The nano-sizing is performed at a shearing temperature that is at least equal to at least one of the first ***Ts, Tm*** and ***Tg*** of the CSSC, at least equal to at least one of the second ***Ts, Tm*** and ***Tg*** of the CSSC if plasticized, and can be, in some embodiments, at least 5°C higher, at least 10°C higher, or at least 15°C higher than the highest characterizing temperature of the CSSC mix being sheared. However, while this is not essential if the shearing step is brief enough and/or the polar liquid in sufficient excess, the shearing temperature should preferably prevent significant amounts of the liquid phase being boiled away and avoid a significant degradation of the fatty amine(s). In some embodiments, the nano-sizing temperature at which shearing is performed does not exceed at least one of the boiling temperature of the liquid phase in which the shearing is being performed (or of any other liquid the evaporation of which should be prevented) and the degradation point of the fatty amine(s) (or the temperature withstood by any other material the thermal degradation (*e.g.,* deactivation, destruction, *etc.*) of which should be prevented). Thus, the shearing temperature is generally lower than the lowest of the ***Tb*** of the polar carrier(s) (referred to as ***Tb_{c}***) and the degradation point(s) of the material(s) mixed with the CSSC at a pressure the nano-sizing step is performed. For instance, when the polar carrier is water, the shearing temperature can be selected to be lower than 95°C, lower than 90°C, lower than 85°C, or lower than 80°C, assuming the nano-sizing is performed at atmospheric pressure. However, if the nano-sizing were to be performed at an elevated pressure, the ***Tb_{c}*** of the polar carrier would be raised, and the shearing temperature could be accordingly increased. Still illustrating with water, while its ***Tb*** is 100°C at about 100 kPa, this boiling temperature rises to 120°C at about 200 kPa, in which case the nanosizing temperature not to be exceeded could be of up to 115°C. As mentioned, these upper limits, while preferred, are not essential, as any boiling away or degradation of a part, *e.g.,* of the polar carrier or fatty amine(s) could be prevented at even higher temperatures if the step is brief enough, and/or the polar carrier in sufficient excess and/or the nano-sizing is performed in a chamber sufficiently sealed to limit its evaporation / favor its condensation back to the nano-suspension.

At shearing temperatures in this range of higher than ***Ts, Tm*** or ***Tg*** and optionally lower than ***Tb_{c}*** of the liquid carrier or degradation temperatures of suitable fatty amines, the CSSC(s), and in particular the CSSP(s), can completely melt and the nano-sizing process can be considered as "melt nano-emulsification".

In a fifth step (**S05**) of the method, the nano-emulsion may be optionally actively cooled down to a temperature below the ***Tm**, **Ts*** or ***Tg*** of the CSSC (or plasticized CSSC), to accelerate the relative solidification of the core-shell nano-elements, if desired in manufacturing. Such cooling can be actively achieved by refrigerating the nano-suspension (*e.g*., placing in a coolant having a desired low temperature), by subjecting the nano-suspension to ongoing agitation to accelerate heat dissipation (and incidentally maintain proper dispersion of the nano-droplets as they cool down), or by combining both approaches. This step is optional, as the nano-emulsions may be allowed to passively cool down without any agitation upon termination of nano-sizing. The nano-emulsions may also be passively cooled when combined with water (pH-modified water) and/or a polar-carrier-soluble active agent in subsequent optional steps, provided they are at sufficiently low temperature that would allow cooling of the nano-emulsion. Despite cooling, if performed during the manufacturing process, the CSSCs (or plasticized CSSCs) may remain in a liquid form as nano-droplets, or may readily convert back to nano-droplets once administered to a subject having a skin surface or body temperature of more than 30°C, such temperatures typically being respectively of more than 32°C and 35°C in mammals.

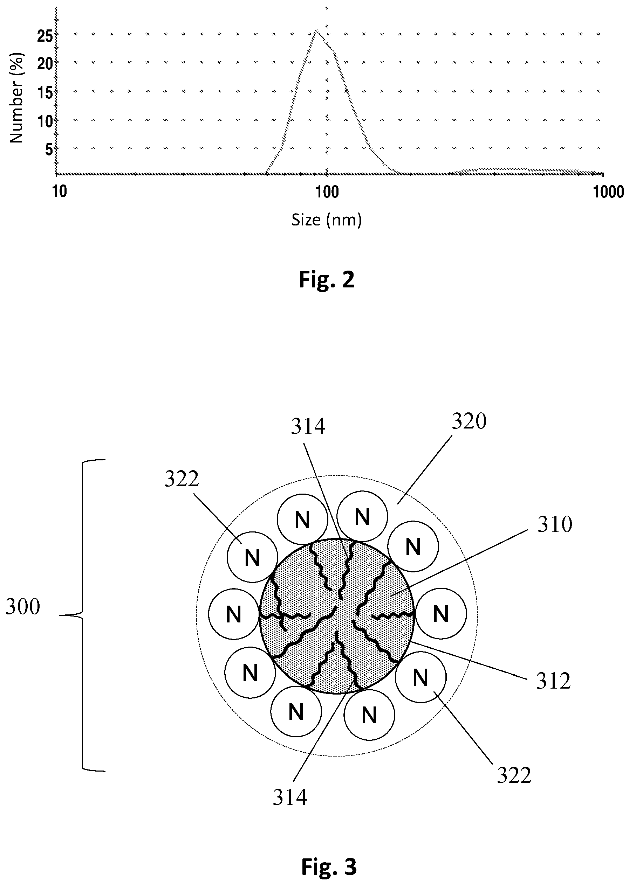

**Figure 3** schematically depicts a core-shell nano-particle **300** in a non-aqueous polar carrier, the absence of water preventing the charging of the amine heads of the fatty amines. The uncharged nano-element contains a core **310**, composed of a CSSC and optionally of a non-volatile liquid and/or carrier-insoluble surfactant and/or carrier-insoluble active agent, the core entrapping at least part of the hydrophobic tails **314** of the fatty amines. The polar heads **322** of the fatty amines, represented by a circled "N" (encompassing primary, secondary, tertiary and quaternary amines), are pointing away from the core's surface **312**, forming the nano-element's shell **320.**

In some embodiments, at least 50% of the total number (D_{N}50) or volume (D_{V}50) of the core-shell nano-elements (*e.g*., nano-droplets or nano-particles) formed in this nano-sizing step have a hydrodynamic diameter of up to 200 nm, up to 190 nm, up to 175 nm, up to 150 nm, up to 125 nm, or up to 100 nm, up to 90 nm, up to 80 nm, or up to 70 nm. In some embodiments, the median diameter of the nano-elements is at least 5 nm, at least 10 nm, at least 15 nm, or at least 20 nm. Advantageously, such values are applicable as determined by the number of the core-shell nano-elements and are generally measured at room temperature.

As readily appreciated, depending on the temperatures characterizing the materials of the nano-elements and/or on the temperature at which measurements may be performed, the core-shell nano-elements can either be relatively liquid nano-droplets or relatively solid nano-particles, as the temperature is reduced. The size of the nano-particles at room temperature is commensurate with the size of the nano-droplets or slightly more compact, their median diameter not exceeding 200 nm.

In some embodiments, the size of the core-shell nano-particles or nano-droplets is determined by microscopy techniques, as known in the art (*e.g.,* by Cryo TEM). In some embodiments, the size of the nano-elements is determined by Dynamic Light Scattering (DLS). In DLS techniques the particles are approximated to spheres of equivalent behavior and the size can be provided in term of hydrodynamic diameter. DLS also allows assessing the size distribution of a population of nano-elements.

Distribution results can be expressed in terms of the hydrodynamic diameter for a given percentage of the cumulative particle size distribution, either in terms of numbers of particles or volumes, and are typically provided for 10%, 50% and 90% of the cumulative particle size distribution. For instance, D50 refers to the maximum hydrodynamic diameter below which 50% of the sample volume or number of particles, as the case may be, exists and is interchangeably termed the median diameter per volume (D_{V}50) or per number (D_{N}50), respectively, and often more simply the average diameter.

In some embodiments, the nano-elements of the disclosure have a cumulative particle size distribution of D90 of 500 nm or less, or a D95 of 500 nm or less, or a D97.5 of 500 nm or less or a D99 of 500 nm or less, *i.e.,* 90%, 95%, 97.5% or 99% of the sample volume or number of particles respectively, have a hydrodynamic diameter of no greater than 500 nm.

In some embodiments, the cumulative particle size distribution of the population of core-shell nano-elements (*e.g*., nano-particles) is assessed in term of number of particles (denoted D_{N}) or in term of volume of the sample (denoted D_{V}) comprising particles having a given hydrodynamic diameter.

Any hydrodynamic diameter having a cumulative particle size distribution of 90%, or 95%, or 97.5%, or 99% of the particles' population, whether in terms of number of particles or volume of sample, may be referred to hereinafter as the "maximum diameter", *i.e.,* the maximum hydrodynamic diameter of particles present in the population at the respective cumulative size distribution.

It is to be understood that the term "maximum diameter" is not intended to limit the scope of the present teachings to nano-particles having a perfect spherical shape. This term as used herein encompasses any representative dimension of the particles at cumulative particle size distribution of at least 90%, *e.g.,* 90%, 95%, 97.5% or 99%, or any other intermediate value, of the distribution of the population.

The core-shell nano-particles or nano-droplets may, in some embodiments, be uniformly shaped and/or within a symmetrical distribution relative to a median value of the population and/or within a relatively narrow size distribution.

A particle size distribution is said to be relatively narrow if at least one of the following conditions applies:
A) the difference between the hydrodynamic diameter of 90% of the nano-elements and the hydrodynamic diameter of 10% of the nano-elements is equal to or less than 250 nm, equal to or less than 200 nm, equal to or less than 150 nm, or equal to or less than 100 nm, which can be mathematically expressed by: (D90 - D10) ≤ 250 nm and so on;
B) the ratio between a) the difference between the hydrodynamic diameter of 90% of the nano-elements and the hydrodynamic diameter of 10% of the nano-elements; and b) the hydrodynamic diameter of 50% of the nano-elements, is no more than 2.5, no more than 2.0, no more than 1.5, or even no more than 1.0, which can be mathematically expressed by: (D90 - D10)/D50 ≤ 2.5 and so on; and
C) the polydispersity index of the nano-elements is equal to or less than 0.5, equal to or less than 0.4, equal to or less than 0.3, or equal to or less than 0.2, which can be mathematically expressed by: **PDI = σ²/d²** ≤ 0.5 and so on, wherein **σ** is the standard deviation of the particles distribution and **d** is the mean size of the particles, the PDI optionally being equal to 0.01 or more, 0.05 or more, or 0.1 or more.

The PDI information is generally readily obtained from the instrument used to measure the hydrodynamic diameter of the nano-particles.

When the nano-elements are electrostatically charged, preferably positively charged, this facilitates their further coating by an external shell of carrier-soluble active agents suitably having an opposite charge, the resulting core-multi-shells forming more moderately charged or even uncharged nano-elements. The moderately charged core-multi-shells can typically remain positive but less than their respective core-shell, lacking the second shell of the active agent. Alternatively, the core-multi-shells can become negatively charged, provided that collapsing of the composition (*e.g*., by aggregation of the nano-elements at their isoelectric point) can be avoided.

The skin's surface is generally negatively charged, and therefore the overall charge of the nano-elements contributed *inter alia* by the CSSCs of the cores, the fatty amines of the first shells and the active agents may modulate the efficacy of skin penetration. It is believed that nano-elements more positively charged (regardless of the number of shells), while expected to be better retained on the skin surface, would have a slower rate of transdermal delivery than less positively charged or even negatively charged nano-elements (*e.g*., typically core-multi-shells nano-elements sufficiently surrounded by a negatively charged active agent). Negatively charged nano-elements may experience a slight repulsion when transiting via the skin, this phenomenon pushing them further "down" across the skin.

Based on this theory, it is believed that the relative penetration of the nano-elements into the skin can to some extent be controlled, not only by their dimensions and/or malleability (*e.g*., viscosity), but also according to their charge (*e.g.,* by the magnitude of their positive charge). If the problem to be treated by the present dermatological compositions occurs at the skin surface, resulting for illustration from an infection by microorganisms, it may be beneficial to use core-shell nano-elements additionally containing within their cores and/or in secondary shells active agents capable of alleviating or treating the infection. In this case, the nano-elements may be sufficiently positively charged to prolong their retention on or near the skin surface, so that the active agents may more rapidly act at the target sites upon their release from the nano-elements. Conversely, if the sites to be targeted by the activity of the CSSC and/or active agents carried thereby are farther away from the skin surface, it may be beneficial to use core-shell nano-elements having a charge promoting their transdermal delivery.

When compositions comprising charged core-shell nano-elements are to be repeatedly applied to the skin, *e.g.,* for the treatment of chronic skin disorders (*e.g.,* atopic dermatitis, psoriasis *etc.*) or for accidental skin wounds typically requiring lengthy healing (*e.g.,* skin burns, skin grafting, significant surgical incision *etc.*)*,* it may be beneficial to retain the nano-elements applied in a first dose on the surface of the skin to be treated for as long as possible, so as to prolong efficacy and/or delay the need for a following dose. Yet, it is possible that a subsequent dose of the nano-elements is applied when the surface to be retreated still includes "leftovers" of the previous dose. Assuming for illustration that the core-shell nano-elements of the first dose are positively charged, conceivably at least a portion of the leftovers may remain on the negatively charged skin, even after the surface to be retreated is washed before a subsequent application. Without wishing to be bound by theory, it is believed that repeated application of such charged nano-elements could result over time in electrostatic repulsion of a newly applied dose having the same charge. In practice, the effect of such a dose may no longer be perceived by the surface to be treated. In such a case of prolonged treatment that may adversely lead to the dismissal of newly applied nano-elements of same charge, it may be beneficial to alternate the charges of the core-shell nano-elements being applied to the skin. In the present illustration, core-shell nano-elements having a negative charge could be used to overcome the rejection of positively-charged elements by previously applied nano-elements of same charge. Negatively-charged nano-elements could be constituted only of a core negatively chargeable in physiological conditions or can be core-multi-shells nano-elements having a negative outer shell surrounding a first shell being positive.

Without wishing to be bound by theory, it is believed that the charge of the nano-elements having at first only a single shell is mainly contributed by the amine heads of the fatty amines. When exposed to an aqueous environment, the fatty amine heads become positively charged due to protonation of the amine group at a suitable pH, the protons being donated from the water. Hence, when the polar carrier is an aqueous polar carrier (*e.g.*, water, or an aqueous mixture), the nano-elements can develop a positive charge.

However, when a non-aqueous polar carrier is used during the preparation of the nano-elements, a coordinate bond is believed to form with the amine heads, a polar carrier unable to protonate the molecules of the first shell "masking" any positive charge that may have otherwise potentially developed on the surface of the core-shell nano-elements in presence of a liquid enabling protonation. In such case, or when the positive charge detected in the liquid carrier is deemed insufficient, un-masking of the charge or its increase may be performed by replacing at least part of the polar carrier by water (such as, by combining the nano-suspension with water), thus creating an aqueous environment wherein the charge can be made available or develop.

While the water itself is capable of creating an environment wherein protons are available to some extent for creating a positive charge on the amine heads, a pH-modifying agent, preferably an acid, may be added to further promote protonation. The acid is believed to react with the polar carrier molecules (supposedly by hydrogen bonding), distancing them from the shell surface of the nano-particles, and leaving the amine heads exposed to the proton-rich aqueous environment, thus allowing the formation of a positive charge on the shell surface. Alternatively, a base may be added to the polar carrier, contributing to the positive charging of the amines, even if slightly less than an acidic agent. A basic pH-modifying agent may optionally further contribute to the negative charging of a carrier-soluble active agent, if added to the composition. Such addition of an acid or a base to the nano-suspension may be referred to as "acid doping" or "base doping", respectively.

The negative charge of materials due to form a second shell, whether or not enhanced by doping, is expected to promote their attraction to the positively charged core-shell nano-elements, facilitating the formation of core-multi-shells nano-elements via non-covalent electrostatic charges. Alternatively, or additionally, the active agents of the second shell may be selected to covalently bind to materials of the first shell (*e.g.,* fatty amines, surfactants, *etc.*)*.*

The amount of the pH-modifying agent added to the composition should be controlled, so as to create an environment that keeps the balance between a sufficient positive charging of the fatty amines, and a sufficient negative charging of the carrier-soluble active agent, their respective opposite charges being adequate for attraction of one to the other. For instance, the amount of an acidic agent should be sufficiently high to allow optimal positive charging of the amine heads, but sufficiently low so as to keep the carrier-soluble adequately negatively charged.

As the size of particles decreases, their specific surface area increases, hence the amount of materials that may be present on their outer surfaces. As the nano-elements of the present invention are *inter alia* characterized by a D_{N}50 of 200 nm or less, in some embodiments, the nano-elements can be sufficiently small to reach a specific surface area that is high enough to allow the presence of enough amine heads in the shell being able to contribute to a desirable positive charge of the nano-dispersion. In such a case, the use of a pH-modifying agent, which may be used to further boost a charge, can be superfluous (as demonstrated in Example 4 below).

While the formation of core-multi-shells nano-elements has been illustrated for core-shell nano-elements having a first shell including positively chargeable amine portions of fatty amines, so that the second shell may include materials negatively chargeable under the same conditions, this should not be construed as limiting. Core-multi-shells nano-elements can alternatively be prepared using core-shell nano-elements having a first shell including negatively chargeable portions of fatty compounds (*e.g*., the carboxylic acid moiety of a fatty acid), so that the second shell that can be formed thereon may include materials positively chargeable under the same conditions.

When the polar carrier added in step **S03** for the sake of nano-sizing is non-aqueous, the method may optionally include a sixth step (**S06** of **Figure 1**), whereby replacement of at least part of the polar carrier by water or pH-modified water is carried out. As previously described, when the polar carrier is water or a mixture thereof with a non-aqueous polar carrier, such replacement step may not be necessary.

Alternatively, an aqueous pH-modified solution of the polar carrier may be utilized in the third step of the method (**S03** of **Figure 1**), resulting in hydrogen ions being present in the mixture, allowing for the protonation of the amines group of the fatty amines, whereby a possible masking effect of the polar carrier is at least partially avoided.

In some embodiments, the pH-modifying agent is added in an amount resulting in the composition having a pH between 1 and 10, between 1 and 9, between 1 and 8, between 2 and 7, or between 2 and 5. In particular embodiments, the pH modifying agent is added in an amount which provides for a pH of 7 and less, 6 or less, 5 or less, or 4 or less.

In some embodiments, the core-shell nano-elements have a positive charge of +5 mV or more, +10 mV or more, +20 mV or more, +30 mV or more, or +40 mV or more, when placed in an aqueous environment at room temperature.

In other embodiments, the core-shell nano-elements have a negative charge between -60 mV and -5 mV, between -50 mV and -5 mV, between -40 mV and -5 mV, between -30 mV and -5 mV, or between -20 mV and -5 mV, when placed in an aqueous environment at room temperature.

In further embodiments, the core-shell nano-elements have a near neutral charge between -5 mV and +5 mV, when placed at room temperature in an aqueous environment, for instance, in presence of a non-ionic surfactant.

The core-shell nano-elements described above may be formulated into a dermatological composition (*e.g.,* cream, gel *etc.*) and administered to the skin of a subject (*e.g.,* human or non-human animals, including but not limited to mammalians) to promote, *inter alia,* collagen synthesis, or any other cosmetical or pharmaceutical effect, which might be enabled by an optional active agent.

Alternatively, the core-shell nano-elements can be used as nano-carriers for other active agents. Hence, in an optional seventh step of the method (**S07** of **Figure 1**), a carrier-soluble active agent is added to the core-shell nano-elements previously obtained. The active agents that are added can envelop the outer surface of the core-shell nano-elements, forming a second shell. The second shell may be covalently and/or non-covalently attached to the first shell. Such nano-elements can be referred to as core-multi-shells nano-elements, or core-shells nano-elements or active agent-coated core-shell nano-elements.

The amount of the carrier-soluble active agent to be added to the nano-suspension (optionally being a nano-dispersion) may depend *inter alia* on the particle size of the nano-elements and the surface area accordingly available to attachment. Moreover, the respective zeta potentials of the core-shell nano-elements and of the carrier-soluble active agent may also indicate the respective proportions at which they can be present in the composition to optimize the formation of the core-multi-shells nano-elements, avoiding if desired the presence of excess active-agents in the polar carrier. While such excess is permitted, in particular as long as promoting a stabilization of the second shell, too much of unbound active agents can be superfluous if the molecules of the carrier-soluble active agents have a high molecular weight precluding their transdermal delivery, if desired.

The attachment of the carrier-soluble active agent to the core-shell nano-elements is enabled or promoted as a result of a difference in zeta potential between the zeta potential (ζ1) of original core-shell nano-elements, induced by the fatty amines of the first shell, and the zeta potential of the active agent (ζ2), due to form the second shell. In some embodiments, the absolute value of the zeta potential differential (Δζ) defined as Δζ = |ζ2 - ζ1| is at least 5 mV, at least 10 mV, at least 15 mV, at least 20 mV, at least 25 mV, or at least 30 mV, as measurable in the presence of water or an aqueous polar carrier.

In some embodiments, the active agent-coated nano-elements of the present invention have an average hydrodynamic diameter D_{N}50 of 200 nm or less, 190 nm or less, 175 nm or less, 150 nm or less, 125 nm or less, 100 nm or less, 90 nm or less, 80 nm or less, or 70 nm or less. In some embodiments, the D_{N}50 of the active agent-coated core-shell nano-elements is 5 nm or more, 10 nm or more, 15 nm or more, or 20 nm or more.

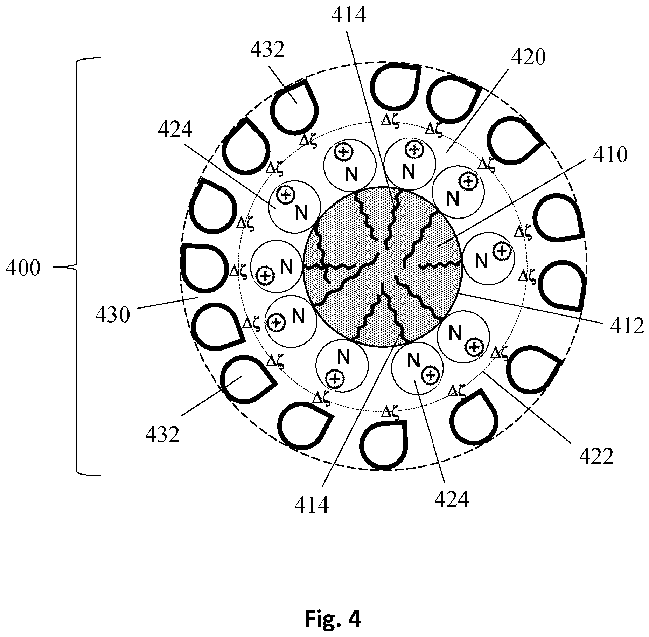

**Figure 4** schematically depicts such a core-multi-shells nano-particle **400**, coated with a carrier-soluble active agent, in an aqueous environment.

Similar to the nano-particle **300** previously described in **Figure 3****,** nano-particle **400** is composed of a core **410**, in which at least part of the hydrophobic tails **414** of the fatty amines are embedded. Unlike nano-particle **300**, which is dispersed in a non-aqueous polar carrier, the aqueous environment, in which nano-particle **400** is dispersed, allows for the polar heads **424** of the fatty amines to be positively charged, forming a now positively charged nano-particle first shell **420**, surrounding the core's surface **412.** This charging of the first shell allows the attachment of carrier-soluble active agent molecules **432** to the surface of the nano-particle first shell **422,** resulting in the formation of a second shell **430** comprising the active agent, wherein the Δζ between the active agent and the first shell of the nano-elements is as described above. The polar soluble active agent molecules may be negatively charged, resulting in a relatively larger Δζ between the first shell **420** of the nano-particle, being positively charged, and the active agent molecules **432.** Alternatively, the active agent molecules may be positively charged in the polar carrier, resulting in a relatively smaller Δζ, and a relatively weaker attraction compared to negatively charged molecules. Yet, as long as the Δζ is of 5 mV or more, the conditions are sufficient for the formation of the second shell **430.** In particular embodiments, the carrier-soluble active agent molecules are negatively charged. While Figure 4 depicts an exemplary first shell surrounding the cores enabling their positive charging, so that the carrier-soluble active agents adapted to form a second shell thereon can be negatively charged, the core-shell nano-elements can alternatively be negatively charged, so as to permit the formation of second shells made from positively charged carrier-soluble active agents. Negatively charged inner core-shell can be obtained by modulating the environment of the nano-element, or by using for the formation of the first shell, a fatty compound having a negatively chargeable hydrophilic head instead of a positively chargeable one. Replacing, for illustration, the fatty amines by fatty acids.

While in the method detailed above, some ingredients have been described as being introduced (or optionally introduced) in the composition at a particular step, this should not be construed as limiting. For instance, pH-modifying agents may, depending on the material they are selected from, be added with the non-volatile liquid during optional step **S02',** if intended for delayed leaching, or with the polar carrier during step **S03,** or to the liquid polar phase of the nano-emulsion as currently described in optional step **S06.** Alternatively, such agents may be omitted, provided that the nano-elements including the CSSC core can be retained on skin surface or transdermally delivered in an amount sufficiently effective for the sought effect.

Thus, the above-described steps can be modified, omitted (*e.g.,* **S02', S05, S06** or **S07**) and additional steps may be included. For instance, the dermatological composition may comprise any additive customary to cosmetical or pharmaceutical compositions, such as moisturizers, emollients, humectants, UV-protective agents, thickeners, preservatives, antioxidants, bactericides, fungicides, chelating agents, vitamins and fragrances, or any other material adapted for their formulation in a form suiting their application to the skin. The nature and concentration of each such conventional compounds suitable for such compositions are known to the skilled person and need not be further detailed herein. The additives may be added during steps of the method already described or via new steps. Furthermore, the composition may be further treated (*e.g.,* sterilized, filtered, irradiated, *etc.*) in accordance with health regulations, to make it suitable for dermatological uses, in particular on human skin.

Advantageously, the present method, regardless of the steps involved and the compositions being prepared, does not seek to chemically modify its ingredients, as might have been required for instance to jointly attach them. The absence of such modifications in the present compositions is expected to prevent formation of large particles that would be unable to pass the skin barrier, and/or is believed to prevent an undesirable decrease in the biological activity these ingredients might provide in their native (unmodified) form, assuming they successfully penetrated the skin.

### Routes and forms of administration

The present compositions may be delivered by any route suitable for their intended use depending on the object, the subject and/or the condition to be treated therewith. Such modes of administration or application to a surface are known. Considering for illustration a living subject, it is known that for an effect substantially limited to the site of administration local routes (*e.g.,* dermal, buccal, rectal, *etc.*) are to be used, whereas for a system-wide effect systemic routes (*i.e.,* enteral or parenteral, such as injection, oral, sublingual, inhalation *etc.*) can be selected according to the intended use of the composition.

A main use of core-shell nano-elements according to the present teachings is in dermatological compositions for application onto the skin, allowing for the transdermal delivery of the CSSCs (and of any other active agents present in the composition) and their activity on skin surface, within skin layers, beneath skin layers, and/or even systemically. Advantageously, transdermal delivery typically occurs over larger skin areas than permitted when a same product is injected locally (*e.g*., subcutaneously). Hence, the effect of a CSSC (or other active agent) so delivered is expected to be wider with respect to the area being treated, generally providing for a more uniform effect over a larger surface.

The composition can be applied topically to the skin where it may serve as a cosmetic composition (*e.g*., to improve appearance, as an anti-aging treatment, a skin protective treatment, a skin filling treatment, a skin smoothing treatment, and the like) or as a pharmaceutical composition (*e.g*., to relieve or treat a disorder).

While for simplicity the effect of the present compositions, when deemed cosmetical, is referred to as "anti-aging", for delaying, reducing, or preventing skin aging, this should not be construed as limiting, as processes similar to those leading to natural time-dependent skin aging are encountered in additional circumstances, such as degenerative disorders, or benign and malignant neoplasms, to name a few. Therefore, while for brevity the present invention is detailed for its cosmetic role and improvement of skin appearance (or postponement and/or diminution of deterioration of look), the compositions and the methods of preparation disclosed herein can have a broader beneficial impact on skin health, at least in the realm of dermatological treatment of conditions wherein one or more of the structural skin proteins that can be restored by the present compositions are pathologically reduced.

Thus, use of a dermatological composition comprising the core-shell nano-elements of the present invention should be broadly understood as use of a cosmetical or a pharmaceutical composition achieving any desirable cosmetic or pharmaceutic improvement of the skin.

Typically, dermatological compositions can be formulated as creams, foams, gels, lotions or ointments, the preparation of which is known to the skilled person and need not be detailed herein. The compositions can be applied to the skin by any known method *e.g.,* it may be rubbed onto the skin once, or more, at any frequency desired to achieve the sought cosmetical or pharmaceutical activity in absence of adverse effects. The present dermatological composition can also be delivered using any suitable dressing, such as a dermal or transdermal patch, a pad, a gauze, *etc.,* this route being advantageous when intending to slowly release an active agent incorporated in the present nano-elements, the composition being loaded in the dressing, *e.g*., deposited in a reservoir of the patch or impregnating a cloth pad. A dermal patch or skin patch is a medicated adhesive patch adapted to deliver the active agent into the skin, whereas a transdermal patch will deliver the active agent through the skin and into the bloodstream.

The compositions of the present invention may also be agrochemical compositions, generally in the form of solutions, emulsions, powders, granules, pellets or aerosols to be applied to a suitable target surface, such as soil or water in which plants are growing or pests are present and the surfaces of the plants and the pest, the application being by spreading, dispersing or spraying of the agrochemical composition on the surface to be treated.

When the composition is intended to be an industrial composition, it can be in the form of paints, pastes, varnishes, lacquers, sprays or powders, to be applied to a surface of an inert object, the application being by spreading, spraying or dispersing of the industrial composition on the inert surface.

### Uses

Cosmetic, therapeutic, agrochemical or industrial uses are enabled by the delivery to a target surface of efficacious amounts of the present core-shell nano-elements comprising CSSCs and optional active agents, according to the effect being achieved.

Cosmetic effects for improving skin appearance, achievable by the present core-shell compositions once applied onto a skin to be treated therewith, include combating collagen elastin or glycosaminoglycan breakdown, promoting collagen, elastin or glycosaminoglycan neo-synthesis, treating signs of ageing of the skin, combating wrinkles and fine lines, combating wizened skin, combating flaccid skin, combating thinned skin, combating dull, lifeless skin, combatting uneven skin pigmentation, combating lack of elasticity and/or tonicity of the skin, as well as skin bleaching or tanning.

Pharmaceutical effects, achievable by the present core-shell compositions once applied onto a skin to be treated therewith, include improving skin appearance and/or health by treating chronic skin disorders, skin lesions, restoring skin integrity, promoting wounds or surgical incisions healing, facilitating skin grafting, alleviating or treating local skin inflammation (e.g., acne), skin irritation, skin infection, skin rashes, skin pain, skin burns, skin swelling, skin allergy, as well as promoting or inhibiting hair growth, or treating dandruff.

Alternatively, the non-cosmetic effects of the present compositions are not directed to the treatment of the skin *per se* but to the protection of the skin to which they are applied from external factors.

The present core-shell dermatological compositions may further be used for delivery of active agents (whether CSSC-miscible and present within the core, or water-soluble and present as a second shell), to transdermally exert their pharmaceutical effects on organs other than the skin, by delaying, alleviating, preventing or treating a transdermally treatable disorder. The additional pharmaceutical effects that can be achieved by the present core-shell compositions once delivered through the skin to the bloodstream or an organ be treated therewith, include all activities such active agents are known, or will be found, to have when delivered via the skin. Such effects can be an ADHD-relieving effect, an Amyotrophic Lateral Sclerosis (ALS,) Alzheimer's disease, Huntington's disease, or Parkinson's disease relieving effect (or like effect for additional neurodegenerative disorders), an analgetic effect, an anti-addiction withdrawal effect, an anti-anginal effect, an anti-depressant effect, an anti-emetic effect, an anti-psychotic effect, an anti-seizure effect, an anti-spasmodic effect, a blood pressure regulating effect, a contraceptive effect, a glaucoma relieving effect, a hormone replacement effect, a thyroid regulating effect and a protective effect from nerve agents poisoning.

Agrochemical and/or industrial effects can also be achieved by the present core-shell compositions once applied onto a surface of an inert object, a plant or a pest, or the soil or the water in which plants are growing or pests are present. Such an agrochemical and/or industrial product can be used as an acaricide, an algicide, an anthelmintic, an anti-moth, an avicide, a bactericide, a chemo-sterilant, a fertilizer, a fungicide, a herbicide, an insecticide including an ovicide, an insect repellent, an insect pheromone, a molluscicide, a nematicide, a nitrification inhibitor, a pesticide, a pest repellent, a plant growth promotor, a rodenticide, a soil fertilizer, a termicide, a virucide, and a plant wound protectant.

### EXAMPLES

### Materials

The materials used in the following examples are listed in Table 1 below. The reported properties were retrieved from the product data sheets provided by the respective suppliers or estimated by standard methods. Unless otherwise stated, all materials were purchased at highest available purity level. N/A means that a particular information is not available.

**Table 1**

| **Component** | **Chemical Name** | **Product Name** | **Supplier** | **CAS No.** |
|---|---|---|---|---|
| Biodegradable CSSCs | Polycaprolactone | PCL-14 | Sigma Aldrich, USA | 24980-41-4 |
| | | MW ~ 14 kDa | | |
| | | PCL-37 | Polysciences, USA | |
| | | MW ~ 37 kDa | | |
| | | PCL-80 | | |
| | | MW ~ 80 kDa | | |
| | Polylactic acid | Ecosoft^{®} 608XF | Micro Powders, USA | 9051-89-2 |
| | Poly(lactic-coglycolic acid) | PLGA | Nomisma Healthcare Private, India | 26780-50-7 |
| | | MW ~ 24-38 kDa | | |
| | Poly(butylene succinate-coadipate) | PBSA | Shaanxi Dideu Medichem, China | 67423-06-7 |
| | Coenzyme Q10 | Coenzyme Q10 | Thermo Fisher Scientific, USA | 303-98-0 |
| | Dewaxed shellac | Dewaxed shellac type 4621 | Worlée, Germany | 9000-59-3 |
| | Gum rosin | Gum rosin | Guangxi Wuzhou Foreign Trade, China | 8050-09-7 |
| Non-biodegradable thermoplastic polymers | Poly(ethylene-comethacrylic acid) | Nucrel^{™} 599 (PEMAA) | DuPont, USA | 25053-53-6 |
| | Poly(ethylene-coacrylic acid) | Primacor^{®} 5990i (PEAA) | Dow, USA | 9010-77-9 |
| | Poly(ethylene-covinyl acetate) | Elvax^{™} 250 (PEVA) | Dow, USA | 24937-78-8 |
| Fatty amines | N,N-Dimethyldodecylamine | DMDA | Sigma Aldrich, USA | 112-18-5 |
| | Cetrimonium chloride | CTAC | Sigma Aldrich, USA | 112-02-7 |
| | Oleyl amine | Oleyl amine | Arcos Organics, USA | 112-90-3 |
| | Octyl amine | Octyl amine | Sigma Aldrich, USA | 111-86-4 |
| | N,N-Bis-(2-hydroxyethyl) C₁₂-C₁₈-alkylamine | Genamin^{®} O 020 Special | Clariant, Switzerland | 25307-17-9 |
| Non-volatile liquid | Dibutyl adipate | Cetiol^{®} B | BASF, Germany | 105-99-7 |
| | Triethyl O-acetylcitrate | Citrofol^{®} AII | Jungbunzkauer, Switzerland | 77-89-4 |
| | Dibutyl sebacate | Dibutyl sebacate | Sigma Aldrich, USA | 109-43-3 |
| | Isoparaffin | Isopar^{™} M | ExxonMobil, USA | 64742-47-8 |
| Polar carriers | Glycerol | Glycerol | Sigma Aldrich, USA | 7325-17-9 |
| | Propylene glycol | Propylene glycol | Chen Shmuel Chemicals, Israel | 57-55-6 |
| Surfactants | Mixture of: olive oil and glutamic acid | Olivoil^{®} glutamate | Kalichem, Italy | 67762-27-0 |
| | | | | 8005-44-5 |
| | | | | 31566-31-1 |
| | Sodium dioctyl sulfosuccinate | Sodium dioctyl sulfosuccinate (AOT) | Sigma-Aldrich, USA | 577-11-7 |
| pH-modifying agents | Acetic acid | Acetic acid (99.8%) | Chen Shmuel Chemicals, Israel | 64-19-7 |
| | Hydrochloric acid | HCl (1M) | Sigma Aldrich, USA | 7647-01-0 |
| | Lactic acid | Lactic acid | Chen Shmuel Chemicals, Israel | 79-33-4 |
| Water-soluble active agents | Sodium hyaluronate | Low molecular weight hyaluronic acid (LMW HA, MW 3 kDa) | Xi'an Lyphar Biotech, China | 9067-32-7 |
| | L-ascorbic acid | Vitamin C | Sigma Aldrich, USA | 50-81-7 |
| | Collagen peptides | Verisol^{®} | Gelita, Germany | 92113-31-0 |
| | Elastin | Elastin | Koken, Japan | 9007-58-3 |
| CSSC-miscible active agents | Benzoyl peroxide | Benzoyl peroxide | Sigma Aldrich, USA | 94-36-0 |
| | Tadalafil | Tadalafil | Teva Pharmaceutical industries, Israel | 171596-29-5 |
| | Retinyl palmitate | Retinyl palmitate | Kingdomway Nutrition | 79-81-2 |
| | Bakuchiol | Sytenol^{®} A | Sytheon, USA | 10309-37-2 |
| | Curcumin | Curcumin | Jiaherb, China | 458-37-7 |
| | Neem oil | Neem oil | Manorama Industries, India | 84696-25-3 |
| | Castor oil | Castor oil | Sigma Aldrich, USA | 8001-79-4 |
| Fluorescent marker | 9-(diethylamino) -5H-benzo[a] phenoxazin-5-one | Nile red fluorescent dye | GIHI Chemicals, China | 7385-67-3 |
| Fixation fluid | Paraformaldehyde | Paraformaldehyde | N/A | 30525-89-4 |

### Equipment

Oven: DFO-240, by MRC, Israel
Thermo-rheometer: Thermo Scientific (Germany) Haake Mars III, with a C20/1° spindle, a gap of 0.052 mm, and a shear rate of 10 sec⁻¹
Sonicator: VCX 750, by Sonics & Materials, USA
Dynamic Light Scattering and Zeta Potential measurement: Malvern Zetasizer Nano ZS, Malvern Instruments Ltd., UK
Conductivity meter: Eutech CON 700 by Thermo Fisher Scientific, USA
CryoTEM: Talos 200C by Thermo Fisher Scientific, USA, with a Lacey grid
Fluorescence microscope: BX60 microscope, equipped with a digital camera model DP73, by Olympus, Japan

### Example 1: Reducing viscosity of the CSSC

In the present study, the viscosity of a CSSC being polycaprolactone having a molecular weight of 14 kDa (PCL-14, referred to as PCL in the present example) and of its mixtures with a non-volatile liquid or fatty amines, used as plasticizing agents, were measured at different weight per weight ratios of the PCL to the plasticizing agents.

In a first series of experiments, the viscosity was measured on blends of materials forming a homogeneous mass adapted for nano-sizing by the present methods. PCL and a non-volatile liquid or a fatty amine at various weight per weight ratios were combined in a glass vial and the sealed vials were placed in an oven pre-heated to 80°C. The PCL:non-volatile liquid ratios tested were 1:1 and 1:2.33 and the PCL:fatty amine ratios tested were 1:0.1, 1:0.33, 1:0.5 and 1:1. Following 1 hour of incubation under heat, the contents of the vials were mixed by hand for about 30 seconds, until clear solutions were obtained. The samples were allowed to cool down overnight (*i.e.,* at least 12 hours) at room temperature so as to solidify. None of the plasticizing agents so tested displayed leaching out of the solidified polymer, suggesting that they might be used at even higher proportion with respect to the PCL.

Solid samples were then transferred to a rheometer where their respective viscosity was measured as a function of temperature between room temperature and 70°C at a ramping up temperature of 10°C/min. The viscosities (or 2^{nd} viscosities) of the samples as measured at 50°C and 70°C are summarized in Table 2A, including in the first row the first viscosities of a reference sample made of PCL alone, unplasticized, under same conditions.

**Table 2A**

| **Plasticizing agent** | **PCL:plasticizing agent w/w ratio** | **PCL 2^{nd} viscosity at 50°C [mPa·s]** | **PCL 2^{nd} viscosity at 70°C [mPa·s]** |
|---|---|---|---|
| None | Not Relevant | 1.7x10⁵ | 7.0x10⁴ |
| Cetiol^{®} B | 1:1 | 5.0x10³ | 2.2x10³ |
| | 1:2.33 | 215 | 164 |
| Oleyl amine | 1:0.33 | 1.9x10³ | 1.7x10³ |
| | 1:0.5 | 6.7x10³ | 4.4x10³ |
| Octyl amine | 1:1 | 544 | 279 |

As can be seen from the above table, at the temperatures of 50°C and 70°C, all of the materials tested as plasticizing agents (either the non-volatile liquids dedicated to this purpose or the fatty amines inherently contributing to this effect) decreased viscosity of the PCL to less than 10⁴ mPa·s at the weight per weight ratios tested. Different weight ratios, such as with lower relative amount of plasticizing agent or fatty amine to CSSC (*e.g.,* 1:0.1 of oleyl amine), may be less efficient, however suitable ratios and agents capable of plasticizing a material can be readily determined by routine experimentation, as herein demonstrated.

Based on the above results or similar experiments, ratios of PCL:plasticizing agents providing a dynamic viscosity of 1x10⁴ mPa·s or less, as measured at least at 50°C, (though higher viscosities are permissible) were selected for the preparation of core-shell nano-particles to be used in topical compositions, as detailed in the following examples.

In a second series of experiments, the viscosity was measured at the other end of the process, that is to say on core-shell nano-elements isolated from compositions prepared as detailed in the following examples. Nano-particles only containing PCL-14 were used as reference and compared to nano-particles containing either 1 wt.% or 5 wt.% of Genamin^{®} O 020 Special (GenA2 in the following table) as fatty amine blended with the PCL and to nano-particles including 30 wt.% of PCL-14, 30 wt.% of Gen A2 and additionally containing 40 wt.% of a non-volatile liquid, either Cetiol^{®} B or dibutyl sebacate (DBS). Viscosity was measured as a function of temperature (in °C) as previously described but up to 85°C and the results in mPa·s are presented in decreasing order of temperature in Table 2B.

**Table 2B**

| **Temp.** | **100% PCL-14** | **99% PCL-14 + 1% GenA2** | **95% PCL-14 + 5% GenA2** | **30% PCL-14 + 30% GenA2 + 40% Cetiol^{®} B** | **30% PCL-14 + 30% GenA2 + 40% DBS** |
|---|---|---|---|---|---|
| 85 | 1.24 x10⁴ | 1.14 x10⁴ | 9.58 x10³ | 1.37 x10⁴ | 9.15 x10⁴ |
| 80 | 1.44 x10⁴ | 1.32 x10⁴ | 1.10 x10⁴ | 7.57 x10⁴ | 9.35 x10⁴ |
| 75 | 1.66 x10⁴ | 1.53 x10⁴ | 1.28 x10⁴ | 8.49 x10⁴ | 9.42 x10⁴ |
| 70 | 1.92 x10⁴ | 1.79 x10⁴ | 1.49 x10⁴ | 8.77 x10⁴ | 9.48 x10⁴ |
| 65 | 2.26 x10⁴ | 2.10 x10⁴ | 1.74 x10⁴ | 9.29 x10⁴ | 9.51 x10⁴ |
| 60 | 2.67 x10⁴ | 2.49 x10⁴ | 2.06 x10⁴ | 9.99 x10⁴ | 9.79 x10⁴ |
| 55 | 3.20 x10⁴ | 2.98 x10⁴ | 2.46 x10⁴ | 1.02 x10⁵ | 9.91 x10⁴ |
| 50 | 3.89 x10⁴ | 3.62 x10⁴ | 2.97 x10⁴ | 1.08 x10⁵ | 1.03 x10⁵ |
| 45 | 4.79 x10⁴ | 4.45 x10⁴ | 3.64 x10⁴ | 8.97 x10⁵ | 4.69 x10⁵ |
| 40 | 6.43 x10⁴ | 6.27 x10⁴ | 4.67 x10⁴ | 2.57 x10⁶ | 1.49 x10⁶ |
| 35 | 1.17 x10⁹ | 6.79 x10⁷ | 8.29 x10⁶ | 4.63 x10⁶ | 2.15 x10⁶ |
| 30 | 1.33 x10⁹ | 1.30 x10⁹ | 1.24 x10⁹ | 6.19 x10⁶ | 2.48 x10⁶ |
| 25 | 1.35 x10⁹ | 1.34 x10⁹ | 1.25 x10⁹ | 7.69 x10⁶ | 2.71 x10⁶ |

As can be seen from the above table, while at temperatures between 30°C and 85°C, the presence of even minute amounts of fatty amines (e.g., 1 wt.% GenA2) could be detected by way of their plasticizing effect on the CSSC, at lower temperatures the detection of a plasticizing effect required a higher proportion of a plasticizing fatty amine to CSSC. Interestingly, even low amounts of fatty amines capable of plasticization (e.g., 5 wt.% GenA2) provided for core-shell nano-elements having a viscosity of less than 1x10⁷ mPa·s at near body temperature measured at 35°C.

### Example 2: Preparation of core-shell nano-dispersions

2 g of PCL-14, 2 g of the fatty amine N,N-dimethyldodecylamine (DMDA) and 6 g of the non-volatile liquid Cetiol^{®} B, serving as a dedicated plasticizer, were placed in a 20 ml glass vial. The vial contents were sonicated for 2 minutes at about 70°C until a clear CSSC/fatty amine premix including the non-volatile liquid was obtained.

In a separate 20 ml glass vial, 8 g of glycerol were heated to about 70°C using a sonicator, followed by addition of 2 g of the hot premix prepared above, and the composition was sonicated for 5 minutes whilst maintained at 70°C to obtain a nano-emulsion. The nano-emulsion was then allowed to cool down to room temperature until a nano-dispersion containing core-shell nano-particles was obtained.

This nano-dispersion (*ND1*) is reported in Table 3, which presents additional nano-dispersions prepared according to similar procedures, each nano-dispersion containing different components in different amounts, and prepared under different conditions, as specified in the table. Unless otherwise stated, the values reported in this and following tables describing compositions and their preparation, correspond to the concentration of each component in weight percent (wt.%) by total weight of the nano-dispersion, except for the values in the CSSC/fatty amine premix section, which correspond to the weight percentage of each component in that particular premix which may further include any other CSSC-miscible ingredient, such as a non-volatile liquid. Water refers to double distilled water.

The size of the nano-particles produced according to this or similar following examples was measured by Dynamic Light Scattering (DLS) on samples of the compositions, diluted to 1:100 in water, and the measured median diameter per number (D_{N}50), as well as polydispersity indices (PDI), are also presented in the relevant tables below. The maximum hydrodynamic diameters below which 10% and 90% of the sample number of particles exist (D_{N}10 and D_{N}90, respectively), when measured, are also presented in the pertinent tables.

**Table 3**

| **Component** | **Composition** | | | | | |
|---|---|---|---|---|---|---|
| | ***ND1*** | ***ND2*** | ***ND3*** | ***ND4*** | ***ND5*** | ***ND6*** |
| **CSSC/fatty amine premix** | | | | | | |
| PCL-14 | 20 | 30 | 20 | 20 | 20 | 20 |
| DMDA | 20 | | 20 | | | |
| CTAC | | 3 | | | | |
| Genamin^{®} O 020 Special | | | | 20 | 20 | |
| Oleyl amine | | | | | | 20 |
| Cetiol^{®} B | 60 | 67 | 60 | 60 | 60 | 60 |
| **Premix preparation** | 2 min @70°C | | | 1 min @80°C | | |

| **Nano-dispersion** | | | | | | |
|---|---|---|---|---|---|---|
| PCL-14 | 4 | 6 | 2 | 4 | 4 | 8.9 |
| DMDA | 4 | | 2 | | | |
| CTAC | | 0.6 | | | | |
| Genamin^{®} O 020 Special | | | | 4 | 4 | |
| Oleyl amine | | | | | | 8.9 |
| Cetiol^{®} B | 12 | 13.4 | 6 | 12 | 12 | |
| Citrofol^{®} AII | | | | | | 26.7 |
| Olivoil^{®} glutamate | | | | 20 | | |
| Sodium dioctyl sulfosuccinate | | | | | 1.7 | |
| Glycerol | 80 | | 90 | | | |
| Propylene glycol | | | | | | 10.1 |
| Water | | 80 | | 60 | 78.3 | 45.4 |
| **Nano-sizing** | 5 min @70°C | | | 1 min @80°C | | |
| **D_{N}10 [nm]** | 117 | 57.4 | 75.6 | N/A | N/A | N/A |
| **D_{N}50 [nm]** | 176 | 78.3 | 100 | 50.4 | 98.7 | N/A |
| **D_{N}90 [nm]** | N/A | 126 | 293 | N/A | N/A | N/A |
| **PDI** | 0.277 | 0.460 | 0.477 | 0.224 | 0.151 | N/A |

As can be seen in Table 3, the present method is suitable to prepare nano-suspensions of core-shell nano-elements containing a CSSC and a fatty amine, the nano-elements having a D_{N}50 not exceeding 200 nm, this value being even lower than 100 nm for some of the nano-suspensions reported above. The PDI of the populations of the core-shell nano-elements was at most about 0.5.

Representative results of particle size distribution in a sample of *ND3,* showing the percentage (per number) of PCL/DMDA core-shell nano-particles having hydrodynamic diameters in the range of 10-1,000 nm, are presented in **Figure 2****.**

### Example 3: Preparation of positively charged core-shell nano-particles by acid-doping

8 g of double distilled water were placed in a 20 ml glass vial. 2 g of the nano-dispersion *ND1* obtained in Example 2 were added, and the contents of the vial were shaken by hand for about 10 seconds until a homogeneous mixture was obtained (referred to as *charged ND1* or *cND1*)*.* Zeta potential of the mixture was measured by Malvern Zetasizer Nano ZS and found to be -14.7 mV. Unless otherwise stated, all measurements made with this instrument (*e.g*., zeta potential and particle size distribution) were performed on samples diluted 1:100 in double distilled water. All pH measurements reported in this and following examples were made at least using a pH test strip adapted to the pH range of relevance, and confirmed in some cases by using a suitable pH meter.

1 drop of acetic acid was then added to the mixture, yielding a pH of 5.5, and the vial was shaken by hand for about 10 seconds until a nano-dispersion containing positively charged core-shell PCL/DMDA nano-particles was obtained, referred to as *cND1'.* The zeta potential of the nano-particles in the acid doped nano-dispersion was measured and confirmed to be positive with a charge of +52.4 mV.

The hydrodynamic diameter of the nano-particles obtained in the acid-doped *cND1'* was determined by DLS and the D_{N}50 of the sample was found to be similar to that of the nano-particles of *ND1,* indicated in Table 3.

### Example 4: Preparation of positively charged core-shell nano-particles, in absence of acid

9.9 g of double distilled water were placed in a 20 ml glass vial. 0.1 g of nano-dispersion *ND3* obtained in Example 2 were added, and the contents of the vial were shaken by hand for 10 seconds until a homogeneous mixture, referred to as *cND3,* was obtained. The zeta potential of the mixture was measured and found to be +43 mV. The particle size distribution of the nano-particles so obtained was determined by DLS and the sample was found to have a D_{N}50 similar to that of the nano-particles of *ND3,* indicated in Table 3.

### Example 5: Preparation of positively charged core-shell nano-particles by acid-doping of diluted sample

9 g of double distilled water were placed in a 20 ml glass vial. 1 g of the nano-dispersion *cND3,* obtained in Example 4 were added, and the contents of the vial were shaken by hand for 10 seconds until a homogeneous mixture, referred to as *cND3',* was obtained. The zeta potential of the mixture was measured and found to be -9.9 mV. This mixture differs from the one described in Example 4 by containing 10-times less *ND3* in a same total weight of aqueous carrier. The 10-fold diluted nano-dispersion presently prepared displayed a relatively lower charging, having a charge of -9.9 mV, as compared to a charge of +43 mV in previous case.

1 drop of acetic acid was then added to the relatively diluted mixture, yielding a pH of 5.5, and the vial was shaken by hand for about 10 seconds until an acid-doped nano-dispersion containing positively charged core-shell PCL/DMDA nano-particles, referred to as *cND3",* was obtained. The zeta potential of the acid-doped nano-dispersion *cND3"* was measured and confirmed to be positive with a charge of +7.8 mV. The hydrodynamic diameter of the obtained nano-particles was determined and the D_{N}50 was found to be similar to that of the nano-particles of *ND3,* indicated in Table 3.

### Example 6: Preparation of core-shell nano-particles in an acidic polar carrier

2 g of PCL-14, 2 g of DMDA and 6 g of Cetiol^{®} B were placed in a 20 ml glass vial. The vial contents were sonicated for 2 minutes at about 70°C until a clear CSSC/fatty amine premix was obtained.

In a separate 20 ml glass vial, 8 g of glycerol and 1 g of a 3 wt.% HCl solution were mixed, whereby a solution having a pH of 3 was obtained. 1 g of the hot CSSC/fatty amine premix prepared above was added to the acidic liquid carrier, and the contents of the vial were sonicated for 30 seconds to obtain a nano-emulsion. The nano-emulsion was allowed to cool down to room temperature until a nano-dispersion was obtained.

The zeta potential of the nano-dispersion was measured and was found to be +72.8 mV, this charge resulting from the dilution of the sample in water. Hence, positively charged nano-particles were readily obtained following their sonication in the acidic polar carrier, instead of a two-step process, as previously described.

The nano-dispersion so obtained (*ND7*) is reported in Table 4A. Additional nano-dispersions were prepared according to similar procedures, all being chargeable in the presence of water, each nano-dispersion containing different components in different amounts, and prepared under different conditions, as specified in Tables 4A-4C, parameters measured in samples of some of the nano-dispersions, diluted to 1:100 in double distilled water, and the resulting values being also presented in the tables below. The pH reported for the nano-dispersions *ND15* and *ND16* was obtained by adding about one drop of acetic acid to their respective aqueous phase.

**Table 4A**

| **Component** | **Composition** | | | | | | | | | |
|---|---|---|---|---|---|---|---|---|---|---|
| | ***ND 7*** | ***ND 8*** | ***ND 9*** | ***ND 10*** | ***ND 11*** | ***ND 12*** | ***ND 13*** | ***ND 14*** | ***ND 15*** | ***ND 16*** |
| **CSSC/fatty amine premix** | | | | | | | | | | |
| PCL-14 | 20 | 20 | 30 | 30 | 30 | 20 | 20 | 20 | | |
| PCL-37 | | | | | | | | | 20 | |
| PCL-80 | | | | | | | | | | 20 |
| DMDA | 20 | 20 | 12 | | | | | | | |
| Oleyl amine | | | | 25 | 25 | 20 | | | | |
| Genamin^{®} O 020 Special | | | | | | | 20 | 20 | 20 | 20 |
| Cetiol^{®} B | 60 | 60 | 58 | | | | 60 | 60 | 60 | 60 |
| Citrofol^{®} AII | | | | 45 | 45 | 60 | | | | |
| **Premix preparation** | 2 min @70°C | | | 1 min @70°C | | 1 min @80°C | | 2 min @80°C | | |

| **Nano-dispersion** | | | | | | | | | | |
|---|---|---|---|---|---|---|---|---|---|---|
| PCL-14 | 2 | 6 | 6 | 8.4 | 8.7 | 7.6 | 4 | 4 | | |
| PCL-37 | | | | | | | | | 4 | |
| PCL-80 | | | | | | | | | | 4 |
| DMDA | 2 | 6 | 2.4 | | | | | | | |
| Oleyl amine | | | | 7 | 7.2 | 7.6 | | | | |
| Genamin^{®} O 020 Special | | | | | | | 4 | 4 | 4 | 4 |
| Cetiol^{®} B | 6 | 18 | 11.6 | | | | 12 | 12 | 12 | 12 |
| Citrofol^{®} AII | | | | 12.6 | 13.1 | 22.8 | | | | |
| Glycerol | 80 | 60 | 70 | 37 | 20.3 | 17.7 | | | | |
| Propylene glycol | | | | 10 | 14.5 | 12.7 | | | | |
| Water | 9.7 | 9.7 | 9.7 | 24.2 | 35.1 | 30.7 | 79.9 | 80 | 80 | 80 |
| HCl | 0.3 | 0.3 | 0.3 | 0.8 | 1.1 | 0.9 | | | | |
| Lactic acid | | | | | | | 0.1 | | | |
| **Nano-sizing** | 30 sec @70°C | | | | | | 1 min @80°C | | | |
| **D_{N}10 [nm]** | 55.3 | 77.4 | 95.4 | 58.3 | N/A | N/A | N/A | N/A | N/A | N/A |
| **D_{N}50 [nm]** | 73.5 | 106 | 138 | 80.3 | N/A | N/A | 73.4 | 75.2 | 96.1 | 41.9 |
| **D_{N}90 [nm]** | 106 | 186 | 238 | 134 | N/A | N/A | N/A | N/A | N/A | N/A |
| **PDI** | 0.424 | 0.332 | 0.148 | 0.242 | N/A | N/A | 0.202 | 0.240 | 0.227 | 0.355 |
| **pH** | 3 | 3 | 3 | 3 | 3 | 3 | 3 | 3.5 | 4 | 4 |
| **Zeta potential [mV]** | +72.8 | +73.0 | +59.7 | +48.9 | N/A | N/A | N/A | +56.0 | +68.4 | +85.0 |

In addition to measuring the zeta potential of the nano-dispersions, the effect of the acid in the polar liquid carrier was monitored by measuring the pH changes along the preparation of the nano-dispersions. Taking *ND7* for illustration, when combining an acidic glycerol solution having a pH of 3 with the CSSC/fatty amine mixture, the pH increased to 6, indicating the elimination of the hydrogen ions of the liquid carrier, which are believed to have reacted with the amine groups of the fatty amine shell. The pH remained at 6 after cooling and increased to 6.5 following dilution with water, indicating that the amine groups remain protonated, as confirmed by the positive zeta potential.

Additional nano-dispersions prepared using various CSSCs of synthetic and natural origins are summarized in Table 4B. The aqueous phases of the nano-dispersions reported in the table were acidified (by adding about one drop of acetic acid for *ND17-ND23,* and a specified amount for *ND24* and *ND25*)*.*

**Table 4B**

| **Component** | **Composition** | | | | | | | | |
|---|---|---|---|---|---|---|---|---|---|
| | ***ND17*** | ***ND18*** | ***ND19*** | ***ND20*** | ***ND21*** | ***ND22*** | ***ND23*** | ***ND24*** | ***ND25*** |
| **CSSC/fatty amine premix** | | | | | | | | | |
| PCL-14 | | | | | | | | 30 | 30 |
| PLA | 20 | | | | | | | | |
| PLGA | | 20 | | | | | | | |
| PBSA | | | 20 | | | | | | |
| Coenzyme Q10 | | | | 20 | | | | | |
| Shellac | | | | | 20 | 20 | | | |
| Gum rosin | | | | | | | 20 | | |
| Genamin^{®} O 020 Special | 20 | 20 | 20 | 20 | 80 | 20 | 20 | 30 | 30 |
| Cetiol^{®} B | 60 | 60 | 60 | 60 | | 60 | 60 | 40 | |
| Dibutyl sebacate | | | | | | | | | 40 |
| **Premix preparation** | 2 min @80°C | 3 min @80°C | | 2 min @80°C | | 3 min @80°C | 2 min @80°C | | |

| **Nano-dispersion** | | | | | | | | | |
|---|---|---|---|---|---|---|---|---|---|
| PCL-14 | | | | | | | | 6 | 6 |
| PLA | 2 | | | | | | | | |
| PLGA | | 2 | | | | | | | |
| PBSA | | | 2 | | | | | | |
| Coenzyme Q10 | | | | 2 | | | | | |
| Shellac | | | | | 4 | 2 | | | |
| Gum rosin | | | | | | | 2 | | |
| Genamin^{®} O 020 Special | 2 | 2 | 2 | 2 | 16 | 2 | 2 | 6 | 6 |
| Cetiol^{®} B | 6 | 6 | 6 | 6 | | 6 | 6 | 8 | |
| Dibutyl sebacate | | | | | | | | | 8 |
| Acetic acid | | | | | | | | 1.5 | 0.25 |
| Water | 90 | 90 | 90 | 90 | 80 | 90 | 90 | 78.5 | 79.75 |
| **Nano-sizing** | 1 min @80°C | | | | | | | | |
| **D_{N}50 [nm]** | 90.8 | 89.6 | 115 | 41.9 | 82.4 | 189 | 85.3 | 47.9 | 56.8 |
| **PDI** | 0.389 | 0.176 | 0.246 | 0.267 | 0.354 | 0.249 | 0.133 | 0.21 | 0.169 |
| **pH** | 4 | 4 | 4 | 4 | 3.5 | 4 | 4 | 5 | 5 |
| **Zeta potential [mV]** | +55.2 | +95 | +108 | +57.3 | +83.6 | +101 | +45.1 | +48.2 | +68.7 |

The core-shell nano-elements of *ND24* and *ND25* were isolated and subjected to thermo-rheological analysis as reported in Table 2B.

Nano-dispersions prepared using non-biodegradable thermoplastic polymers are summarized in Table 4C. The pH reported for the nano-dispersions in the table was obtained by adding about one drop of acetic acid to their aqueous phase.

**Table 4C**

| **Component** | **Composition** | | |
|---|---|---|---|
| | ***ND26*** | ***ND27*** | ***ND28*** |
| **Polymer/fatty amine premix** | | | |
| EMAA | 20 | | |
| EAA | | 20 | |
| EVA | | | 10 |
| Genamin^{®} O 020 Special | 20 | 20 | 20 |
| Isopar ^{®} M | 60 | 60 | 70 |
| Dibutyl sebacate | | | |
| **Premix preparation** | 3 min @80°C | | |

| **Nano-dispersion** | | | |
|---|---|---|---|
| EMAA | 2 | | |
| EAA | | 2 | |
| EVA | | | 1 |
| Genamin^{®} O 020 Special | 2 | 2 | 2 |
| Isopar ^{®} M | 6 | 6 | 7 |
| Dibutyl sebacate | | | |
| Water | 90 | 90 | 90 |
| **Nano-sizing** | 1 min @80°C | | |
| **D_{N}50 [nm]** | 79.5 | 101 | 173 |
| **PDI** | 0.378 | 0.256 | 0.129 |
| **pH** | 4 | 4 | 4 |
| **Zeta potential [mV]** | +81.7 | +106.0 | +80.4 |

### Example 7: Preparation of core-shell nano-particles containing acid in the core

In the following example, the nano-dispersion was pH-modified from within the core of the core-shell, as opposed to forming the nano-emulsion by combining the core-shells with acidified polar carrier, as previously done (*e.g.,* in example 6).

2 g of PCL-14, 2 g of Genamin^{®} O 020 Special and 6 g of Cetiol^{®} B were placed in a 20 ml glass vial. 0.01 g of anhydrous acetic acid were added, and the vial contents were sonicated for 2 minutes at about 70°C until a clear CSSC/fatty amine/acid premix was obtained.

In a separate 20 ml glass vial, 9 g of water were placed, 1 g of the hot CSSC/fatty amine/acid premix prepared above was added, and the contents of the vial were sonicated for 1 minute at about 80°C to obtain a nano-emulsion. The nano-emulsion was allowed to cool down to room temperature until a nano-dispersion was obtained.

The pH was measured and found to be 5. The zeta potential, was measured in samples of the nano-dispersion, diluted to 1:100 in double distilled water and was found to be +54.1 mV, indicating the positive charging of the nano-particles, supposedly due to the acetic acid leaching out of the core of the nano-particles into the polar carrier, thus protonating the amine heads in the shell of the nano-particles. D_{N}50 and PDI were similarly measured, and were found to be 35.3 nm and 0.198, respectively.

### Example 8: Preparation of core-shell nano-particles containing CSSC-miscible active agents within the cores

2 g of PCL-14 and 0.1 g of benzoyl peroxide as a CSSC-miscible active agent were placed in a 20 ml glass vial. 2 g of Genamin^{®} O 020 Special and 5.9 g of Cetiol^{®} B were added, and the vial contents were sonicated for 2 minutes at about 70°C until a clear CSSC/fatty amine/active agent premix was obtained.

In a separate 20 ml glass vial, 9 g of water were placed and acidified to a pH of 4 by adding a drop of acetic acid. 1 g of the hot CSSC/fatty amine/active agent premix prepared above was added to the acidic liquid carrier, and the contents of the vial were sonicated for 1 minute at about 80°C to obtain a nano-emulsion. The nano-emulsion was allowed to cool down to room temperature until a nano-dispersion was obtained.

The nano-dispersion so obtained (*ND29*) is reported in Table 5. Additional nano-dispersions were prepared according to similar procedures, all being chargeable in the presence of water, each nano-dispersion containing a different CSSC-miscible and water-insoluble active agent (either a pharmaceutical agent or an agricultural agent), as specified in the table.

The zeta potential, D_{N}50 and PDI were measured in samples of some of the nano-dispersions, diluted to 1:100 in double distilled water, resulting in their charging, and the values are presented in Table 5.

**Table 5**

| **Component** | **Composition** | | | | | | | |
|---|---|---|---|---|---|---|---|---|
| | ***ND29*** | ***ND30*** | ***ND31*** | ***ND32*** | ***ND33*** | ***ND34*** | ***ND35*** | ***ND36*** |
| **CSSC/fatty amine/active agent premix** | | | | | | | | |
| PCL-14 | 20 | 20 | 20 | 20 | 20 | 20 | 20 | 20 |
| Benzoyl peroxide | 1 | | | | | | | |
| Tadalafil | | 1 | | | | | | |
| Retinyl palmitate | | | 1 | | | | | |
| Bakuchiol | | | | 1 | | | | |
| Curcumin | | | | | 1 | | | |
| Neem oil | | | | | | 1 | 60 | |
| Castor oil | | | | | | | | 60 |
| Genamin^{®} O 020 Special | 20 | 20 | 20 | 20 | 20 | 20 | 20 | 20 |
| Cetiol^{®} B | 59 | 59 | 59 | 59 | 59 | 59 | | |

| **Nano-dispersion** | | | | | | | | |
|---|---|---|---|---|---|---|---|---|
| PCL-14 | 2 | 2 | 2 | 2 | 2 | 2 | 4 | 4 |
| Benzoyl peroxide | 0.1 | | | | | | | |
| Tadalafil | | 0.1 | | | | | | |
| Retinyl palmitate | | | 0.1 | | | | | |
| Bakuchiol | | | | 0.1 | | | | |
| Curcumin | | | | | 0.1 | | | |
| Neem oil | | | | | | 0.1 | 12 | |
| Castor oil | | | | | | | | 12 |
| Genamin^{®} O 020 Special | 2 | 2 | 2 | 2 | 2 | 2 | 4 | 4 |
| Cetiol^{®} B | 5.9 | 5.9 | 5.9 | 5.9 | 5.9 | 5.9 | | |
| Water | 90 | 90 | 90 | 90 | 90 | 90 | 80 | 80 |
| **D_{N}50 [nm]** | 74.1 | 77.8 | 39.2 | 35.0 | 74.7 | 69.5 | 86.5 | 107.0 |
| **PDI** | 0.157 | 0.163 | 0.171 | 0.138 | 0.163 | 0.160 | 0.178 | 0.287 |
| **Zeta potential [mV]** | +48.8 | +48.0 | +49.6 | +48.7 | +46.4 | +55.4 | +96.2 | +69.9 |

As can be seen, *ND35* and *ND36* do not contain a dedicated non-volatile liquid. It is believed that the CSSC-miscible active agents neem oil and castor oil, respectively included in *ND35* and *ND36* in relatively high amounts of three-fold the CSSC weight, additionally served to plasticize the pre-mix including the CSSC.

### Example 9: Preparation of core-shell nano-particles coated with water-soluble active agents

1 g collagen peptides powder and 2.1 g water were placed in a 20 ml glass vial and mixed at room temperature until complete dissolution.

In a separate 20 ml vial, 6.9 g of the core-shell nano-dispersion *ND11* obtained in Example 6 were placed, and the collagen solution providing the molecules of water-soluble active agents due for form the second shells was added. The contents of the vial were shaken by hand for 10 seconds until a homogeneous mixture was obtained. The composition of collagen-coated nano-elements so obtained (*Collagen-ND11*) is reported in Table 6, which also presents a composition of LMW HA-coated nano-elements (*LMW HA-ND11*)*,* similarly prepared. A reference composition containing uncoated nano-elements (*Uncoated-cND11*) was also prepared, comprising 3.1 g water lacking any carrier-soluble active agent.

Three additional nano-dispersions were prepared, based on *ND6* and *ND14* (referred to as *LMW HA-ND6* and *LMW HA-ND14,* respectively), to which LMW HA was added to achieve the concentrations reported in Table 6. Samples of *LMW HA-ND6* and of *LMW HA-ND14* were prepared, wherein the LMW HA solution was mixed with the *ND6* or *ND14* compositions by sonication. For comparison, another sample of *LMW HA-ND6* with same concentrations of constituents was prepared by mixing the LMW HA solution with the *ND6* composition by hand.

These compositions are also reported in Table 6, wherein the different components and amounts are specified. The values reported in the table correspond to the concentration of each component in wt.% by total weight of the nano-dispersion. D_{N}50 and zeta potential values of most compositions are also presented in Table 6.

**Table 6**

| **Component** | **Composition** | | | | |
|---|---|---|---|---|---|
| | ***Uncoated-ND11*** | ***Collagen-ND11*** | ***LMW HA-ND11*** | ***LMW HA-ND6*** | ***LMW HA-ND14*** |
| PCL-14 | 6 | 6 | 6 | 8.65 | 4 |
| Oleyl amine | 5 | 5 | 5 | 8.65 | |
| Genamin^{®} O 020 Special | | | | | 15.8 |
| Citrofol^{®} AII | 9 | 9 | 9 | 25.9 | |
| Glycerol | 14 | 14 | 14 | | |
| Propylene glycol | 10 | 10 | 10 | 9.8 | |
| HCl | 25 | 25 | 25 | | |
| Collagen | | 10 | | | |
| LMW HA | | | 1 | 2.9 | 1 |
| Water | 31 | 21 | 30 | 44.1 | 79.2 |
| **D_{N}50 [nm]** | 50.1 | 43.4 | 40.4 | N/A | 93.3 |
| **Zeta potential [mV]** | +58.6 | +43.6 | +54.2 | N/A | +34.9 |

As can be seen from the changes in zeta potential, the addition of active agents to previously prepared core-shell nano-particles decreased the charges perceived at the newly formed outer surface of the nano-particles, in support of modifications at this interface with the liquid carrier, hence indicating the formation of a 2^{nd} shell composed of the active agent. For instance, while nano-dispersion of uncoated nano-elements *ND14* had a zeta potential of +56.0 mV (according to Table 4A of Example 6), it can be seen that coating these nano-elements with LMW HA decreased their zeta potential to +34.9 mV.

For reference, the zeta potential of 0.1 g of the collagen peptides dispersed in 9.9 g of distilled water was found to be -7.7 mV, indicating a satisfactory Δζ between the zeta potential of the collagen intended to coat the nano-particles and of the nano-particles to be coated thereby, which allows the attachment of the collagen to the surface of the nano-particles previously including only a first shell. The zeta potential of a similarly prepared solution of LMW HA in water was measured and found to be -17 mV, resulting in an even higher Δζ compared to the one observed for the preparation of the composition of collagen-coated nano-particles.

The speed at which the carrier-soluble active agent forming the second shell is mixed with the "uncoated" core-shell having only a first shell of positively chargeable amines is believed to contribute to the form of the core-multi-shells nano-particles that can be obtained.

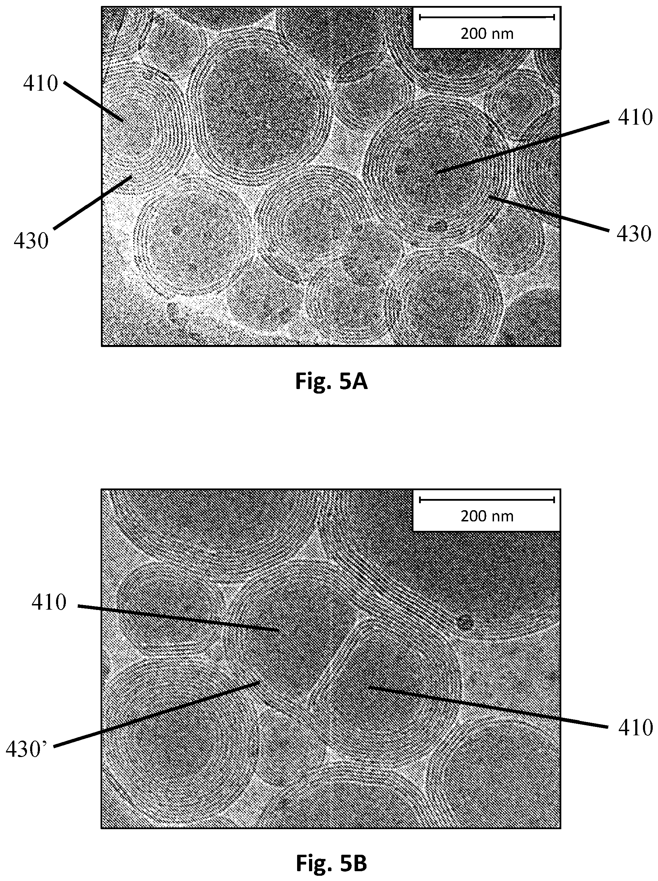

**Figure 5A** shows a CryoTEM analysis of the first sample of composition *LMW HA-ND6,* obtained by high shear mixing (*i.e.,* sonication) of the LMW HA with the core-shell nano-elements of *ND6.* In the figure, the cores **410** comprising the PCL, seen as dark globules, bear a second shell **430**, composed of LMW HA. As can be seen in **Figure 5A****,** the second shell can comprise a number of layers of the HA, formed one on top of the other.

**Figure 5B** is another image captured by CryoTEM, of the second sample *LMW HA-ND6* composition, wherein the LMW HA was mixed in manually with the core-shell nano-elements of *ND6.* In the image, two dark PCL cores **410** can be seen surrounded by a mutual shell **430',** also composed of layers of the LMW HA, forming a larger nano-element. It is believed that manual mixing might be too slow / low energy, and the shells of the active agent formed on the cores coalesce and merge into such larger particles. Hence, it is suggested that high energy mixing (*e.g*., high shear) is more favorable than low energy mixing methods for the formation of second shells surrounding individual cores, such core-multi-shells nano-elements therefore having a particle size distribution commensurate with the size of the individual core-shell, more easily remaining within the ranges of size suitable for transdermal delivery.

Similarly, the *ND12* nano-particles, prepared in Example 6, were coated with various water-soluble active agents, and an uncoated *ND12* composition was also prepared and used as reference. The *ND12*-based compositions, including their respective ingredients and concentrations thereof, are summarized in Table 7, the concentration of each of the components being provided in wt.% by total weight of the nano-dispersion. D_{N}50 and zeta potential values of each composition are also presented in Table 7.

**Table 7**

| | **Composition** | | | | |
|---|---|---|---|---|---|
| | ***Uncoated-ND12*** | ***LMW HA-ND12*** | ***Vitamin C-ND12*** | ***Collagen-ND12*** | ***Elastin-ND12*** |
| PCL-14 | 6 | 6 | 6 | 6 | 6 |
| Oleyl amine | 6 | 6 | 6 | 6 | 6 |
| Citrofol^{®} AII | 18 | 18 | 18 | 18 | 18 |
| Glycerol | 14 | 14 | 14 | 14 | 14 |
| Propylene glycol | 10 | 10 | 10 | 10 | 10 |
| HCl | 25 | 25 | 25 | 25 | 25 |
| LMW HA | | 0.5 | | | |
| Vitamin C | | | 0.5 | | |
| Collagen | | | | 1 | |
| Elastin | | | | | 1 |
| Water | 21 | 20.5 | 20.5 | 20 | 20 |
| **D_{N}50 [nm]** | 72.2 | 67.5 | 66.1 | 72.8 | 61.6 |
| **Zeta potential [mV]** | + 66.6 | + 50.8 | + 44.5 | + 33.5 | + 39.9 |

### Example 10: Conductivity measurements

In order to further demonstrate the adsorption of the water-soluble active agent to the surface of the core-shell nano-particles, conductivities of samples of the various *ND12*-based compositions were measured using a conductivity meter and are presented in microSiemens (µS) in Table 8.

The rationale for this study relies on the expectation that if two species are mixed, each independently having a conductivity as an electrolyte in a particular medium, the conductivity of the mixture is the sum of the relative contributions of the species, only if the species remain separate. In other words, if the conductivity of a mixture of species is not the sum of the individual conductivities of its constituents, it can be assumed that the species interact one with the other.

In the present case, it is assumed that the molecules of the water-soluble active agent are able to attach to the core-shell nano-particles, so that when core-shell nano-particles having a conductivity **A** at a given concentration in a medium are mixed with an active agent having a conductivity **B** at a given concentration in substantially the same medium, the conductivity of the mixture should be lower than the combined conductivities ***A**+**B**.*

Samples of the various water-soluble active agents were prepared in a carrier being a mixture of liquids substantially similar to the one in which the nano-particles of *ND12* and their coated versions were prepared. The conductivities of the active agents alone, of the core-shell nano-particles prior to coating by the second shell of active agents, and of the active agent-coated nano-particles were measured and are all reported in Table 8.

**Table 8**

| | **Conductivity [µS]** | | |
|---|---|---|---|
| **Sample contents** | **Active agent in carrier only** | **Uncoated nano-particles** | **Active agent-coated nano-particles** |
| ***LMW HA-ND12*** | 401 | 735 | 651 |
| ***Vitamin C-ND12*** | 511 | 754 | 835 |
| ***Collagen-ND12*** | 104 | 720 | 521 |
| ***Elastin-ND12*** | 125 | 731 | 700 |

As can be seen from the above table, the conductivities of the uncoated nano-particles only containing the first shell formed by the hydrophilic portion of the fatty amines are substantially similar, as expected for similar samples of core-shell nano-particles in a similar carrier, without any specific externally applied active agents.

In contrast, the conductivities of each active agent alone, or of the core-shell nano-particles coated thereby, depended on the active agent (*e.g.,* LMW HA, vitamin C, collagen and elastin) being considered. Noticeably, the conductivities of all the samples of the active agent-coated nano-particles exhibited conductivities that were lower than the sum of: the conductivity (*A*) of the core-shell nano-particles in the carrier and the conductivity (***B***) of the active agent in substantially the same carrier. These results support that the present method enables the preparation of core-shell nano-particles having a core made of a CSSC material, a first shell made of a fatty amine and a second shell, interacting with the first shell, the second shell being made of a water-soluble active agent.

### Example 11: Patch test protocol for skin irritation analysis

The irritating effect, if any, of topical compositions according to the present teachings, such as prepared in Examples 2-9, can be tested on skin of human volunteers by application of the formulations to be tested via a patch.

Each volunteer applies a predetermined volume of a tested composition (*e.g*., 0.02 ml) in a small plastic cavity (*e.g.,* of 0.64 cm²) of an occlusive patch with a filter tissue coming in contact with the skin of the volunteer in a predetermined body area (*e.g.,* on the back). The patch is attached to the skin area by a hypoallergenic non-woven adhesive tape and the test formulation is kept in contact with the skin for 48 hours.

The appearance of the treated area is assessed before the application of the topical compositions and 30 minutes after patch removal. Empty patches, lacking any composition, can serve as negative controls.

Skin reactions (erythema, dryness and oedema) are scored throughout the test according to the following pre-defined scoring scale:

### Erythema

0 = no evidence of erythema; 0.5 = minimal or doubtful erythema; 1 = slight redness, spotty and diffuse; 2 = moderate, uniform redness; 3 = strong uniform redness; 4 = fiery redness

### Dryness (Scaling)

0 = no evidence of scaling; 0.5 = dry without scaling; appears smooth and taut; 1 = fine/mild scaling; 2 = moderate scaling; 3 = severe scaling with large flakes

### Oedema

- = absence of oedema; + = presence of oedema

The results obtained with any test composition are compared to those obtained on the control zone (naive skin surface under the empty patch) and the compositions classified as: non-irritant, very slightly irritant, slightly irritant, moderately irritant, irritant, or very irritant, according to the combined effect a composition has with respect to the aforesaid prospective skin reactions.

### Example 12: Penetration of composition into biological tissue

The distribution of nano-elements of the present compositions in various skin layers was tested *ex vivo* by applying core-shell and core-multi-shells (including a second shell forming active agent) nano-elements onto the skin of pigs' ears, the cores of the tested compositions including a fluorescent dye (Nile red) allowing histological monitoring.

### Tested compositions:

The following test and control compositions were prepared and used as set in Table 9.

**Table 9**

| **Tested composition** | **No. of repeats** |
|---|---|
| (1) Saline, with no fluorescent marker | 2 |
| (2) *ND14,* modified to contain Nile red in PCL core | 6 |
| (3) *LMW HA-ND14,* modified to contain Nile red in PCL core | 6 |

Tested compositions (2) and (3) were prepared by adding 2 mg of Nile red fluorescent marker during the preparation of the CSSC/fatty amine premix, as described in Example 6. All tested compositions were stored in closed containers at room temperature until they were used.

### Testing procedure:

The compositions were tested on three freshly harvested pig ears, collected on the same day, and kept fresh and cooled till use. The ears were washed with excess saline solution and wiped dry. They were then immobilized for the duration of the study by taping them onto a glass surface, their inner side facing down. Each ear was marked on its outer side with 6 circles having a diameter of about 1 cm.

25 microliters of each one of the samples were applied onto the skin near the center of the marked circle, each tested composition being applied in two or more separate circles for repeats. The ears with the applied test samples were then placed in an oven for an incubation time of 20 hours at a temperature of 32°C, to simulate the pigs' body temperature.

Following this incubation, the ears were punched with a metal punch having a diameter of about 5 mm to extract tissue samples of each circle onto which the different compositions were applied. Each punched-out skin sample was placed in a 50 ml plastic cup and immediately topped with 5 ml of a 4% paraformaldehyde solution, used as a fixation fluid to preserve the tissue sample and reduce leaching out of the dye. The skin samples were kept in the paraformaldehyde solution for at least 48 hours at 4°C for complete fixation.

### Tissue collection, fixation and slide preparation:

The tissue samples were trimmed, each tissue sample was longitudinally cut in half and each half cylinder placed in an embedding cassette. A first half of the samples were processed for paraffin embedding by immersing each sample in various reagents (formaldehyde, then water, then ethanol at various grades and finally xylene) for periods of 2-10 minutes at temperatures ranging from 37-45°C, to remove traces of the paraformaldehyde and any other contaminants. Subsequently, the samples were immersed in paraffin 3 times for 5-10 minutes each time, at a temperature of 65°C. The second half of each sample was processed in an Optimal Cutting Temperature (OCT) block, according to a standard procedure.

Sections of each processed sample were prepared using a frozen section microtome and placed on glass slides, the thickness of the paraffin and OCT sections being of 4 µm and 10 µm, respectively.

### Analysis and results:

The above tissue sections were evaluated by an expert for the presence and intensity of the marker using a fluorescence microscope at objective magnification of x10 and x20.

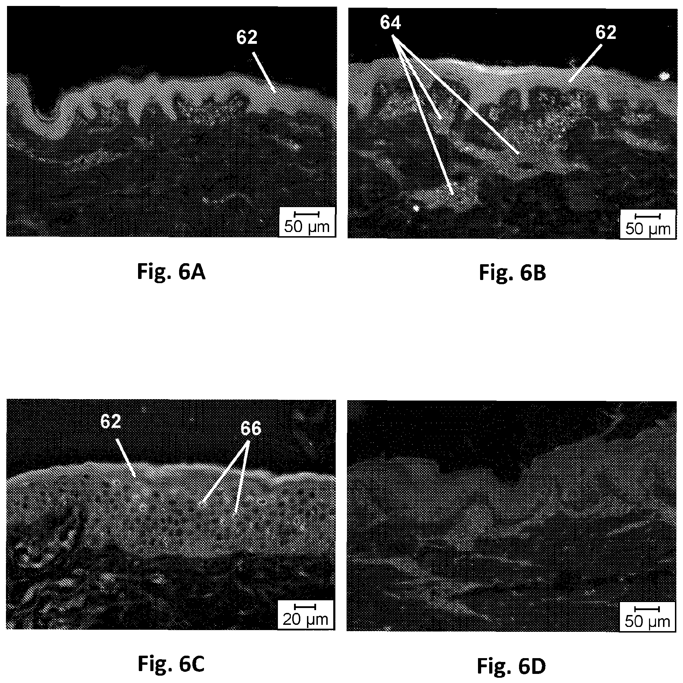

The group of samples treated with fluorescently-marked *Composition ND14,* containing the core-shell nano-elements, showed positive cells, *i.e.,* being labelled with the fluorescent marker, in four out of the six tested tissue samples. This is shown in **Figure 6A****,** presenting a histological picture of a cross-section of one of the tissue samples, processed for paraffin embedment and taken by the microscope camera at a magnification of x10. In Figure 6A, the penetration of the composition into the epidermis **62** is demonstrated as a bright area, indicating the presence of the fluorescent marker in this skin layer.

The group of samples treated with fluorescently-marked *Composition LMW HA-ND14,* containing the core-multi-shells nano-elements including an external shell of HA, also showed positive cells in four of the six tested samples. An exemplary image is provided in **Figure 6B****,** taken at a magnification of x10 of a sample processed for paraffin embedment, in which fluorescence labeling of the entire epidermis layer **62** is shown, as well as labeling of a part of the superficial dermis **64.**

As can be seen, *Composition LMW HA-ND14* penetrated into a deeper layer of the skin (see Figure 6B) as compared to *Composition ND14* (see Figure 6A). This is to be expected considering that the positive charge of *Composition ND14,* which may delay penetration into a negatively-charged skin, is higher than that of *Composition LMW HA-ND14,* being masked by the HA layer (as indicated by the charges listed, respectively, in Tables 4A and 6).

The image displayed in **Figure 6C** corresponds to a sample treated with fluorescently-marked *Composition ND14* as shown in Figure 6A, the sample however being processed in an OCT block, and the image captured at a magnification of x20. In this cross-section it can be seen that in addition to epidermal **62** penetration, the core-shell nano-elements also display transcellular penetration. The fluorescent marker is visible within the intracellular matrix of keratinocytes **66,** wherein the black rounded areas are the keratinocytes' nuclei (unpenetrated by the marker).

**Figure 6D****,** showing a sample image of a cross-section from the control group, treated with saline and embedded in paraffin, displays very low fluorescence of the skin layers, believed to be attributed to auto-fluorescence expressed by the skin tissue itself. This phenomenon is expressed in lower fluorescence levels compared to Figures 6A, 6B or 6C, as can be expected.

It is stressed that these findings can be unambiguously attributed to the behavior of the nano-elements of the tested compositions, and do not result from the independent penetration of the fluorescent Nile red marker. This was established in a control experiment in which samples containing: i) the marker in water; or ii) the marker in a solution containing water and the surfactants of *ND14*; were separately applied to marked circles on pig ear skin. The study was performed as described above for the tested compositions. No penetration of the fluorescent marker was observed with any of the control compositions lacking the nano-elements, indicating that the marker on its own, or even surrounded by surfactants, cannot penetrate into the skin. Hence, this *ex vivo* experiment demonstrates, among other things, that a) the present dermatological compositions and the core-shell nano-elements contained therein can act both on the skin upon which they are applied and within underlying skin layers, being therefore suitable for topical and transdermal uses; b) the present dermatological compositions enable the transdermal penetration of CSSC materials having a molecular weight significantly greater than the generally acknowledged size limit of about 0.5 kDa, the CSSC used in the tested *ND14* related compositions being a polycaprolactone polymer having an average MW of about 14 kDa; and c) the present dermatological compositions enable the transdermal delivery of active agents also having a molecular weight expected to preclude dermal penetration, the active agent used in the tested *ND14* related compositions being LMW hyaluronic acid having an average MW of about 3 kDa.

### Example 13: Effect of the compositions on facial skin appearance

The cosmetic effect of topical compositions according to the present teachings can be tested on skin of healthy human volunteers by application of the formulations to be tested to facial skin. The volunteers are free of dermatological problems, irritated skin, blemishes, or such marks on test site(s) that may impair the study. The samples may include:
a) a dermatological composition containing a predetermined concentration of a nano-dispersion of core-shell nano-particles in a polar carrier, which can have any desirable charge and/or viscosity (*e.g.,* which can be prepared according to Examples 2-7);
b) a dermatological composition containing a predetermined concentration of a nano-dispersion of core-shell nano-particles which may further contain in their core a desirable cosmetically active agent (*e.g.,* which can be prepared according to Example 8);
c) a dermatological composition containing a predetermined concentration of a nano-dispersion of core-multi-shells nano-particles which may further contain in a second shell a desirable cosmetically active carrier-soluble agent (*e.g.,* which can be prepared according to Example 9);
d) a control dermatological composition containing a predetermined concentration of a nano-dispersion in a polar carrier of core nano-particles, lacking any external shell, the core of the reference corresponding to the core of the sample being tested (*e.g., Composition 2.2* in Example 2 of co-owned WO 2022/243899 wherein the core is made from PCL-14 and Cetiol^{®} B; and
e) a placebo composition comprising the polar carrier of any of the samples being tested, but no core-shell(s) nano-particles.

The clinical study is conducted in a double-blind manner, at least ten volunteers being randomly assigned to each arm of the study (*i.e.,* half for each one of the compositions containing the core-shell(s) nano-particles, and the second half for each one of the respective placebo compositions), the concentration of the nano-particles being the same in the non-placebo groups. All groups apply 1 ml of their respective compositions twice-daily (morning and evening) by gently rubbing on facial skin.

### Facial skin parameters

### Wrinkle counts:

The effect of the various compositions on facial skin's wrinkles can be determined using Canlfield's VISIA system (by Canfield Scientific, USA), consisting of the VISIA imaging booth and VISIA software, for capturing and storing facial images using standard lighting, cross-polarized flash, and UV flash.

Measurements are taken from the targeted areas before the first application (baseline), after one (T₁), two (T₂) and three (T₃) months of twice-daily applications of the dermatological compositions being tested. The results of the T₁, T₂ and T₃ time-points are compared to the baseline values initially obtained for each volunteer, as well as to the results of the placebo arm at same timepoints.

The software automatically isolates or "masks" specific areas of the face, as captured in the images, and then performs an extensive analysis of these areas to evaluate skin features such as wrinkles. The data provided by the VISIA system is displayed as "Feature Counts", which provides a count of the number of discrete instances of the feature being evaluated (*e.g.,* wrinkles and lines), regardless of the size or intensity of each instance. The values can be presented as the counted wrinkles on both the left and right sides of the face ("all wrinkles"), or as average values of the wrinkles counted on each of the two facial sides ("average wrinkles").

The results explained in Example 6 with respect to *Composition 2.2* and shown in Figures 4 of WO 2022/243899, as well as the pictures provided in Figures 7A and 7B and explained in Example 10, can be provided as a control experiment. In this publication, the average wrinkle count of the dermatological compositions containing core-only nano-particles (*i.e.*, composition d) mentioned above) decreased compared to the wrinkle count of the placebo composition (*i.e.,* composition e). Similar results are expected for compositions of the present invention which at least include a similar core, the shells being believed to modify the charge of the nano-elements without affecting the intrinsic activity of the core, only its projected efficacy in view of its charge-dependent delivery.

### Elasticity:

The effect of the compositions of the present invention on skin elasticity can be tested using Dermal Torque Meter^{®} (DTM310) (by Dia-Stron, United Kingdom), whereby a mechanical probe having a diameter adapted to the depth of the skin layers to be analysed thereby exerts a predetermined torque for a predetermined length of time ("torque on") onto the selected area of the surface of the subject's facial skin, followed by a "torque off" period, in which the force is rapidly released and the skin attempts to restore the distortion created by the torsional forces. The angular rotation of the torque disk is measured throughout this process and provided as the ratio of the "torque on" to "torque off" periods.

### Facial skin hydration:

The effect of the compositions of the present invention on skin hydration can be measured to confirm that the changes in wrinkle count and/or elasticity can indeed be attributed to a specific collagen-stimulating activity of the compositions and not to a change in hydration level of the skin surface.

Skin hydration analysis can be performed using Corneometer^{®} CM 825 (by Courage+Khazaka electronic GmbH, Germany), which measures the capacitance of the stratum corneum, using a probe capacitor effecting an electric scatter field penetrating the first layers of the stratum corneum (10-20 µm). The results are reported in arbitrary units.

The capacitance variation due to skin surface hydration is measured before application of the composition (baseline) and compared to the values measured after one month of application, for the tested compositions as well as for the placebo being used as control.

As explained in Example 8 of WO 2022/243899, compositions containing CSSC cores including PCL-14, and which may serve as composition d) in the present study, showed a very slight decrease in hydration level compared to the placebo. It is similarly expected that neither the core-shell nano-particles compositions nor the control composition would significantly affect the skin hydration level. Hydration levels are also not expected to change after continued application of the present compositions, such values reaching saturation relatively rapidly and only fluctuating as a result of climatic conditions. These anticipated results would indicate that the effect of the tested compositions on reducing the number of wrinkles and/or increasing skin elasticity can be specifically attributed to the core-shell nano-elements and do not result from an effect of the liquid phase on skin properties.

### In vivo collagen production within facial skin:

To confirm that the efficacy of the present compositions stems *inter alia* from the core-shell nano-elements, their ability to be transdermally delivered, and their ability to fulfil their biological role of stimulating neo-synthesis, collagen levels in facial skin can be measured before and after application of the compositions.

Measurements can be made using DermaLab Combo (by Cortex Technology, Denmark), a skin analysis device whereby an ultrasound probe capable of high frequency and resolution analysis is used by passing the probe on the targeted area in the face. The output includes an image of varying colors and intensities showing *inter alia* spots of collagen presence, the intensity of the spots corresponding to the collagen being recorded in arbitrary unis.

The assessment is performed following application of the tested compositions by volunteers according to the methodology described above, as compared to a corresponding placebo composition.

As demonstrated in Example 9 of WO 2022/243899, as well as in Figure 5B, the collagen levels of a subject treated with a composition containing CSSC cores including PCL-14, and which may serve as composition d) in the present study, have dramatically increased, compared to the levels prior to this treatment. Similar results are expected for the present compositions which include at least a similar core, in addition to the shell(s) and/or cosmetically active agents they may contain.

The results of the above-mentioned parameters (wrinkle counts, elasticity, facial skin hydration and *in vivo* collagen production within the facial skin) for all volunteers in a same group can be averaged and the results of the different groups at the different time points for each parameter can be compared. A decrease of at least 10%, at least 15%, or at least 20% in each of the above parameters for any group at a given timepoint as compared to the group having received the placebo composition at the same timepoint is deemed satisfactory.

### Example 14: Effect of the compositions on wound healing

The following study was conducted to determine the efficacy of a composition according to the present teachings on wound healing in pigs, following its topical application to wounds on the pigs' skin for 15 days during which the wounds were observed and assessed for healing.

### Study system

The study followed the Guidance for Industry "Chronic Cutaneous Ulcer and Burn Wounds - Developing Products for Treatment" with regards to the pre-clinical model and ISO10993-6 for histology evaluation.

Three young adult healthy male pigs (Danish Landrace X-Large White Crossbred), each initially weighing between 13 and 17 kg were used in this study. They were individually ear marked and housed in stainless steel walled pens, in a controlled environment at a temperature of 17-24°C, with a relative humidity (RH) of 30-70%, a 12:12 hour light : dark cycle and 15 air changes/h in the study room. They were provided a commercial pig diet and had free access to fresh drinking water during acclimation and throughout the entire study duration. The study was initiated following 5 days of acclimatation.

### Wound formation surgery

The acclimated pigs were anesthetized by an isoflurane/oxygen mixture, which was delivered through a facemask, and the animals were kept under anesthesia for the duration of the surgery. Six full skin and fascia incisions of 2cm x 2cm were made, each using a separate sterile set of surgery tools, in 6 different locations on the back of each pig (half on each side). Each incision was given a unique identification number, treated as detailed in the following section and was then covered with a sterile transparent film dressing, 3M^{™} Tegaderm^{™}.

Following the surgery, and the application of the first treatment dose, the pigs were returned to their pens for recovery and observation. For 4 consecutive days, they received 0.5 ml of antibiotic (Marbofloxacin 10%, commercially available as Marbocyl^{®} by Vetoquinol, USA) and 0.5 ml of painkiller (Metamizole, commercially available as Calmagine^{®} by Vetoquinol, USA), administered by intramuscular (IM) route at the animal neck.

### Tested compositions and their application

The application of test compositions was initiated immediately after the surgical formation of the wounds, which was defined as the first dose of the first study day. The experimental groups comprised in the study and the regimen of administration are presented in Table 10. Before use in the present study, all tested compositions were sterilized, G1-G2 by exposure to 25-40 KGy Gamma radiation and G3 by autoclave.

**Table 10**

| **Group No.** | **Tested composition** | **Volume of treatment** | **Regimen** |
|---|---|---|---|
| G1 | *ND25,* as listed in Table 4B | 300-700 µl | Twice a day with 10 hours apart in all study days |
| G2 | Placebo: a composition comprising the polar carrier of *ND25* (*i.e.,* 99.75 wt.% water and 0.25 wt.% acetic acid, pH 5) | | |
| G3 | Un-treated: saline | | |

Before the application of the tested compositions, the pigs were anesthetized with a 3% isoflurane/97% oxygen mixture as described for the initial surgery and the first dose. Whilst connected to the anesthesia, each pig was placed on its belly (prone position), the dressing was removed, and the entire wound area was cleaned with saline. Each one of the three tested compositions was dripped into a separate wound, one on each side of the back of the animal, so as to have two applications of each composition per animal. Each wound was thereafter covered with a new transparent sterile dressing. The same dermal test site was used for the same composition in each application per animal throughout the entire study. The dermal test sites varied between animals, for instance, if tested composition of group G1 was applied onto an incision near the neck of a first pig, its application on a second pig occurred on a farther away incision closer to the pig's tail. The variation in the dermal test sites across different pigs allowed to obtain wound healing results of the same composition at various dermal sites.

### Observations and examinations

General clinical signs were recorded once daily during the acclimation period and during the treatment period. Animal body weight was measured at the beginning of acclimation, on study day 1 (prior to surgery), on study day 7, on study day 10 and at the end of the study (day 15). The animals naturally gained weight during the duration of the study, suggesting that none of the wounds and their treatment affected their appetite.

Once a day, while the animals were under anesthesia and the dressing removed, a representative photo of the wound was taken using a specialized camera and 3D scanner adapted for wound and skin assessment (SilhouetteStar by Aranz Medical, New Zealand). Wound inspection and recording of changes were also performed at that time. The wounds were then treated as planned, covered with a new sterile dressing, and the animals returned to their cages for recovery.

The wounds were evaluated prior to the daily administration of the tested compositions, using the score detailed in Table 11.

**Table 11**

| **Observation** | **Definition/Gradation** |
|---|---|
| Erythema (redness) | 0 = Absent |
| | 1 = Mild |
| | 2 = Moderate |
| | 3 = Severe |
| Edema (swelling) | 0 = Absent |
| | 1 = Mild |
| | 2 = Moderate |
| | 3 = Severe |
| Exudate/Drainage | 0 = Absent |
| | 1 = Serous (clear to yellow-tinged) |
| | 2 = Sanguineous (blood-like) |
| | 3 = Serous-Sanguineous (mixture of clear fluid and blood-like fluid) |
| | 4= Purulent (ex. White-tan, creamy, thick) |
| Exudate/Drainage Amount | 0 = Absent |
| | 1 = Mild |
| | 2 = Moderate |
| | 3 = Heavy |
| Granulation tissue filling of the wound bed | 0 = 0 to 33% |
| | 1 = 34 to 66% |
| | 2 = 67 to 90% |
| | 3 = 91 to 100% |
| Infection (based on above findings) | Yes |
| | No |
| Scab | 0 = 0 to 33% |
| | 1 = 34 to 66% |
| | 2 = 67 to 90% |
| | 3 = 91 to 100% |
| Odor recording | Yes |
| | No |

First, it should be generally noted that no bad odor was perceived, nor infection of the wounds observed, during the entire duration of the study with any of the treatment groups. Such observations were reinforced by a lack of exudate from wounds of all groups. Noticeably, as early as from day 3 wounds treated in the G1 group started to display the progressive formation of a layer of composition steadily accumulating on the open area of the wound. As such layer did not wash away with the periodic saline rinsing, it is assumed that the present compositions can be potently retained on wound surface, upon which they may form a protective coat.

With respect to erythema, mild redness was observed in the area surrounding the wounds from day 3 to about day 8 of the study, with a peak average value of about 1.2 on day 6, both for the G2 and G3 control groups. On the same day, wounds of the G1 group, treated with the present composition, displayed an average erythema score of only 0.17. Mild swelling was observed between day 4 and day 7, but totally disappeared in all treated groups from day 8 and on. Wounds of the G1 group displayed their highest swelling on day 4 and even then, their average edema score was only 0.67. Granulation tissue filling of the wound bed started to be observed from day 8 of the study, while scabs were first observed on day 7. The average scab score displayed on this day of first occurrence by wounds of the G1 group was 0.67, while the G2 and G3 control groups displayed no scabs or an average score of only 0.17, respectively. Finally, the wound area was averaged on each day and for each group, and the measurements made from day 2 onward were normalized to day 1, set to display 100% of wound area or 0% of wound closing. It is to be noted, that while the incisions made in the skin were intended to form wounds of about 4 cm² with a perimeter of about 8 cm, the natural stretching of the skin surrounding the excised area resulted in larger wounds of about 9 cm² initial area with a perimeter of about 12 cm. Wounds of the G1 group steadily closed over time, with an average daily decrease in wound area of about 5% so that at the end of the study the average area of G1 wounds was about 30% of their original size. Thus, wounds treated with a composition according to the present teachings achieved a closing of about 70% over 15 days of study duration for full-thickness excision wounds having an initial size of about 9 cm² and depth between 3 mm and 6 mm, depending on the localization of the wound.

### Termination and histology analysis

At the end of the study, the animals were euthanized by pentobarbitone sodium (>100 mg/kg intraperitoneally), and samples were taken for histological tests The entire wound area of each incision was harvested from each animal and fixed in 4% formalin for histology. The tissues were labeled with the wound identification number and animal number. From the formalin-fixed tissues, a 5mm thick slice from the center of each wound was cut, processed, and embedded in paraffin for Hemotoxylin and Eosin (H&E) staining. Each slide can be scanned using a high-resolution digital scanner to enable detailed analysis of the H&E-stained tissues and comparison between the treatment groups. Histopathological microscopic analysis of the H&E slides can evaluate any of the following parameters (according to ISO10993-6, and to the extent applicable): 1. Extent of wound closure; 2. Extent of epithelial regeneration; 3. Amount of necrotic tissue; 4. Hemorrhage; 5. Fibrin; 6. Severity of inflammation; 8. Amount of granulation and fibrous tissue; 8. Fatty infiltrate; 9. Neovascularization; 10. Presence of bacteria or evidence of bacterial infection; 11. Amount of test item wound matrix remnants; 12. Tissue integration; and 13. Encapsulation.

### Example 15: Use of the compositions for the transdermal delivery of active agents

The following pharmacokinetic study can be conducted to determine the effect of a composition according to the present teachings on the delivery of an active agent to the blood stream of a living animal. The active agent can be CSSC-miscible and disposed within the core of the present nano-shell nano-elements, such as done in compositions of Example 8, or can be disposed within surrounding second shells, if water miscible, such as done in compositions of Example 9. The active agent used in the present study should be unambiguously detectable in blood samples of the study animals (*e.g.,* rodents or larger mammals). Tadalafil, such as found in the core of the nano-elements of *ND30,* can be measured, *e.g.,* by liquid chromatography with tandem mass spectrometry (LC-MS-MS) and can therefore be suitable.

The study can be conducted with one or two branches. In the primary branch of the study, a single effective dose of the composition containing the active agent can be applied onto the shaven backs of the study animal (*e.g.,* mice), under anesthesia. The mice, which can be divided into groups, are bled at pre-defined time points (*e.g.,* 15 min, 30 min, 1 hr, 2 hrs, 4 hrs, 8 hrs, 12 hrs and 24 hrs after application), each group (typically of 3 mice each) participating in no more than 3 samplings. The blood samples can be further manipulated as needed to enable measuring the level of the active agent. Skin sampling at pre-defined time points can also be performed for histological analysis.

In a second and optional branch of the study, a reference composition comprising a same dose of the same active agent, albeit as conventionally prepared (*i.e.,* not in the form of the present core-shell nano-elements) can be administered by any suitable route other than transdermal for comparison with the first branch of the study. For illustration, the reference composition can be administered orally by gavage. The animals can then be bled at the same pre-defined time points following ingestion and the level of the active agent measured in the blood samples.

It is appreciated that certain features of the disclosure, which are, for clarity, described in the context of separate embodiments or a particular aspect, may also be provided in combination in a single embodiment, or may apply to any other aspect or embodiments of said other aspects, unless incompatible therewith. Conversely, various features of the disclosure, which are, for brevity, described in the context of a single embodiment, may also be provided separately or in any suitable sub-combination or as suitable in any other described embodiment of the disclosure. Certain features described in the context of various embodiments are not to be considered essential features of those embodiments, unless the embodiment is inoperative without those elements.

Although the present disclosure has been described with respect to various specific embodiments presented thereof for the sake of illustration only, such specifically disclosed embodiments should not be considered limiting.

In the description and claims of the present disclosure, each of the verbs "comprise", "include" and "have", and conjugates thereof, are used to indicate that the object or objects of the verb are not necessarily a complete listing of features, members, steps, components, elements or parts of the subject or subjects of the verb. Yet, it is contemplated that the compositions of the present teachings also consist essentially of, or consist of, the recited components, and that the methods of the present teachings also consist essentially of, or consist of, the recited process steps.

As used herein, the singular form "a", "an" and "the" include plural references and mean "at least one" or "one or more" unless the context clearly dictates otherwise. At least one of A and B is intended to mean either A or B, and may mean, in some embodiments, A and B. A "material" that may be present in the composition alone or in combination with other materials of the same type can be referred to as "material(s)"; CSSC(s), CSSP(s), fatty amine(s), polar carrier(s), non-volatile liquid(s), surfactant(s), active agent(s) and the like, respectively indicating that at least one CSSC, at least one CSSP, at least one fatty amine, at least one polar carrier, at least one non-volatile liquid, at least one surfactant, at least one active agent, and so on, can be used in the present methods or be included in the composition or satisfy the recited parameter or suitable range thereof.

Unless otherwise stated, the use of the expression "and/or" between the last two members of a list of options for selection indicates that a selection of one or more of the listed options is appropriate and may be made.

Unless otherwise stated, when the outer bounds of a range with respect to a feature of an embodiment of the present technology are noted in the disclosure, it should be understood that in the embodiment, the possible values of the feature may include the noted outer bounds as well as values in between the noted outer bounds.

As used herein, unless otherwise stated, adjectives such as "substantially", "approximately" and "about" that modify a condition or relationship characteristic of a feature or features of an embodiment of the present technology, are to be understood to mean that the condition or characteristic is defined to within tolerances that are acceptable for operation of the embodiment for an application for which it is intended, or within variations expected from the measurement being performed and/or from the measuring instrument being used. When the term "about" and "approximately" precedes a numerical value, it is intended to indicate +/-15%, or +/-10%, or even only +/-5%, and in some instances the precise value. Furthermore, unless otherwise stated, the terms (e.g., numbers) used in this disclosure, even without such adjectives, should be construed as having tolerances which may depart from the precise meaning of the relevant term but would enable the invention or the relevant portion thereof to operate and function as described, and as understood by a person skilled in the art.

While this disclosure has been described in terms of certain embodiments and generally associated methods, alterations and permutations of the embodiments and methods will be apparent to those skilled in the art. The present disclosure is to be understood as not limited by the specific embodiments described herein. Therefore, the terms "as described herein", should be more broadly understood to mean "substantially as described herein" or "essentially as described herein" including, when applicable, as shown in the examples and/or illustrated in the drawings.

Certain marks referenced herein may be common law or registered trademarks of third parties. Use of these marks is by way of example and shall not be construed as descriptive or limit the scope of this disclosure to material associated only with such marks.

## Claims

1. A composition comprising core-shell nano-elements dispersed in a polar carrier, the core-shell nano-elements being composed of:
a) a core comprising at least one water-insoluble collagen synthesis stimulating compound (CSSC) having a molecular weight of 0.6 kDa or more; and
b) a shell surrounding the core, the shell comprising at least one water-insoluble fatty amine, non-covalently bonded to the CSSC;
wherein at least 50% of the total number of the core-shell nano-elements have an average diameter (*e.g.,* D_{N}50) of 200 nanometer (nm) or less; and
wherein the core-shell nano-elements have a viscosity of 10⁷ mPa·s or less, as measured at a temperature of 50°C and a shear rate of 10 sec⁻¹.

2. The composition as claimed in claim 1, wherein the core further comprises at least one of: a non-volatile liquid, a water-insoluble active agent, and a pH modifying agent; each being miscible with the CSSC, the non-volatile liquid being non-soluble in the polar carrier and being optionally selected from a group comprising monofunctional or polyfunctional aliphatic esters, fatty esters, cyclic organic esters, aromatic esters, fatty acids, terpenes, aromatic alcohols, aromatic ethers, aldehydes and combinations thereof.

3. The composition as claimed in claim 1 or claim 2, wherein the CSSC and/or the core-shell nano-elements is/are each independently **characterized by** at least one, at least two, or at least three of the following properties:
i. the CSSC and/or the core-shell nano-elements is/are insoluble in the polar carrier;
ii. the CSSC and/or the core-shell nano-elements is/are biodegradable and/or biocompatible;
iii. the CSSC has at least one of a first melting temperature (***Tm***)*,* a first softening temperature (***Ts***) and a first glass transition temperature (***Tg***) of at most 300°C, at most 250°C, at most 200°C, at most 180°C, at most 150°C, or at most 120°C;
iv. the core-shell nano-elements have at least one of a second melting temperature (***Tm***)*,* a second softening temperature (***Ts***) and a second glass transition temperature (***Tg***) of at most 300°C, at most 250°C, at most 200°C, at most 180°C, at most 150°C, or at most 120°C;
v. the CSSC and/or the core-shell nano-elements has/have each respectively at least one of a first ***Tm*** and a second ***Tm*** of at least 0°C, at least 10°C, at least 20°C, at least 30°C, at least 40°C, at least 50°C, or at least 60°C;
vi. the CSSC and/or the core-shell nano-elements has/have each respectively at least one of a first ***Tm*** and a second ***Tm*** between 0°C and 300°C, between 10°C and 300°C, between 20°C and 300°C, between 20°C and 250°C, between 20°C and 200°C, between 30°C and 180°C, between 40°C and 180°C, or between 50°C and 150°C;
vii. the CSSC and/or the core-shell nano-elements has/have each respectively at least one of a first ***Tg,*** a first ***Ts,*** a second ***Tg*** and a second ***Ts*** of -75°C or more, -50°C or more, -25°C or more, 0°C or more, 25°C or more, or 50°C or more;
viii. the CSSC and/or the core-shell nano-elements has/have each respectively at least one of a first ***Tg,*** a first ***Ts,*** a second ***Tg*** and a second ***Ts*** between -75°C and 300°C, between -50°C and 250°C, between -25°C and 200°C, between 0°C and 180°C, between 20°C and 180°C, or between 30°C and 150°C;
ix. the core-shell nano-elements have a viscosity of 5x10⁶ mPa·s or less, 10⁶ mPa·s or less, 5x10⁵ mPa·s or less, 10⁵ mPa·s or less, 5x10⁴ mPa·s or less, 10⁴ mPa·s or less, 5x10³ mPa·s or less, or 10³ mPa·s or less, as measured at a temperature of 50°C and a shear rate of 10 sec⁻¹;
x. the CSSC has a molecular weight of 0.7 kDa or more, 0.8 kDa or more, 0.9 kDa or more, 1 kDa or more, 2 kDa or more, or 5 kDa or more;
xi. the CSSC has a molecular weight of 500 kDa or less, 300 kDa or less, 200 kDa or less, 100 kDa or less, 80 kDa or less, 50 kDa or less, 25 kDa or less, or 15 kDa or less; and
xii. the CSSC has a molecular weight between 0.6 kDa and 500 kDa, between 0.7 kDa and 300 kDa, between 0.8 kDa and 200 kDa, between 1 kDa and 100 kDa, or between 2 kDa and 80 kDa.

4. The composition as claimed in any one of claim 1 to claim 3, wherein the CSSC is selected from:
(I) a polymer selected from a group of polymer families comprising aliphatic polyesters, polyhydroxy-alkanoates, poly(alkene dicarboxylates), polycarbonates, aliphatic-aromatic copolyesters, polysaccharides, lignins, isomers thereof, copolymers thereof and combinations thereof;
(II) a natural polymerizable CSSC selected from resins, gums and gum-resins; and
(III) a quinone selected from a group including Coenzyme Q10.

5. The composition as claimed in any one of claim 1 to claim 4, wherein the composition fulfills at least one of:
a- the water-insoluble fatty amine being a primary, a secondary, a tertiary or quaternary C₈-C₂₂ straight, branched, or cyclic, saturated or unsaturated, aliphatic or aromatic, alkyl or aryl amine, or a combination thereof; and
b- the polar carrier having a boiling temperature (***Tb**_{c}*) between 45°C and 350°C, between 70°C and 300°C, between 85°C and 250°C, or between 90°C and 250°C, the polar carrier being optionally selected from a group comprising water, glycols, glycerols and combinations thereof.

6. The composition as claimed in any one of claim 1 to claim 5, further comprising at least one of:
i) a surfactant being an emulsifier or a hydrotrope; and
ii) a carrier-soluble active agent forming a second shell on the outer surface of the core-shell nano-elements.

7. The composition as claimed in any one of claim 1 to claim 6, wherein the core-shell nano-elements are **characterized by** at least one of:
I. being chargeable in presence of water, the charge, as measured at room temperature, being selected from:
A) a positive charge of +5 mV or more, +10 mV or more, +20 mV or more, +30 mV or more, or +40 mV or more; and
B) a negative charge between -60 mV and -5 mV, between -50 mV and -5 mV, between -40 mV and -5 mV, between -30 mV and -5 mV, or between -20 mV and -5 mV;
II. having a D_{N}50 of 190 nm or less, 175 nm or less, 150 nm or less, 125 nm or less, 100 nm or less, 90 or less, 80 or less, or 70 or less; and optionally of 5 nm or more, in absence or presence of a second shell made of a carrier-soluble active agent; and
III. containing less than 2 wt.%, less than 1.5 wt.%, or less than 1 wt.% a volatile organic compound (VOC) by weight of the nano-elements.

8. The composition as claimed in any one of claim 2 to claim 7, wherein the active agent is selected from:
a) a cosmetic agent selected from a group comprising: anti-aging, anti-acne, anti-oxidant, bleaching and tanning agents;
b) a pharmaceutical agent selected from a group comprising: ADHD-relieving agents, analgesics, anesthetics, anthelmintics, anti-addiction withdrawal agents, anti-anginal agents, anti-arthritic agents, anti-bacterial agents, anti-depressants, anti-emetics, antifungal agents, anti-inflammatory agents, anti-parasitic agents, anti-rheumatic agents, anti-seizure agents, anti-virals, blood pressure regulating agents, contraceptives, hair growth promoting or inhibiting agents, glaucoma relieving agents, hormones, thyroid regulating agents, neuroprotective agents and agents protecting from nerve agents poisoning; and
c) an agrochemical and/or industrial agent selected from a group comprising: acaricides, algicides, animal repellents, anthelmintics, anti-moth agents, avicides, bactericides, chemo-sterilants, fertilizers, fungicides, herbicides, insect pheromones, insect repellents, insecticides, molluscicides, nematicides, nitrification inhibitors, ovicides, plant growth activators, rodenticides, termicides and virucides.

9. The composition as claimed in claim 1 to claim 8, for use in pharmaceutically treating a skin of a living subject, wherein the composition is a pharmaceutical product for achieving a pharmaceutic effect, said effect being selected from:
A- improving skin health and/or appearance, the effect including at least one of treating skin lesions, restoring skin integrity, promoting wounds healing, alleviating or treating local skin inflammation, skin irritation, skin infection, skin rashes, skin pain, skin burns, skin swelling and/or skin allergy; and promoting or inhibiting hair growth; and
B- delaying, alleviating, preventing or treating a transdermally treatable disorder, the effect being selected from a group comprising an ADHD-relieving effect, an analgetic effect, an anthelmintic effect, an anti-addiction withdrawal effect, an anti-anginal effect, an anti-arthritic effect, an anti-depressant effect, an anti-emetic effect, an anti-rheumatic effect, an anti-seizure effect, a blood pressure regulating effect, a contraceptive effect, a glaucoma relieving effect, a hormone replacement effect, a neuroprotective effect, a thyroid regulating effect and a protective effect from nerve agents poisoning.

10. A method for preparing a composition comprising core-shell nano-elements dispersed in a polar carrier, the core-shell nano-elements comprising a collagen-synthesis stimulating compound (CSSC) in a core thereof, the method comprising the steps of:
a) providing at least one CSSC, wherein the CSSC, or each CSSC:
i. is water-insoluble;
ii. has a molecular weight of at least 0.6 kDa;
iii. has at least one of a first melting temperature (***Tm***)*,* a first softening temperature (***Ts***), and a first glass transition temperature (***Tg***) of 300°C or less; and
iv. has a first viscosity optionally higher than 10⁷ mPa·s, as measured at 50°C and a shear rate of 10 sec⁻¹;
b) mixing the at least one CSSC with at least one water-insoluble fatty amine miscible with the CSSC(s), the mixing being at a mixing temperature equal to or higher than at least one of the first ***Tm, Ts,*** and ***Tg*** of the CSSC, whereby a homogeneous mixture including the fatty amine(s) and an optionally plasticized CSSC(s) is formed, the mixture having:
A- a second ***Tm, Ts,*** or ***Tg*** optionally lower than the respective first ***Tm, Ts,*** or ***Tg*** of the CSSC by at least 5°C, at least 10°C, at least 15°C, at least 20°C, at least 25°C, at least 30°C, at least 35°C, at least 40°C, at least 45°C, or at least 50°C; and
B- a second viscosity optionally lower than the first viscosity, at least one of the first and the second viscosity being of 10⁷ mPa·s or less, as measured at 50°C and a shear rate of 10 sec⁻¹;
c) combining a polar carrier with the homogenous mixture obtained in step b); and
d) nano-sizing the combination of step c) by applying shear at a shearing temperature equal to or higher than at least one of the first and second ***Tm, Ts,*** and ***Tg,*** so as to obtain a nano-suspension of core-shell nano-elements dispersed in the polar carrier;
wherein at least 50% of the total number of the core-shell nano-elements have an average diameter (e.g., D_{N}50) of 200 nanometer (nm) or less.

11. The method as claimed in claim 10, **characterized by** at least one of:
A) the at least one CSSC is further **characterized by** at least one, at least two, or at least three of the following properties:
i. the CSSC is insoluble in the polar carrier;
ii. the CSSC has at least one of a first ***Tm, Ts,*** or ***Tg*** of at most 250°C, at most 200°C, at most 180°C, at most 150°C, or at most 120°C;
iii. the CSSC has a first ***Tm*** of at least 0°C, at least 10°C, at least 20°C, at least 30°C, at least 40°C, at least 50°C, or at least 60°C;
iv. the CSSC has a first ***Tm*** between 0°C and 300°C, between 10°C and 300°C, between 20°C and 300°C, between 20°C and 250°C, between 20°C and 200°C, between 30°C and 180°C, between 40°C and 180°C, or between 50°C and 150°C;
v. the CSSC has at least one of a first ***Tg*** and a first ***Ts*** of -75°C or more, -50°C or more, -25°C or more, 0°C or more, 25°C or more, or 50°C or more;
vi. the CSSC has at least one of a first ***Tg*** and a first ***Ts*** between -75°C and 300°C, between -50°C and 250°C, between -25°C and 200°C, between 0°C and 180°C, between 20°C and 180°C, or between 30°C and 150°C;
vii. the CSSC has a molecular weight of 0.7 kDa or more, 0.8 kDa or more, 0.9 kDa or more, 1 kDa or more, 2 kDa or more, or 5 kDa or more;
viii. the CSSC has a molecular weight of 500 kDa or less, 300 kDa or less, 200 kDa or less, 100 kDa or less, 80 kDa or less, 50 kDa or less, 25 kDa or less, or 15 kDa or less;
ix. the CSSC has a molecular weight between 0.6 kDa and 500 kDa, between 0.7 kDa and 300 kDa, between 0.8 kDa and 200 kDa, between 1 kDa and 100 kDa or between 5 kDa and 80 kDa;
x. the CSSC has a first viscosity of 10⁷ mPa·s or less, 5x10⁶ mPa·s or less, 10⁶ mPa·s or less, 5x10⁵ mPa·s or less, 10⁵ mPa·s or less, 5x10⁴ mPa·s or less, 10⁴ mPa·s or less, 5x10³ mPa·s or less, or 10³ mPa·s or less, as measured at 50°C and a shear rate of 10 sec⁻¹; and
xi. the CSSC is biodegradable and/or biocompatible; and
B) the water-insoluble fatty amine miscible with the CSSC is a primary, a secondary, a tertiary or quaternary C₈-C₂₂ straight, branched, or cyclic, saturated or unsaturated, aliphatic or aromatic, alkyl or aryl amine, or a combination thereof.

12. The method as claimed in claim 10 or claim 11, wherein a non-volatile liquid miscible with the CSSC is mixed with the CSSC and the fatty amine in step b), and:
i. the non-volatile liquid is added at a concentration within a range of 5 wt.% to 350 wt.%, 10 wt.% to 200 wt.%, 25 wt.% to 150 wt.%, 25 wt.% to 100 wt.%, or 50 wt.% to 125 wt.% by weight of the CSSC; and/or
ii. the homogeneous mixture obtained thereby has a second ***Tm*** within a range of from 0°C to 290°C, 10°C to 250°C, from 20°C to 200°C, from 30°C to 190°C, from 40°C to 180°C, or from 50°C to 170°C; and/or
iii. the homogeneous mixture obtained thereby has at least one of a second ***Tg*** and a second ***Ts*** within a range from -20°C to 290°C, from -10°C to 290°C, from 0°C to 290°C, from 10°C to 250°C, from 20°C to 200°C, from 30°C to 190°C, from 40°C to 180°C, or from 50°C to 170°C; and/or
iv. the homogeneous mixture obtained thereby has a second viscosity of 10⁷ mPa·s or less, 5x10⁶ mPa·s or less, 10⁶ mPa·s or less, 5x10⁵ mPa·s or less, 10⁵ mPa·s or less, 5x10⁴ mPa·s or less, 10⁴ mPa·s or less, 5x10³ mPa·s or less, or 10³ mPa·s or less, as measured at 50°C and a shear rate of 10 sec⁻¹.

13. The method as claimed in any one of claim 10 to claim 12, further comprising combining during step b) at least one material miscible with the CSSC, the material being selected from a group comprising:
i. a polar-carrier-insoluble surfactant;
ii. an intermediate emulsifier;
iii. a polar-carrier-insoluble active agent; and
iv. a pH-modifying agent.

14. The method as claimed in any one of claim 10 to claim 13, further comprising during or after step c) or step d), adding at least one material soluble in the polar-carrier, the material being selected from a group comprising:
i. a polar-carrier-soluble active agent, at least a part of said active agent forming a second shell on the outer surface of the core-shell nano-elements;
ii. a surfactant;
iii. an intermediate emulsifier;
iv. a skin penetration enhancer; and
v. a pH modifying agent.

15. Use of the composition as claimed in claim 1 to claim 8, for applying to a target surface, wherein the use is a cosmetic use, an agrochemical use, or an industrial use, wherein the composition is selected from:
i) a cosmetical product, wherein the surface is a skin of a living subject, and the composition is for achieving a cosmetic effect improving skin appearance, which effect includes at least one of combating collagen, elastin or glycosaminoglycan breakdown, promoting collagen, elastin or glycosaminoglycan neo-synthesis, treating signs of ageing of the skin, combating wrinkles and fine lines, combating wizened skin, combating flaccid skin, combating thinned skin, combating dull, lifeless skin, combatting uneven skin pigmentation, combating lack of elasticity and/or tonicity of the skin, and skin bleaching or tanning; and
ii) an agrochemical and/or an industrial product, wherein the surface is the surface of an inert object, a plant or a pest or the soil or the water in which plants are growing or pests are present, and the composition is for achieving an agrochemical effect, said effect being as one or more of an acaricide, an algicide, an anthelmintic, an anti-moth, an avicide, a bactericide, a chemo-sterilant, a fertilizer, a fungicide, a herbicide, an insecticide including an ovicide, an insect repellent, an insect pheromone, a molluscicide, a nematicide, a nitrification inhibitor, a pesticide, a pest repellent, a plant growth promotor, a rodenticide, a soil fertilizer, a termicide, a virucide, and a plant wound protectant.

## Patentansprüche

1. Zusammensetzung, umfassend Kern-Schale-Nanoelemente, die in einem polaren Träger dispergiert sind, wobei die Kern-Schale-Nanoelemente zusammengesetzt sind aus:
a) einem Kern, umfassend mindestens eine wasserunlösliche, die Kollagensynthese stimulierende Verbindung (CSSC) mit einem Molekulargewicht von 0,6 kDa oder mehr; und
b) einer Schale, welche den Kern umgibt, wobei die Schale mindestens ein wasserunlösliches Fettamin umfasst, das nicht-kovalent an die CSSC gebunden ist;
wobei mindestens 50 % der Gesamtanzahl der Kern-Schale-Nanoelemente einen durchschnittlichen Durchmesser (*z. B.* D_{N}50) von 200 Nanometern (nm) oder weniger aufweisen; und
wobei die Kern-Schale-Nanoelemente eine Viskosität von 10⁷ mPa·s oder weniger aufweisen, gemessen bei einer Temperatur von 50 °C und einer Scherrate von 10 s⁻¹.

2. Zusammensetzung nach Anspruch 1, wobei der Kern des Weiteren mindestens eines von einer nicht-flüchtigen Flüssigkeit, einem wasserunlöslichen Aktivstoff und einem pH-Modifizierungsmittel umfasst; wobei jedes mit der CSSC mischbar ist, die nicht-flüchtige Flüssigkeit in dem polaren Träger unlöslich ist und gegebenenfalls ausgewählt ist aus einer Gruppe, die monofunktionale oder polyfunktionale aliphatische Ester, Fettester, cyclische organische Ester, aromatische Ester, Fettsäuren, Terpene, aromatische Alkohole, aromatische Ether, Aldehyde und Kombinationen davon umfasst.

3. Zusammensetzung nach Anspruch 1 oder Anspruch 2, wobei die CSSC und/oder die Kern-Schale-Nanoelemente jeweils unabhängig durch mindestens eine, mindestens zwei oder mindestens drei der folgenden Eigenschaften gekennzeichnet ist/sind:
i. die CSSC und/oder die Kern-Mantel-Nanoelemente ist/sind in dem polaren Träger unlöslich;
ii. die CSSC und/oder die Kern-Mantel-Nanoelemente ist/sind biologisch abbaubar und/oder biokompatibel;
iii. die CSSC weist mindestens eine von einer ersten Schmelztemperatur **(*Tm*)*,*** einer ersten Erweichungstemperatur (***Ts***) und einer ersten Glasübergangstemperatur (***Tg***) von höchstens 300 °C, höchstens 250 °C, höchstens 200 °C, höchstens 180 °C, höchstens 150 °C oder höchstens 120 °C auf;
iv. die Kern-Schale-Nanoelemente weisen mindestens eine von einer zweiten Schmelztemperatur (***Tm***), einer zweiten Erweichungstemperatur (***Ts***) und einer zweiten Glasübergangstemperatur (***Tg***) von höchstens 300 °C, höchstens 250 °C, höchstens 200 °C, höchstens 180 °C, höchstens 150 °C oder höchstens 120 °C auf;
v. die CSSC und/oder die Kern-Schale-Nanoelemente weist/weisen jeweils mindestens eine von einer ersten ***Tm*** und einer zweiten ***Tm*** von mindestens 0 °C, mindestens 10 °C, mindestens 20 °C, mindestens 30 °C, mindestens 40 °C, mindestens 50 °C oder mindestens 60 °C auf;
vi. die CSSC und/oder die Kern-Schale-Nanoelemente weist/weisen jeweils mindestens eine von einer ersten ***Tm*** und einer zweiten ***Tm*** zwischen 0 °C und 300 °C, zwischen 10 °C und 300 °C, zwischen 20 °C und 300 °C, zwischen 20 °C und 250 °C, zwischen 20 °C und 200 °C, zwischen 30 °C und 180 °C, zwischen 40 °C und 180 °C oder zwischen 50 °C und 150 °C auf;
vii. die CSSC und/oder die Kern-Schale-Nanoelemente weist/weisen jeweils mindestens eine von einer ersten ***Tg**,* einer ersten ***Ts**,* einer zweiten ***Tg*** und einer zweiten ***Ts*** von -75 °C oder mehr, -50 °C oder mehr, -25 °C oder mehr, 0 °C oder mehr, 25 °C oder mehr, oder 50 °C oder mehr auf;
viii. die CSSC und/oder die Kern-Schale-Nanoelemente weist/weisen jeweils mindestens eine von einer ersten ***Tg**,* einer ersten ***Ts**,* einer zweiten ***Tg*** und einer zweiten ***Ts*** zwischen -75 °C und 300 °C, zwischen -50 °C und 250 °C, zwischen -25 °C und 200 °C, zwischen 0 °C und 180 °C, zwischen 20 °C und 180 °C, oder zwischen 30 °C und 150 °C auf;
ix. die Kern-Schale-Nanoelemente weisen eine Viskosität von 5x10⁶ mPa·s oder weniger, 10⁶ mPa·s oder weniger, 5x10⁵ mPa·s oder weniger, 10⁵ mPa·s oder weniger, 5x10⁴ mPa·s oder weniger, 10⁴ mPa·s oder weniger, 5x10³ mPa·s oder weniger, oder 10³ mPa·s oder weniger auf, gemessen bei einer Temperatur von 50 °C und einer Scherrate von 10 s⁻¹;
x. die CSSC weist ein Molekulargewicht von 0,7 kDa oder mehr, 0,8 kDa oder mehr, 0,9 kDa oder mehr, 1 kDa oder mehr, 2 kDa oder mehr, oder 5 kDa oder mehr auf;
xi. die CSSC weist ein Molekulargewicht von 500 kDa oder weniger, 300 kDa oder weniger, 200 kDa oder weniger, 100 kDa oder weniger, 80 kDa oder weniger, 50 kDa oder weniger, 25 kDa oder weniger, oder 15 kDa oder weniger auf; und
xii. die CSSC weist ein Molekulargewicht zwischen 0,6 kDa und 500 kDa, zwischen 0,7 kDa und 300 kDa, zwischen 0,8 kDa und 200 kDa, zwischen 1 kDa und 100 kDa, oder zwischen 2 kDa und 80 kDa auf.

4. Zusammensetzung nach einem von Anspruch 1 bis Anspruch 3, wobei die CSSC ausgewählt ist aus:
(I) einem Polymer ausgewählt aus einer Gruppe von Polymerfamilien, die aliphatische Polyester, Polyhydroxy-alkanoate, Poly(alkendicarboxylate), Polycarbonate, aliphatisch-aromatische Copolyester, Polysaccharide, Lignine, Isomere davon, Copolymere davon und Kombinationen davon umfassen;
(II) einer natürlichen polymerisierbaren CSSC ausgewählt aus Harzen, Gummis und Gummiharzen; und
(III) einem Chinon ausgewählt aus einer Gruppe, die Coenzym Q10 einschließt.

5. Zusammensetzung nach einem von Anspruch 1 bis Anspruch 4, wobei die Zusammensetzung mindestens eines der folgenden erfüllt:
a- das wasserunlösliche Fettamin ist ein primäres, ein sekundäres, ein tertiäres oder quartäres, geradkettiges, verzweigtes oder cyclisches, gesättigtes oder ungesättigtes, aliphatisches oder aromatisches C₈-C₂₂-Alkyl- oder Arylamin oder eine Kombination davon; und
b- der polare Träger weist eine Siedetemperatur (***Tb**_{c}*) zwischen 45 °C und 350 °C, zwischen 70 °C und 300 °C, zwischen 85 °C und 250 °C, oder zwischen 90 °C und 250 °C auf, wobei der polare Träger gegebenenfalls ausgewählt ist aus einer Gruppe, die Wasser, Glykole, Glycerine und Kombinationen davon umfasst.

6. Zusammensetzung nach einem von Anspruch 1 bis Anspruch 5, des Weiteren umfassend mindestens eines der Folgenden:
i) ein Tensid, das ein Emulgator oder ein Hydrotrop ist; und
ii) einen trägerlöslichen Aktivstoff, der eine zweite Schale auf der Außenoberfläche der Kern-Schale-Nanoelemente bildet.

7. Zusammensetzung nach einem von Anspruch 1 bis Anspruch 6, wobei die Kern-Schale-Nanoelemente durch mindestens eines der folgenden gekennzeichnet sind:
I. sie sind in Gegenwart von Wasser ladbar, wobei die Ladung, gemessen bei Raumtemperatur, ausgewählt ist aus:
A) einer positiven Ladung von +5 mV oder mehr, +10 mV oder mehr, +20 mV oder mehr, +30 mV oder mehr, oder +40 mV oder mehr; und
B) einer negativen Ladung zwischen -60 mV und -5 mV, zwischen -50 mV und -5 mV, zwischen -40 mV und -5 mV, zwischen -30 mV und -5 mV, oder zwischen -20 mV und -5 mV;
II. sie weisen einen D_{N}50 von 190 nm oder weniger, 175 nm oder weniger, 150 nm oder weniger, 125 nm oder weniger, 100 nm oder weniger, 90 oder weniger, 80 oder weniger, oder 70 oder weniger und gegebenenfalls 5 nm oder mehr in Abwesenheit oder Anwesenheit eines zweiten Mantels auf, der aus einem trägerlöslichen Aktivstoff ist; und
III. sie enthalten weniger als 2 Gew.%, weniger als 1,5 Gew.% oder weniger als 1 Gew.% einer flüchtigen organischen Verbindung (VOC), bezogen auf das Gewicht der Nanoelemente.

8. Zusammensetzung nach einem von Anspruch 2 bis Anspruch 7, wobei der Aktivstoff ausgewählt ist aus:
a) einem Kosmetikum ausgewählt aus der Gruppe, welche Anti-Aging-, Anti-Akne-, Antioxidans-, Bleich- und Bräunungsmittel umfasst;
b) einem Pharmazeutikum ausgewählt aus einer Gruppe, welches Folgende umfasst: ADHD-Linderungsmittel, Analgetika, Anästhetika, Antihelmintika, Suchtentzugssymptome erleichternde Mittel, Antianginamittel, Antiarthritismittel, antibakterielle Mittel, Antidepressiva, Antiemetika, antifungale Mittel, antientzündliche Mittel, Antiparasitika, Antirheumatika, Antikrampfmittel, antivirale Mittel, Blutdruckregulierungsmittel, Kontrazeptiva, Haarwachstum fördernde oder inhibierende Mittel, Glaukomlinderungsmittel, Hormone, Schilddrüsenregulierungsmittel, Neuroprotektiva und Mittel, die vor Vergiftung durch Nervengifte schützen; und
c) einem agrochemischen und/oder industriellen Mittel ausgewählt aus einer Gruppe, welche umfasst: Akarizide, Algizide, Tierabwehrmittel, Antihelmintika, Antimottenmittel, Avizide, Bakterizide, Chemosterilantien, Düngemittel, Fungizide, Herbizide, Insektenpheromone, Insektenabwehrmittel, Insektizide, Molluskizide, Nematizide, Nitrifizierungsinhibitoren, Ovizide, Pflanzenwachstumsaktivatoren, Rodentizide, Termitizide und Viruzide.

9. Zusammensetzung nach Anspruch 1 bis Anspruch 8 zur Verwendung in der pharmazeutischen Behandlung einer Haut eines lebenden Subjekts, wobei die Zusammensetzung ein pharmazeutisches Produkt zum Erreichen eines pharmazeutischen Effekts ist, wobei der Effekt ausgewählt ist aus:
A- Verbesserung von Hautgesundheit und/oder Erscheinungsbild, wobei der Effekt mindestens eines von Behandlung von Hautläsionen, Wiederherstellung der Integrität der Haut, Förderung von Wundheilung, Linderung oder Behandlung von lokaler Hautentzündung, Hautreizung, Hautinfektion, Hautausschlägen, Hautschmerz, Hautverbrennungen, Hautschwellung und/oder Hautallergie sowie Förderung oder Inhibierung von Haarwachstum einschließt; und
B- Verzögern, Lindern, Vorbeugen oder Behandeln einer transdermal behandelbaren Störung, wobei der Effekt ausgewählt ist aus einer Gruppe, die einen ADHDlindernden Effekt, einen analgetischen Effekt, einen antihelmintischen Effekt, einen Suchtentzugssymptome lindernden Effekt, einen Antianginaeffekt, einen Antiarthritiseffekt, einen antidepressiven Effekt, einen antiemetischen Effekt, einen Antirheumaeffekt, einen Antikrampfeffekt, einen Blutdruckregulierungseffekt, einen kontrazeptiven Effekt, einen Glaukomlinderungseffekt, einen Hormonersatzeffekt, einen neuroprotektiven Effekt, einen Schilddrüsenregulierungseffekt und einen Schutzeffekt vor Vergiftung durch Nervengifte umfasst.

10. Verfahren zur Herstellung einer Zusammensetzung, umfassend Kern-Schale-Nanoelemente, die in einem polaren Träger dispergiert sind, wobei die Kern-Schale-Nanoelemente eine die Kollagensynthese stimulierende Verbindung (CSSC) in einem Kern derselben umfassen, wobei das Verfahren die Schritte umfasst:
a) Bereitstellen von mindestens einer CSSC, wobei die CSSC oder jede CSSC Folgendes aufweist:
i. sie ist wasserunlöslich;
ii. sie hat ein Molekulargewicht von mindestens 0,6 kDa;
iii. sie weist mindestens eine von einer ersten Schmelztemperatur (***Tm***), einer ersten Erweichungstemperatur (***Ts***) und einer ersten Glasübergangstemperatur (***Tg***) von 300 °C oder weniger auf; und
iv. sie weist eine erste Viskosität auf, die gegebenenfalls höher als 10⁷ mPa·s ist, gemessen bei 50 °C und einer Scherrate von 10 s⁻¹;
b) Mischen der mindestens einen CSSC mit mindestens einem wasserunlöslichen Fettamin, das mit der CSSC/den CSSCs mischbar ist, wobei das Mischen bei einer Mischtemperatur gleich oder höher als mindestens einer der ersten ***Tm**, **Ts*** und ***Tg*** der CSSC erfolgt, wodurch eine homogene Mischung gebildet wird, welche das Fettamin/die Fettamine einschließt, und wobei eine oder mehrere gegebenenfalls plastifizierte CSSC(s) gebildet wird/werden, wobei die Mischung aufweist:
A- eine zweite ***Tm**, **Ts*** oder ***Tg**,* die gegebenenfalls um mindestens 5 °C, mindestens 10 °C, mindestens 15 °C, mindestens 20 °C, mindestens 25 °C, mindestens 30 °C, mindestens 35 °C, mindestens 40 °C, mindestens 45 °C oder mindestens 50 °C niedriger als die jeweilige erste ***Tm**, **Ts*** oder ***Tg*** der CSSC ist; und
B- eine zweite Viskosität, die gegebenenfalls niedriger als die erste Viskosität ist, wobei mindestens eine von der ersten und der zweiten Viskosität 10⁷ mPa·s oder weniger ist, gemessen bei 50 °C und einer Scherrate von 10 s⁻¹;
c) Kombinieren eines polaren Trägers mit der in Schritt b) erhaltenen homogenen Mischung; und
d) Bringen der Kombination aus Schritt c) in Nanogröße durch Anwenden von Scherung bei einer Schertemperatur gleich oder höher als mindestens eine von der ersten und der zweiten ***Tm, Ts*** und ***Tg**,* um so eine Nanosuspension von Kern-Schale-Nanoelementen zu erhalten, die in dem polaren Träger dispergiert sind;
wobei mindestens 50 % der Gesamtanzahl der Kern-Schale-Nanoelemente einen durchschnittlichen Durchmesser (*z. B.* D_{N}50) von 200 Nanometern (nm) oder weniger aufweisen.

11. Verfahren nach Anspruch 10, **gekennzeichnet durch** mindestens eines der Folgenden:
A) die mindestens eine CSSC ist weiter **durch** mindestens eine, mindestens zwei oder mindestens drei der folgenden Eigenschaften gekennzeichnet:
i. die CSSC ist in dem polaren Träger unlöslich;
ii. die CSSC weist mindestens eine von einer ersten ***Tm, Ts*** oder ***Tg*** von höchstens 250 °C, höchstens 200 °C, höchstens 180 °C, höchstens 150 °C oder höchstens 120 °C auf;
iii. die CSSC weist eine erste ***Tm*** von mindestens 0 °C, mindestens 10 °C, mindestens 20 °C, mindestens 30 °C, mindestens 40 °C, mindestens 50 °C oder mindestens 60 °C auf;
iv. die CSSC weist eine erste ***Tm*** zwischen 0 °C und 300 °C, zwischen 10 °C und 300 °C, zwischen 20 °C und 300 °C, zwischen 20 °C und 250 °C, zwischen 20 °C und 200 °C, zwischen 30 °C und 180 °C, zwischen 40 °C und 180 °C oder zwischen 50 °C und 150 °C auf;
v. die CSSC weist mindestens eine von einer ersten ***Tg*** und einer ersten ***Ts*** von -75 °C oder mehr, -50 °C oder mehr, -25 °C oder mehr, 0 °C oder mehr, 25 °C oder mehr, oder 50 °C oder mehr auf;
vi. die CSSC weist mindestens eine von einer ersten ***Tg*** und einer ersten ***Ts*** zwischen -75 °C und 300 °C, zwischen -50 °C und 250 °C, zwischen -25 °C und 200 °C, zwischen 0 °C und 180 °C, zwischen 20 °C und 180 °C, oder zwischen 30 °C und 150 °C auf;
vii. die CSSC weist ein Molekulargewicht von 0,7 kDa oder mehr, 0,8 kDa oder mehr, 0,9 kDa oder mehr, 1 kDa oder mehr, 2 kDa oder mehr, oder 5 kDa oder mehr auf;
viii. die CSSC weist ein Molekulargewicht von 500 kDa oder weniger, 300 kDa oder weniger, 200 kDa oder weniger, 100 kDa oder weniger, 80 kDa oder weniger, 50 kDa oder weniger, 25 kDa oder weniger, oder 15 kDa oder weniger auf;
ix. die CSSC weist ein Molekulargewicht zwischen 0,6 kDa und 500 kDa, zwischen 0,7 kDa und 300 kDa, zwischen 0,8 kDa und 200 kDa, zwischen 1 kDa und 100 kDa, oder zwischen 5 kDa und 80 kDa auf;
x. die CSSC weist eine erste Viskosität von 10⁷ mPa·s oder weniger, 5x10⁶ mPa·s oder weniger, 10⁶ mPa·s oder weniger, 5x10⁵ mPa·s oder weniger, 10⁵ mPa·s oder weniger, 5x10⁴ mPa·s oder weniger, 10⁴ mPa·s oder weniger, 5x10³ mPa·s oder weniger oder 10³ mPa·s oder weniger auf, gemessen bei 50 °C und einer Scherrate von 10 s⁻¹, und
xi. die CSSC ist biologisch abbaubar und/oder biokompatibel; und
B) das wasserunlösliche Fettamin, welches mit der CSSC mischbar ist, ist ein primäres, ein sekundäres, ein tertiäres oder quartäres, geradkettiges, verzweigtes oder cyclisches, gesättigtes oder ungesättigtes, aliphatisches oder aromatisches C₈-C₂₂-Alkyl- oder Arylamin oder eine Kombination davon.

12. Verfahren nach Anspruch 10 oder Anspruch 11, wobei eine mit der CSSC mischbare nicht-flüchtige Flüssigkeit mit der CSSC und dem Fettamin in Schritt b) gemischt wird, und:
i. die nicht-flüchtige Flüssigkeit in einer Konzentration innerhalb eines Bereichs von 5 Gew.% bis 350 Gew.%, 10 Gew.% bis 200 Gew.%, 25 Gew.% bis 150 Gew.%, 25 Gew.% bis 100 Gew.% oder 50 Gew.% bis 125 Gew.% zugegeben wird, bezogen auf das Gewicht der CSSC; und/oder
ii. die dadurch erhaltene homogene Mischung eine zweite ***Tm*** innerhalb eines Bereichs von 0 °C bis 290 °C, 10 °C bis 250 °C, von 20 °C bis 200 °C, von 30 °C bis 190 °C, von 40 °C bis 180 °C oder von 50 °C bis 170 °C aufweist; und/oder
iii. die dadurch erhaltene homogene Mischung mindestens eine von einer zweiten ***Tg*** und einer zweiten ***Ts*** innerhalb eines Bereichs von -20 °C bis 290 °C, von -10 °C bis 290 °C, von 0 °C bis 290 °C, von 10 °C bis 250 °C, von 20 °C bis 200 °C, von 30 °C bis 190 °C, von 40 °C bis 180 °C oder von 50 °C bis 170 °C aufweist; und/oder
iv. die dadurch erhaltene homogene Mischung eine zweite Viskosität von 10⁷ mPa·s oder weniger, 5x10⁶ mPa·s oder weniger, 10⁶ mPa·s oder weniger, 5x10⁵ mPa·s oder weniger, 10⁵ mPa·s oder weniger, 5x10⁴ mPa·s oder weniger, 10⁴ mPa·s oder weniger, 5x10³ mPa·s oder weniger oder 10³ mPa·s oder weniger aufweist, gemessen bei 50 °C und einer Scherrate von 10 s⁻¹.

13. Verfahren nach einem von Anspruch 10 bis Anspruch 12, des Weiteren umfassend Kombinieren von mindestens einem mit der CSSC mischbaren Material während Schritt b), wobei das Material ausgewählt ist aus einer Gruppe, welche Folgendes umfasst:
i. ein in polarem Träger unlösliches Tensid;
ii. einen intermediären Emulgator;
iii. einen in polarem Träger unlöslichen Aktivstoff; und
iv. ein pH-Modifizierungsmittel.

14. Verfahren nach einem von Anspruch 10 bis Anspruch 13, des Weiteren umfassend Zugeben von mindestens einem Material, das in dem polaren Träger löslich ist, während oder nach Schritt c) oder Schritt d), wobei das Material ausgewählt ist aus einer Gruppe, welche Folgendes umfasst:
i. einen in polarem Träger löslichen Aktivstoff, wobei mindestens ein Teil des Aktivstoffs eine zweite Schale auf der Außenoberfläche der Kern-Schale-Nanoelemente bildet;
ii. ein Tensid;
iii. einen intermediären Emulgator;
iv. einen Verstärker der Hautpenetration; und
v. ein pH-Modifizierungsmittel.

15. Verwendung der Zusammensetzung nach Anspruch 1 bis Anspruch 8 zur Aufbringung auf eine Zieloberfläche, wobei die Verwendung eine kosmetische Verwendung, eine agrochemische Verwendung oder eine industrielle Verwendung ist, wobei die Zusammensetzung ausgewählt ist aus:
i) einem Kosmetikprodukt, wobei die Oberfläche eine Haut eines lebenden Subjekts ist, und die Zusammensetzung zum Erreichen eines kosmetischen Effekts dient, der das Erscheinungsbild der Haut verbessert, wobei der Effekt mindestens eines von Bekämpfen des Abbaus von Kollagen, Elastin oder Glykosaminoglykan, Fördern der Neosynthese von Kollagen, Elastin oder Glykosaminoglykan, Behandeln von Anzeichen von Hautalterung, Bekämpfen von Falten und feinen Linien, Bekämpfen von runzeliger Haut, Bekämpfen von schlaffer Haut, Bekämpfen von dünner werdender Haut, Bekämpfen von fahler lebloser Haut, Bekämpfen von ungleichmäßiger Hautpigmentierung, Bekämpfen von fehlender Elastizität und/oder Tonizität der Haut sowie Hautbleichen oder -bräunen einschließt; und
ii) einem agrochemischen und/oder industriellen Produkt, wobei die Oberfläche die Oberfläche eines inerten Objekts, eine Pflanze oder ein Ungeziefer oder der Boden oder das Wasser ist, worin Pflanzen wachsen oder Ungeziefer vorhanden ist, und wobei die Zusammensetzung zum Erreichen eines agrochemischen Effekts dient, wobei der Effekt ein oder mehrere von einem Akarizid, einem Algizid, einem Antihelmintikum, einem Antimottenmittel, einem Avizid, einem Bakterizid, einem Chemosterilans, einem Düngemittel, einem Fungizid, einem Herbizid, einem Insektizid einschließlich eines Ovizids, einem Insektenabwehrmittel, einem Insektenpheromon, einem Molluskizid, einem Nematizid, einem Nitrifizierungsinhibitor, einem Pestizid, einem Ungezieferabwehrmittel, einem Pflanzenwachstumspromotor, einem Rodentizid, einem Bodendüngemittel, einem Termitizid, einem Viruzid und einem Pflanzenwundenschutzmittel ist/sind.

## Revendications

1. Composition comprenant des nano-éléments à noyau-enveloppe dispersés dans un véhicule polaire, les nano-éléments à noyau-enveloppe étant composés de :
a) un noyau comprenant au moins un composé stimulant la synthèse de collagène (CSSC) insoluble dans l'eau ayant un poids moléculaire de 0,6 kDa ou plus ; et
b) une enveloppe entourant le noyau, l'enveloppe comprenant au moins une amine grasse insoluble dans l'eau, liée de manière non covalente au CSSC ;
où au moins 50 % du nombre total des nano-éléments à noyau-enveloppe ont un diamètre moyen (p. ex. D_{N}50) de 200 nanomètres (nm) ou moins ; et
où les nano-éléments à noyau-enveloppe ont une viscosité de 10⁷ mPa·s ou moins, telle que mesurée à une température de 50 °C et à un taux de cisaillement de 10 sec⁻¹.

2. Composition selon la revendication 1, où le noyau comprend en outre au moins un élément parmi : un liquide non volatil, un agent actif insoluble dans l'eau et un agent modificateur de pH ; chacun étant miscible avec le CSSC, le liquide non volatil étant non soluble dans le véhicule polaire et étant éventuellement choisi dans un groupe comprenant des esters aliphatiques monofonctionnels ou polyfonctionnels, des esters gras, des esters organiques cycliques, des esters aromatiques, des acides gras, des terpènes, des alcools aromatiques, des éthers aromatiques, des aldéhydes et des combinaisons de ceux-ci.

3. Composition selon la revendication 1 ou la revendication 2, où le CSSC et/ou les nano-éléments à noyau-enveloppe sont chacun caractérisés indépendamment par au moins une, au moins deux ou au moins trois des propriétés suivantes :
i. le CSSC et/ou les nano-éléments à noyau-enveloppe sont insolubles dans le véhicule polaire ;
ii. le CSSC et/ou les nano-éléments à noyau-enveloppe sont biodégradables et/ou biocompatibles ;
iii. le CSSC a au moins une parmi une première température de fusion (***Tm***), une première température de ramollissement (***Ts***) et une première température de transition vitreuse (***Tg***) d'au plus 300 °C, d'au plus 250 °C, d'au plus 200 °C, d'au plus 180 °C, d'au plus 150 °C ou d'au plus 120 °C ;
iv. les nano-éléments à noyau-enveloppe ont au moins une parmi une deuxième température de fusion (***Tm***), une deuxième température de ramollissement (***Ts***) et une deuxième température de transition vitreuse (***Tg***) d'au plus 300 °C, d'au plus 250 °C, d'au plus 200 °C, d'au plus 180 °C, d'au plus 150 °C ou d'au plus 120 °C ;
v. le CSSC et/ou les nano-éléments à noyau-enveloppe ont chacun respectivement au moins une parmi une première ***Tm*** et une deuxième ***Tm*** d'au moins 0 °C, d'au moins 10 °C, d'au moins 20 °C, d'au moins 30 °C, d'au moins 40 °C, d'au moins 50 °C ou d'au moins 60 °C ;
vi. le CSSC et/ou les nano-éléments à noyau-enveloppe ont chacun respectivement au moins une parmi une première ***Tm*** et une deuxième ***Tm*** comprise entre 0 °C et 300 °C, entre 10 °C et 300 °C, entre 20 °C et 300 °C, entre 20 °C et 250 °C, entre 20 °C et 200 °C, entre 30 °C et 180 °C, entre 40 °C et 180 °C, ou entre 50 °C et 150 °C ;
vii. le CSSC et/ou les nano-éléments à noyau-enveloppe ont chacun respectivement au moins une parmi une première ***Tg,*** une première ***Ts,*** une deuxième ***Tg*** et une deuxième ***Ts*** de -75 °C ou plus, -50 °C ou plus, -25 °C ou plus, 0 °C ou plus, 25 °C ou plus, ou 50 °C ou plus ;
viii. le CSSC et/ou les nano-éléments à noyau-enveloppe ont chacun respectivement au moins une parmi une première ***Tg,*** une première ***Ts,*** une deuxième ***Tg*** et une deuxième ***Ts*** comprise entre -75 °C et 300 °C, entre -50 °C et 250 °C, entre -25 °C et 200 °C, entre 0 °C et 180 °C, entre 20 °C et 180 °C, ou entre 30 °C et 150 °C ;
ix. les nano-éléments à noyau-enveloppe ont une viscosité de 5x10⁶ mPa·s ou moins, 10⁶ mPa·s ou moins, 5x10⁵ mPa·s ou moins, 10⁵ mPa·s ou moins, 5x10⁴ mPa·s ou moins, 10⁴ mPa·s ou moins, 5x10³ mPa·s ou moins, ou 10³ mPa·s ou moins, telle que mesurée à une température de 50 °C et à un taux de cisaillement de 10 sec⁻¹ ;
x. le CSSC a un poids moléculaire de 0,7 kDa ou plus, 0,8 kDa ou plus, 0,9 kDa ou plus, 1 kDa ou plus, 2 kDa ou plus, ou 5 kDa ou plus ;
xi. le CSSC a un poids moléculaire de 500 kDa ou moins, 300 kDa ou moins, 200 kDa ou moins, 100 kDa ou moins, 80 kDa ou moins, 50 kDa ou moins, 25 kDa ou moins, ou 15 kDa ou moins ; et
xii. le CSSC a un poids moléculaire compris entre 0,6 kDa et 500 kDa, entre 0,7 kDa et 300 kDa, entre 0,8 kDa et 200 kDa, entre 1 kDa et 100 kDa, ou entre 2 kDa et 80 kDa.

4. Composition selon l'une quelconque de la revendication 1 à la revendication 3, où le CSSC est choisi parmi :
(I) un polymère choisi dans un groupe de familles de polymères comprenant des polyesters aliphatiques, des polyhydroxyalcanoates, des poly(dicarboxylates d'alcènes), des polycarbonates, des copolyesters aliphatiques-aromatiques, des polysaccharides, des lignines, des isomères de ceux-ci, des copolymères de ceux-ci et des combinaisons de ceux-ci ;
(II) un CSSC polymérisable naturel choisi parmi les résines, les gommes et les gommes-résines ; et
(III) une quinone choisie dans un groupe incluant la coenzyme Q10.

5. Composition selon l'une quelconque de la revendication 1 à la revendication 4, où la composition remplit au moins l'une des conditions suivantes :
a- l'amine grasse insoluble dans l'eau est une alkyl- ou arylamine primaire, secondaire, tertiaire ou quaternaire en C₈-C₂₂ linéaire, ramifiée ou cyclique, saturée ou insaturée, aliphatique ou aromatique, ou une combinaison de celles-ci ; et
b- le véhicule polaire a une température d'ébullition **(*Tb_{c}*)** comprise entre 45 °C et 350 °C, entre 70 °C et 300 °C, entre 85 °C et 250 °C, ou entre 90 °C et 250 °C, le véhicule polaire étant éventuellement choisi dans un groupe comprenant l'eau, des glycols, des glycérols et des combinaisons de ceux-ci.

6. Composition selon l'une quelconque de la revendication 1 à la revendication 5, comprenant en outre au moins l'un des éléments suivants :
i) un tensioactif qui est un émulsifiant ou un hydrotrope ; et
ii) un agent actif soluble dans le véhicule formant une deuxième enveloppe sur la surface extérieure des nano-éléments à noyau-enveloppe.

7. Composition selon l'une quelconque de la revendication 1 à la revendication 6, où les nano-éléments à noyau-enveloppe sont **caractérisés par** au moins l'un des éléments suivants :
I. le fait d'être chargeable en présence d'eau, la charge, telle que mesurée à température ambiante, étant choisie parmi :
A) une charge positive de +5 mV ou plus, +10 mV ou plus, +20 mV ou plus, +30 mV ou plus, ou +40 mV ou plus ; et
B) une charge négative comprise entre -60 mV et -5 mV, entre -50 mV et -5 mV, entre -40 mV et -5 mV, entre -30 mV et -5 mV, ou entre -20 mV et -5 mV ;
II. le fait d'avoir un D_{N}50 de 190 nm ou moins, 175 nm ou moins, 150 nm ou moins, 125 nm ou moins, 100 nm ou moins, 90 ou moins, 80 ou moins, ou 70 ou moins ; et éventuellement de 5 nm ou plus, en l'absence ou en la présence d'une deuxième enveloppe composée d'un agent actif soluble dans le véhicule ; et
III. le fait de contenir moins de 2 % en poids, moins de 1,5 % en poids ou moins de 1 % en poids d'un composé organique volatil (COV) par rapport au poids des nano-éléments.

8. Composition selon l'une quelconque de la revendication 2 à la revendication 7, où l'agent actif est choisi parmi :
a) un agent cosmétique choisi dans un groupe comprenant : des agents antivieillissement, anti-acné, antioxydants, blanchissants et bronzants ;
b) un agent pharmaceutique choisi dans un groupe comprenant : des agents soulageant le TDAH, des analgésiques, des anesthésiques, des anthelminthiques, des agents de sevrage anti-addiction, des agents anti-angineux, des agents anti-arthritiques, des agents antibactériens, des antidépresseurs, des agents antiémétiques, des agents antifongiques, des agents anti-inflammatoires, des agents antiparasitaires, des agents antirhumatismaux, des agents antiépileptiques, des agents antiviraux, des agents régulateurs de la pression artérielle, des contraceptifs, des agents favorisant ou inhibant la pousse des cheveux, des agents soulageant le glaucome, des hormones, des agents régulateurs de la thyroïde, des agents neuroprotecteurs et des agents protégeant contre l'empoisonnement par des agents neurotoxiques ; et
c) un agent agrochimique et/ou industriel choisi dans un groupe comprenant : des acaricides, des algicides, des répulsifs pour animaux, des anthelminthiques, des agents antimites, des avicides, des bactéricides, des chimio-stérilisants, des engrais, des fongicides, des herbicides, des phéromones d'insectes, des répulsifs d'insectes, des insecticides, des molluscicides, des nématicides, des inhibiteurs de nitrification, des ovicides, des activateurs de croissance des plantes, des rodenticides, des termicides et des virucides.

9. Composition selon la revendication 1 à la revendication 8, pour utilisation dans le traitement pharmaceutique de la peau d'un sujet vivant, où la composition est un produit pharmaceutique destiné à obtenir un effet pharmaceutique, ledit effet étant choisi parmi :
A- l'amélioration de la santé et/ou de l'apparence de la peau, l'effet incluant au moins un parmi le traitement des lésions cutanées, la restauration de l'intégrité de la peau, la promotion de la cicatrisation des plaies, le soulagement ou le traitement de l'inflammation cutanée locale, de l'irritation cutanée, de l'infection cutanée, des éruptions cutanées, de la douleur cutanée, des brûlures cutanées, du gonflement cutané et/ou de l'allergie cutanée ; et la promotion ou l'inhibition de la pousse des cheveux ; et
B- le retardement, le soulagement, la prévention ou le traitement d'un trouble pouvant être traité par voie transdermique, l'effet étant choisi dans un groupe comprenant un effet de soulagement du TDAH, un effet analgésique, un effet anthelminthique, un effet de sevrage anti-addiction, un effet anti-angineux, un effet anti-arthritique, un effet antidépresseur, un effet antiémétique, un effet antirhumatismal, un effet antiépileptique, un effet régulateur de la pression artérielle, un effet contraceptif, un effet de soulagement du glaucome, un effet de remplacement hormonal, un effet neuroprotecteur, un effet régulateur de la thyroïde et un effet protecteur contre l'empoisonnement par des agents neurotoxiques.

10. Procédé de préparation d'une composition comprenant des nano-éléments à noyau-enveloppe dispersés dans un véhicule polaire, les nano-éléments à noyau-enveloppe comprenant un composé stimulant la synthèse de collagène (CSSC) dans un noyau de ceux-ci, le procédé comprenant les étapes suivantes :
a) la fourniture d'au moins un CSSC, où le CSSC, ou chaque CSSC :
i. est insoluble dans l'eau ;
ii. a un poids moléculaire d'au moins 0,6 kDa ;
iii. a au moins une parmi une première température de fusion **(*Tm*)*,*** une première température de ramollissement (***Ts***) et une première température de transition vitreuse (***Tg***) de 300 °C ou moins ; et
iv. a une première viscosité éventuellement supérieure à 10⁷ mPa·s, telle que mesurée à 50 °C et à un taux de cisaillement de 10 sec⁻¹ ;
b) le mélange de l'au moins un CSSC avec au moins une amine grasse insoluble dans l'eau miscible avec le ou les CSSC, le mélange étant effectué à une température de mélange égale ou supérieure à au moins l'une des premières ***Tm, Ts*** et ***Tg*** du CSSC, de telle sorte qu'un mélange homogène incluant la ou les amines grasses et un ou plusieurs CSSC éventuellement plastifiés est formé, le mélange ayant :
A- une deuxième ***Tm, Ts*** ou ***Tg*** éventuellement inférieure à la première ***Tm, Ts*** ou ***Tg*** respective du CSSC d'au moins 5 °C, d'au moins 10 °C, d'au moins 15 °C, d'au moins 20 °C, d'au moins 25 °C, d'au moins 30 °C, d'au moins 35 °C, d'au moins 40 °C, d'au moins 45 °C ou d'au moins 50 °C ; et
B- une deuxième viscosité éventuellement inférieure à la première viscosité, au moins une parmi la première et la deuxième viscosité étant de 10⁷ mPa·s ou moins, telle que mesurée à 50 °C et à un taux de cisaillement de 10 sec⁻¹ ;
c) la combinaison d'un véhicule polaire avec le mélange homogène obtenu à l'étape b) ; et
d) le nano-dimensionnement de la combinaison de l'étape c) en appliquant un cisaillement à une température de cisaillement égale ou supérieure à au moins une parmi la première et la deuxième ***Tm, Ts*** et ***Tg,*** de manière à obtenir une nano-suspension de nano-éléments à noyau-enveloppe dispersés dans le véhicule polaire ;
où au moins 50 % du nombre total des nano-éléments à noyau-enveloppe ont un diamètre moyen (p. ex. D_{N}50) de 200 nanomètres (nm) ou moins.

11. Procédé selon la revendication 10, **caractérisé par** au moins l'un des éléments suivants :
A) l'au moins un CSSC est en outre **caractérisé par** au moins une, au moins deux ou au moins trois des propriétés suivantes :
i. le CSSC est insoluble dans le véhicule polaire ;
ii. le CSSC a au moins une parmi une première ***Tm, Ts*** ou ***Tg*** d'au plus 250 °C, d'au plus 200 °C, d'au plus 180 °C, d'au plus 150 °C ou d'au plus 120 °C ;
iii. le CSSC a une première ***Tm*** d'au moins 0 °C, d'au moins 10 °C, d'au moins 20 °C, d'au moins 30 °C, d'au moins 40 °C, d'au moins 50 °C ou d'au moins 60 °C ;
iv. le CSSC a une première ***Tm*** comprise entre 0 °C et 300 °C, entre 10 °C et 300 °C, entre 20 °C et 300 °C, entre 20 °C et 250 °C, entre 20 °C et 200 °C, entre 30 °C et 180 °C, entre 40 °C et 180 °C, ou entre 50 °C et 150 °C ;
v. le CSSC a au moins une parmi une première ***Tg*** et une première ***Ts*** de - 75 °C ou plus, -50 °C ou plus, -25 °C ou plus, 0 °C ou plus, 25 °C ou plus, ou 50 °C ou plus ;
vi. le CSSC a au moins une parmi une première ***Tg*** et une première ***Ts*** comprise entre -75 °C et 300 °C, entre -50 °C et 250 °C, entre -25 °C et 200 °C, entre 0 °C et 180 °C, entre 20 °C et 180 °C, ou entre 30 °C et 150 °C ;
vii. le CSSC a un poids moléculaire de 0,7 kDa ou plus, 0,8 kDa ou plus, 0,9 kDa ou plus, 1 kDa ou plus, 2 kDa ou plus, ou 5 kDa ou plus ;
viii. le CSSC a un poids moléculaire de 500 kDa ou moins, 300 kDa ou moins, 200 kDa ou moins, 100 kDa ou moins, 80 kDa ou moins, 50 kDa ou moins, 25 kDa ou moins ou 15 kDa ou moins ;
ix. le CSSC a un poids moléculaire compris entre 0,6 kDa et 500 kDa, entre 0,7 kDa et 300 kDa, entre 0,8 kDa et 200 kDa, entre 1 kDa et 100 kDa ou entre 5 kDa et 80 kDa ;
x. le CSSC a une première viscosité de 10⁷ mPa·s ou moins, 5x10⁶ mPa·s ou moins, 10⁶ mPa·s ou moins, 5x10⁵ mPa·s ou moins, 10⁵ mPa·s ou moins, 5x10⁴ mPa·s ou moins, 10⁴ mPa·s ou moins, 5x10³ mPa·s ou moins, ou 10³ mPa·s ou moins, telle que mesurée à 50 °C et à un taux de cisaillement de 10 sec⁻¹ ; et
xi. le CSSC est biodégradable et/ou biocompatible ; et
B) l'amine grasse insoluble dans l'eau miscible avec le CSSC est une alkyl- ou arylamine primaire, secondaire, tertiaire ou quaternaire en C₈-C₂₂ linéaire, ramifiée ou cyclique, saturée ou insaturée, aliphatique ou aromatique, ou une combinaison de celles-ci.

12. Procédé selon la revendication 10 ou la revendication 11, où un liquide non volatil miscible avec le CSSC est mélangé avec le CSSC et l'amine grasse à l'étape b), et :
i. le liquide non volatil est ajouté à une concentration comprise dans une plage de 5 % à 350 % en poids, de 10 % à 200 % en poids, de 25 % à 150 % en poids, de 25 % à 100 % en poids ou de 50 % à 125 % en poids du CSSC ; et/ou
ii. le mélange homogène ainsi obtenu a une deuxième ***Tm*** comprise dans une plage de 0 °C à 290 °C, de 10 °C à 250 °C, de 20 °C à 200 °C, de 30 °C à 190 °C, de 40 °C à 180 °C ou de 50 °C à 170 °C ; et/ou
iii. le mélange homogène ainsi obtenu a au moins une parmi une deuxième ***Tg*** et une deuxième ***Ts*** comprise dans une plage de -20 °C à 290 °C, de -10 °C à 290 °C, de 0 °C à 290 °C, de 10 °C à 250 °C, de 20 °C à 200 °C, de 30 °C à 190 °C, de 40 °C à 180 °C ou de 50 °C à 170 °C ; et/ou
iv. le mélange homogène ainsi obtenu a une deuxième viscosité de 10⁷ mPa·s ou moins, 5x10⁶ mPa·s ou moins, 10⁶ mPa·s ou moins, 5x10⁵ mPa·s ou moins, 10⁵ mPa·s ou moins, 5x10⁴ mPa·s ou moins, 10⁴ mPa·s ou moins, 5x10³ mPa·s ou moins, ou 10³ mPa·s ou moins, mesurée à 50 °C et à un taux de cisaillement de 10 sec⁻¹.

13. Procédé selon l'une quelconque de la revendication 10 à la revendication 12, comprenant en outre, pendant l'étape b), la combinaison d'au moins un matériau miscible avec le CSSC, le matériau étant choisi dans un groupe comprenant :
i. un tensioactif insoluble dans un véhicule polaire ;
ii. un émulsifiant intermédiaire ;
iii. un agent actif insoluble dans un véhicule polaire ; et
iv. un agent modificateur de pH.

14. Procédé selon l'une quelconque de la revendication 10 à la revendication 13, comprenant en outre, pendant ou après l'étape c) ou l'étape d), l'ajout d'au moins un matériau soluble dans le véhicule polaire, le matériau étant choisi dans un groupe comprenant :
i. un agent actif soluble dans le véhicule polaire, au moins une partie dudit agent actif formant une deuxième enveloppe sur la surface extérieure des nano-éléments à noyau-enveloppe ;
ii. un tensioactif ;
iii. un émulsifiant intermédiaire ;
iv. un agent favorisant la pénétration cutanée ; et
v. un agent modificateur de pH.

15. Utilisation de la composition selon la revendication 1 à la revendication 8, pour application sur une surface cible, où l'utilisation est une utilisation cosmétique, une utilisation agrochimique ou une utilisation industrielle, où la composition est choisie parmi :
i) un produit cosmétique, où la surface est la peau d'un sujet vivant, et la composition est destinée à obtenir un effet cosmétique améliorant l'apparence de la peau, lequel effet inclut au moins un parmi la lutte contre la dégradation du collagène, de l'élastine ou du glycosaminoglycane, la promotion de la néosynthèse du collagène, de l'élastine ou du glycosaminoglycane, le traitement des signes de vieillissement de la peau, la lutte contre les rides et les ridules, la lutte contre la peau flétrie, la lutte contre la peau flaccide, la lutte contre l'amincissement de la peau, la lutte contre le teint terne et sans vie, la lutte contre la pigmentation irrégulière de la peau, la lutte contre le manque d'élasticité et/ou de tonicité de la peau, et le blanchiment ou le bronzage de la peau ; et
ii) un produit agrochimique et/ou industriel, où la surface est la surface d'un objet inerte, d'une plante ou d'un nuisible ou du sol ou de l'eau dans lesquels poussent des plantes ou sont présents des nuisibles, et la composition est destinée à obtenir un effet agrochimique, ledit effet étant un ou plusieurs parmi un acaricide, un algicide, un anthelminthique, un antimite, un avicide, un bactéricide, un chimio-stérilisant, un engrais, un fongicide, un herbicide, un insecticide y compris un ovicide, un répulsif d'insectes, une phéromone d'insectes, un molluscicide, un nématicide, un inhibiteur de nitrification, un pesticide, un répulsif pour nuisibles, un promoteur de croissance des plantes, un rodenticide, un engrais pour sol, un termicide, un virucide et un protecteur de blessures pour plantes.
